# EUROPEAN PATENT APPLICATION

(11) **EP 4 850 724 A2**
(43) Date of publication of application: **09.12.2026**
(21) Application number: 26213576.7
(22) Date of filing: 10.06.2022
(51) Int. Cl.: A61M 5/38

(54) **GAS STERILIZABLE SYRINGES HAVING APERTURES COVERED BY GAS PERMEABLE BARRIERS FOR ENABLING INGRESS AND EGRESS OF STERILIZATION GASES WHILE PREVENTING LEAKAGE OF FLOWABLE MATERIALS**

(30) Priority: 11.06.2021 US 202163209434 P; 10.08.2021 US 202163231494 P; 17.08.2021 US 202163233910 P; 09.02.2022 US 202217667950
(62) Document number(s) of the earlier application(s) in accordance with Art. 76 EPC: 22735997.3 / 4 164 714
(71) Applicant: Cilag GmbH International, 6300 Zug (CH)
(72) Inventor: OU, Duan Li, Raritan, 08869 (US); ZAVATSKY, Joseph F., Raritan, 08869 (US); QUINTERO, Julian, Raritan, 08869 (US); TANNHAUSER, Robert J., Raritan, 08869 (US); KRIKSUNOV, Leo B., Raritan, 08869 (US); PERKINS, Jason, Raritan, 08869 (US); BLICK, Alison, Raritan, 08869 (US)
(74) Representative: Carpmaels & Ransford LLP

(57) **Abstract**

A gas sterilizable syringe includes an enclosure having walls that define a fluid chamber. A flowable material is disposed within the fluid chamber of the enclosure. A plunger is assembled with the enclosure and is moveable toward a distal end of the enclosure for dispensing the flowable material. One or more apertures are formed in at least one of the walls of the enclosure. A gas permeable barrier covers at least one of the apertures formed in at least one of the walls of the enclosure for enabling sterilization gases to pass through the at least one aperture covered by the gas permeable barrier while preventing the flowable material from passing through the at least one of aperture. The gas permeable barrier is permeable to the sterilization gases and impermeable to the flowable material disposed within the fluid chamber of the enclosure.

## Description

### CROSS-REFERENCE TO RELATED APPLICATIONS

The present patent application claims benefit of U.S. Provisional Application No. 63/209,434, filed on June 11, 2021 (Attorney Docket No. ETH6121USPSP1), U.S. Provisional Application No. 63/231,494, filed August 10, 2021 (Attorney Docket No. ETH6132USPSP1) and U.S. Provisional Application No. 63/233,910, filed on August 17, 2021 (Attorney Docket No. ETH6120USPSP1), the disclosures of which are hereby incorporated by reference herein. The present patent application is also related to commonly assigned U.S. Patent Application Ser. No. 17/667,969 (Attorney Docket No. ETH6141 USNP1), concurrently filed herewith, the disclosure of which is hereby incorporated by reference herein.

### BACKGROUND OF THE INVENTION

### Field of the Invention

The present patent application is generally related to medical devices and is more particularly related to syringes that are used for dispensing flowable materials.

### Description of the Related Art

In order to protect patients and enhance their post-procedure healing and recovery, it is important to maintain sterile conditions when performing surgical procedures. It is also necessary to sterilize the medical devices, tools, and components that are used during surgical procedures.

Thus, there have been many efforts directed to providing sterile medical devices and surgical tools. In some instances, the medical devices and surgical tools are placed inside packages and containers for being sterilized. For example, U.S. Patent No. 7,909,249 to Bagozzi et al. discloses a steam sterilizable apparatus that is designed to hold a medical device. The apparatus includes a capsule having a shape that conforms to the shape of the medical device that is placed inside the capsule. The apparatus includes an enclosure configured to cooperate with the capsule to contain the medical device. The capsule and/or the enclosure may include openings to permit steam to enter and exit the apparatus to sterilize the medical device (e.g., an implant).

U.S. Patent No. 8,276,348 to Mermet et al. discloses a container designed for holding one or more objects for sterilization. The container has an inlet opening and a discharge opening through which the one or more objects may pass into and out of the container. The container includes a rigid part having a peripheral wall bored with small holes, and a non-rigid part that is porous to a sterilization fluid and non-porous to microbial contamination. The openings in the rigid part of the container and the porosity of the non-rigid part allow a sufficient diffusion of the sterilization fluid inside the rigid part and the non-rigid part, and around the one or more objects contained within the rigid part.

U.S. Patent No. 4,154,342 to Wallace discloses a sterilizable package for medical or surgical instruments. The package includes a rigid or semi-rigid container including a filter element made of porous plastic that is adapted to allow the passage of a sterilizing gas therethrough while preventing bacteria from entering into the package.

WO2014/187779 discloses a method of sterilizing the surface of a pre-filled syringe. The syringe is placed inside a package that is constructed of one or more materials that are gas permeable. The sterilization method is carried out at a low temperature of 15-50 degrees Centigrade.

U.S. Patent No. 10,710,759 to Lu et al. discloses a method of packaging pre-filled medical devices. The method includes producing a packaging having a front panel and a back panel defining a compartment capable of holding one or more medical devices. At least one of the front panel or top panel has a portion containing a gas permeable material while the remaining portion of the pouch is gas impermeable. The gas permeable material allows sterilization gas to pass through the material and contact the one or more articles contained within the compartment. Upon completing sterilization, the pouch is sealed, and the gas permeable portion is cut away leaving the sterilized medical device enclosed in a completely gas impermeable pouch.

There have also been efforts directed to sterilizing medical devices without placing the medical devices inside a container for sterilization. For example, U.S. Patent No. 8,617,483 to Fischer et al. disclose a system to sterilize a flowable implant material after it has been packaged inside a sealed syringe. The system includes a porous sleeve that fits within the syringe and fluidly communicates with the atmosphere outside the syringe. Placing the porous sleeve adjacent the flowable implant material creates a path of least resistance for the permeation of gas located at an open end of the container and effectively decreases the maximum gas permeation length through the flowable implant material packaged inside the sealed syringe, which enables the flowable implant material to be sterilized by a gaseous agent while it is inside the syringe.

U.S. Patent No. 10,064,990 to Sodhi discloses a syringe assembly having a fluid path that can be sterilized by gas or radiation. The syringe assembly includes a plunger rod, a syringe barrel, and first and second caps that permit sterilization of a portion of the fluid path by radiation or a gas. The structural features providing for sterilization of the fluid path allows the fluid path to remain sterile without the need for external packaging material surrounding the syringe assembly.

U.S. Patent No. 8,435,217 to Winn discloses a gas sterilizable delivery system for a two-part polymer. The delivery system includes at least two syringe barrels, each barrel being sealed with a gas permeable plunger seal that allows a sterilant gas to permeate through the plunger seal for gas sterilizing the assembly.

In spite of the above advances, there is a continuing need for systems, devices, and methods for easily and efficaciously sterilizing syringes and the materials loaded into syringes, and maintaining the syringes and the pre-loaded materials in sterile conditions prior to and during medical procedures.

There is also a need for low cost, easy to source sterilizable syringes that may be used for dispensing precursors (e.g., silicone-based polymers) having relatively high viscosities (e.g., up to 1,000,000 Centipoise), whereby the precursors may be joined together (e.g., mixed) for forming tissue adhesives.

### SUMMARY OF THE INVENTION

In one embodiment, a gas sterilizable syringe preferably includes an enclosure having one or more walls that define a fluid chamber, and a viscous, flowable material (e.g., a silicone-based polymer; a precursor used to form a tissue adhesive) disposed within the fluid chamber.

In one embodiment, the enclosure preferably includes a plunger (e.g., a syringe plunger with a piston) that is moveable for dispensing the viscous, flowable material.

In one embodiment, the enclosure desirably includes a plurality of micro-apertures formed in at least one of the walls of the enclosure that are in fluid communication with the fluid chamber. The micro-apertures preferably have respective geometries and/or sizes (e.g., cross-sectional diameters) that allow sterilization gases (e.g., ethylene oxide) to pass through the micro-apertures while preventing the viscous, flowable material from passing therethrough. Thus, the micro-apertures are preferably sufficiently large in size to allow the sterilizing gas to pass therethrough but sufficiently small in size to prevent the viscous, flowable material from leaking out of the micro-apertures when positive pressure is applied for dispensing the viscous, flowable material
In one embodiment, the enclosure may include a syringe barrel having a distal end wall, a dispensing tip projecting from the distal end wall of the syringe barrel, an end cap secured to a distal end of the dispensing tip, and a piston secured to a distal end of the plunger.

In one embodiment, the micro-apertures preferably have inner diameters ID₁ of about 0.1 microns to about 25 microns, and more preferably about one (1) micron.

In one embodiment, the viscous, flowable material preferably has a viscosity of about 1,000-100,000 centipoise, more preferably about 2,000-75,000 centipoise, and even more preferably about 30,000-60,000 centipoise.

In one embodiment, a sterilization gas (e.g., ethylene oxide) is able to pass through the micro-apertures formed in one or more walls of the enclosure for sterilizing the viscous, flowable material disposed within the fluid chamber. During a dispensing operation, when the viscous, flowable material is being dispensed under positive pressure, however, the relatively high viscosity of the flowable material prevents the flowable material from passing through and/or leaking out of the micro-apertures.

In one embodiment, a sterilizable syringe may include micro-apertures or micro-holes formed in one or more walls of the syringe barrel, the syringe plunger, the piston, the dispensing tip, and/or the end cap that desirably covers a dispensing opening at a distal end of the dispensing tip.

In one embodiment, the micro-apertures may be formed in one or more components of a syringe using various systems, devices, and methods. For example, in one embodiment, a laser device may be used for laser drilling the micro-apertures in one or more components of a syringe.

In one embodiment, laser drilling, especially of polymeric materials or glass, is very fast and cost-effective, and many micro-apertures may be drilled or formed in a syringe within a short period of time (e.g., in seconds) using a laser drill.

In one embodiment, a mechanical component, such as a micro-drill, may be used for forming the micro-apertures in one or more components of a syringe.

In one embodiment, a heated probe may be used to form micro-apertures in a syringe, whereby the heated probe is used to melt material (e.g., polymeric material) to form the micro-apertures.

In one embodiment, a water jet may be used to form micro-apertures in one or more components of a syringe.

In one embodiment, the viscous, flowable material is preferably a high viscosity precursor that is used to make a tissue adhesive. The viscosity precursor (e.g., a silicone polymer) used to make the tissue adhesive will not leak through very narrow micro-apertures having diameters from about 0.1 microns to about 25 microns, and more preferably about one (1) micron. The diameter of the respective micro-apertures is selected so that at the vacuum used, and the pressure used, minimal or no viscous, flowable material (e.g., silicone fluid) is able to leak through the micro-apertures, however, the amount/areal density of the micro-apertures is sufficient for allowing a sterilizing gas (e.g., ethylene oxide gas) to penetrate into the syringe for sterilizing the syringe and the enclosed viscous, flowable material.

In one embodiment, the syringe barrel preferably has a cylindrical-shaped wall that extends from a proximal end to a distal end of the syringe barrel.

In one embodiment, the syringe barrel may be made of polymer materials or glass.

In one embodiment, at least some of the micro-apertures of the enclosure are formed in the cylindrical-shaped outer wall of the syringe barrel.

In one embodiment, the syringe preferably includes a distal end wall that partially closes the distal end of the syringe barrel. In one embodiment, the distal end wall preferably has an opening for dispensing the viscous, flowable material loaded into the syringe barrel.

In one embodiment, at least some of the micro-apertures are formed in the distal end wall of the syringe barrel.

In one embodiment, the micro-apertures formed in the distal end wall are in fluid communication with the fluid chamber and have respective sizes that allow sterilization gases (e.g., ethylene oxide) to pass therethrough for sterilizing the viscous, flowable material while preventing the viscous, flowable material from passing therethrough.

In one embodiment, the syringe includes the dispensing tip projecting from the distal end wall of the syringe barrel for dispensing the viscous, flowable material.

In one embodiment, the dispensing tip includes a tube-shaped outer wall that defines a conduit that is in fluid communication with the fluid chamber.

In one embodiment, at least some of the micro-apertures are formed in the tube-shaped outer wall of the dispensing tip. The dispensing tip micro-apertures are desirably in fluid communication with the fluid chamber.

In one embodiment, the micro-apertures formed in the tube-shaped outer wall of the dispensing tip are in fluid communication with the fluid chamber and have respective sizes that allow sterilization gases (e.g., ethylene oxide) to pass therethrough for sterilizing the viscous, flowable material while preventing the viscous, flowable material from passing through the micro-apertures.

In one embodiment, the syringe preferably includes an end cap secured to a distal end of the outer wall of the dispensing tip.

In one embodiment, at least some of the micro-apertures are formed in the end cap.

In one embodiment, the micro-apertures formed in the end cap are in fluid communication with the fluid chamber and have respective geometries or sizes (e.g., cross-sectional diameters) that enable sterilization gases (e.g., ethylene oxide) to pass therethrough for sterilizing the viscous, flowable material while preventing the viscous, flowable material from passing therethrough.

In one embodiment, the syringe plunger has a distal end including a piston having an outer perimeter that engages an inner surface of the cylindrical-shaped wall of the syringe barrel that defines the fluid chamber.

In one embodiment, at least some of the micro-apertures of the enclosure are formed in the piston.

In one embodiment, the micro-apertures formed in the piston are in fluid communication with the fluid chamber and have respective geometries or sizes (e.g., cross-sectional diameters) that enable sterilization gases (e.g., ethylene oxide) to pass therethrough for sterilizing the viscous, flowable material while preventing the viscous, flowable material from passing therethrough.

In one embodiment, the enclosure of the gas sterilizable syringe preferably has a proximal end, a distal end, and a longitudinal axis that extends between the proximal and distal ends of the enclosure.

In one embodiment, at least some of the micro-apertures formed in the enclosure preferably extend along respective axes that are perpendicular to the longitudinal axis of the enclosure.

In one embodiment, at least some of the micro-apertures formed in the enclosure preferably extend along respective axes that are parallel with the longitudinal axis of the syringe barrel.

In one embodiment, at least some of the micro-apertures formed in the enclosure preferably extend along respective axes that are diagonal to the longitudinal axis of the syringe barrel.

In one embodiment, the micro-apertures formed in the enclosure are located adjacent the proximal end of the enclosure.

In one embodiment, a gas sterilizable syringe may include a microporous layer, film, or sleeve (hereinafter referred to as a "microporous layer") that covers at least some of the micro-apertures formed therein. The microporous layer (e.g., made of TYVEK^{®}, or a TYVEK-like material) allows the sterilization gases to pass therethrough while preventing the viscous, flowable material disposed within the fluid chamber of the enclosure from passing therethrough. The microporous layer may be a synthetic material, such as a synthetic material made from flashspun, high-density polyethylene fibers.

In one embodiment, a microporous layer covers an outer surface of at least one of the walls of the enclosure (e.g., an outer surface of cylindrical-shaped wall of a syringe barrel).

In one embodiment, a microporous layer covers an inner surface of at least one of the walls of the enclosure (e.g., an inner surface of a cylindrical-shaped wall of a syringe barrel).

In one embodiment, a microporous layer preferably covers one or more micro-apertures formed in a component of a sterilizable syringe for enabling sterilization gases to pass through the microporous layer and the one or more micro-apertures that are covered by the microporous layer.

In one embodiment, a gas sterilizable syringe may include a protective sleeve (e.g., a nonporous sleeve) that covers an outer surface of an outer wall of an enclosure (e.g., a syringe barrel).

In one embodiment, a syringe barrel has an outer wall having a cylindrical shape and the protective sleeve has a cylindrical shape that conforms to the cylindrical-shaped outer wall of the syringe barrel.

In one embodiment, the protective sleeve is moveable between an extended position for covering at least some of the micro-apertures and a retracted position for uncovering and exposing the at least some of the micro-apertures. In one embodiment, a sterilizable syringe is sterilized with the protective sleeve in a retracted position so that the sterilization gases may pass through the uncovered micro-apertures. After sterilization, the protective sleeve may be moved into the extended position for covering the micro-apertures during packaging, shipment, storage and/or use of the syringe. In one embodiment, the protective sleeve, in the extended position, may cover one or more of the micro-apertures to prevent the viscous, flowable material from leaking through the micro-apertures.

In one embodiment, the micro-apertures may extend completely through at least one of the walls of the enclosure, from an outer surface to an inner surface of the wall.

In one embodiment, at least one of the walls of the enclosure desirably has an outer surface and an inner surface, and at least some of the micro-apertures may include blind openings having open, outer ends that are exposed at the outer surface of the wall and closed, inner ends that are spaced away from the inner surface of the wall.

In one embodiment, a wall of the enclosure preferably includes a membrane that is located between the closed, inner ends of the blind openings of the micro-apertures and the inner surface of the wall of the enclosure. The membrane is preferably thin enough to allow sterilization gases to pass therethrough, but sufficiently thick to prevent the viscous, flowable material from passing and/or leaking through the micro-apertures.

In one embodiment, a gas sterilizable syringe preferably includes a syringe barrel having an outer wall that defines and/or surrounds a fluid chamber, a viscous, flowable material disposed within the fluid chamber, and a syringe plunger assembled with the syringe barrel.

In one embodiment, the gas sterilizable syringe desirably includes a plurality of micro-apertures formed in an outer wall of the syringe barrel that are in fluid communication with the fluid chamber.

In one embodiment, the plurality of micro-apertures have respective geometries and/or sizes (e.g., cross-sectional diameters) that allow sterilization gases to pass therethrough for sterilizing the viscous, flowable material while preventing the viscous, flowable material from passing and/or leaking through the micro-apertures, such as during a dispensing operation when a plunger is used to generate positive pressure that is applied to the viscous, flowable material.

In one embodiment, the micro-apertures have respective openings that extend from an outer surface to an inner surface of the outer wall of the syringe barrel.

In one embodiment, the respective openings of the micro-apertures have inner diameters of 0.1 - 25 microns.

In one embodiment, the respective openings of the micro-apertures have inner diameters of 1 micron.

In one embodiment, a gas sterilizable syringe preferably includes a microporous layer overlying a surface of the outer wall of the syringe barrel and covering at least some of the micro-apertures.

In one embodiment, the microporous layer preferably allows sterilization gases to pass therethrough while preventing the viscous, flowable material from passing and/or leaking therethrough.

In one embodiment, the microporous layer may be a material sold under the trademark TYVEK^{®}, which is a brand of flashspun, high-density polyethylene fibers, or a synthetic material that is similar in structure and function to the TYVEK^{®} brand.

In one embodiment, a gas sterilizable syringe preferably includes a protective sleeve covering an outer surface of the outer wall of the syringe barrel.

In one embodiment, the protective sleeve is moveable between an extended position for covering at least some of the micro-apertures and a retracted position for uncovering and exposing at least some of the micro-apertures.

In one embodiment, the micro-apertures extend completely through the outer wall of the syringe barrel, from an outer surface of the outer wall of the syringe barrel to an inner surface of the outer wall of the syringe barrel.

In one embodiment, the micro-apertures include blind openings having open, outer ends that are exposed at an outer surface of the outer wall of the syringe barrel and closed, inner ends that are spaced away from an inner surface of the outer wall of the syringe barrel that defines the fluid chamber.

In one embodiment, the outer wall of the syringe barrel preferably includes a membrane that is located between the closed, inner ends of the blind openings of the micro-apertures and the inner surface of the outer wall of the syringe barrel. Sterilizing gases may diffuse through the membrane, however, the viscous, flowable material may not pass through the membrane.

In one embodiment, the syringe plunger has a distal end including a piston that is disposed within the fluid chamber for engaging the viscous, flowable material.

In one embodiment, the outer wall of the syringe barrel includes a cylindrical-shaped wall including some of the micro-apertures.

In one embodiment, the syringe barrel preferably includes a distal end wall having micro-apertures formed therein that are in fluid communication with the fluid chamber.

In one embodiment, the micro-apertures formed in the distal end wall preferably have respective geometries and/or sizes that allow sterilization gases to pass therethrough for sterilizing the viscous, flowable material while preventing the viscous, flowable material from passing therethrough.

In one embodiment, the gas sterilizable syringe preferably includes a dispensing tip projecting from the distal end wall of the syringe barrel that is in fluid communication with the fluid chamber of the syringe barrel.

In one embodiment, the dispensing tip preferably has an outer wall including micro-apertures formed therein that are in fluid communication with the fluid chamber.

In one embodiment, the micro-apertures formed in the dispensing tip preferably have respective geometries and/or sizes that allow sterilization gases to pass therethrough for sterilizing the viscous, flowable material while preventing the viscous, flowable material from passing therethrough.

In one embodiment, the gas sterilizable syringe may include an end cap connected with a distal end of the dispensing tip. The end cap may be removed from the dispensing tip prior to dispensing the viscous, flowable material.

In one embodiment, the end cap may include end cap micro-apertures formed therein that are in fluid communication with the fluid chamber.

In one embodiment, the micro-apertures formed in the end cap preferably have respective geometries and/or sizes that allow sterilization gases to pass therethrough for sterilizing the viscous, flowable material while preventing the viscous, flowable material from passing therethrough.

In one embodiment, the piston has an outer perimeter that engages an inner surface of a wall of the syringe barrel that defines the fluid chamber.

In one embodiment, the piston preferably includes piston micro-apertures formed therein that extend through the piston for being in fluid communication with the fluid chamber.

In one embodiment, the micro-apertures formed in the piston preferably have respective geometries and/or sizes that allow sterilization gases to pass therethrough for sterilizing the viscous, flowable material while preventing the viscous, flowable material from passing through the piston.

In one embodiment, one or more micro-apertures may be drilled in the piston of the syringe plunger. In one embodiment, any flowable material that escapes via the micro-apertures formed in the piston is preferably captured within a rear/proximal section of the syringe barrel so that the flowable material will not contact the hands of the user.

In one embodiment, a gas sterilizable syringe preferably includes a syringe barrel having a cylindrical-shaped outer wall and a distal end wall that closes a distal end of the cylindrical-shaped outer wall.

In one embodiment, the cylindrical-shaped outer wall and the distal end wall define a fluid chamber of the syringe barrel.

In one embodiment, a gas sterilizable syringe preferably includes a dispensing tip projecting from the distal end wall. The dispensing tip preferably has a conduit that is in fluid communication with the fluid chamber.

In one embodiment, the gas sterilizable syringe preferably has an end cap covering a distal end of the dispensing tip.

In one embodiment, a viscous, flowable material is disposed within the fluid chamber of the syringe barrel, and a syringe plunger is assembled with the syringe barrel that is moveable toward the distal end wall of the syringe barrel for dispensing the viscous, flowable material from the distal end of the dispensing tip.

In one embodiment, a plurality of micro-apertures may be formed in the cylindrical-shaped outer wall of the syringe barrel that are in fluid communication with the fluid chamber. In one embodiment, the micro-apertures have respective geometries and/or sizes (e.g., cross-sectional diameters) that allow sterilization gases (e.g., ethylene oxide) to pass therethrough for sterilizing the viscous, flowable material while preventing the viscous, flowable material from passing therethrough.

In one embodiment, a gas sterilizable syringe preferably includes a microporous layer that covers at least some of the micro-apertures, whereby the microporous layer allows the sterilization gases to pass therethrough for sterilizing the viscous, flowable material while preventing the viscous, flowable material from passing therethrough.

In one embodiment, the microporous layer is in contact with an outer surface of the cylindrical-shaped wall of the syringe barrel.

In one embodiment, the microporous layer is in contact with an inner surface of the cylindrical-shaped wall of the syringe barrel.

In one embodiment, a gas sterilizable syringe may include a distal end wall having distal end wall micro-apertures formed therein that are in fluid communication with the fluid chamber of the syringe barrel.

In one embodiment, the distal end wall micro-apertures have respective cross-sectional diameters that allow sterilization gases to pass therethrough while preventing the viscous, flowable material from passing therethrough.

In one embodiment, a microporous layer may cover at least some of the distal end wall micro-apertures.

In one embodiment, a gas sterilizable syringe may include a dispensing tip having dispensing tip micro-apertures formed in an outer wall thereof that are in fluid communication with the fluid chamber of the syringe barrel.

In one embodiment, the dispensing tip micro-apertures have respective cross-sectional diameters that allow sterilization gases to pass therethrough while preventing the viscous, flowable material from passing therethrough.

In one embodiment, a microporous layer may cover at least some of the micro-apertures formed in the dispensing tip.

In one embodiment, a gas sterilizable syringe preferably includes an end cap having end cap micro-apertures formed therein that are in fluid communication with the fluid chamber of the syringe barrel.

In one embodiment, the micro-apertures formed in the end cap have respective geometries and/or sizes (e.g., cross-sectional diameters) that enable the sterilization gases to pass therethrough while preventing the viscous, flowable material from passing therethrough.

In one embodiment, a microporous layer preferably covers at least some of the micro-apertures formed in the end cap.

In one embodiment, a gas sterilizable syringe may include a protective sleeve covering an outer surface of the cylindrical-shaped outer wall of the syringe barrel.

In one embodiment, the protective sleeve is moveable between an extended position for covering at least some of the micro-apertures and a retracted position for uncovering and exposing the at least some of the micro-apertures.

In one embodiment, a sterilizable syringe may include various components such an enclosure, such as a syringe barrel, having a distal end wall that partially closes the distal end of the enclosure, a syringe plunger having a piston that forms a seal inside the enclosure, a dispensing tip, and an end cap that is configured to cover a distal end of the dispensing tip.

In one embodiment, the enclosure may be a syringe or a vial that is made of polymeric materials and/or glass.

In one embodiment, the piston may be made of polymeric materials and/or rubber.

In one embodiment, a syringe is preferably designed for dispensing viscous, flowable materials such as precursors and/or components (e.g., silicone-based polymers) that may be used to form tissue adhesives, and more preferably high viscosity, flowable precursors (e.g., silicone-based polymers) that may be used to make tissue adhesives.

In one embodiment, the micro-apertures formed in an outer wall of a syringe barrel are preferably located at a proximal end of the syringe barrel and close to the location of the piston at the start of a dispensing procedure, so that as the piston advances distally toward the distal end of the syringe barrel, the piston moves into the area of the outer wall of the syringe barrel that is devoid of micro-apertures to prevent leaks of the flowable material (because the piston has moved distal to the micro-apertures) and to provide for continuous expression of the flowable material.

In one embodiment, the micro-apertures formed in a syringe may be blind micro-apertures that are not drilled completely through a wall that defines a fluid chamber, thereby leaving a small portion of the wall intact as a very thin membrane that may be permeated by the sterilization gas (e.g., ethylene oxide gas) for sterilizing the viscous, flowable material loaded into the syringe barrel.

In one embodiment, a gas sterilizable syringe includes an enclosure having walls that define a fluid chamber, a flowable material disposed within the fluid chamber of the enclosure, and a plunger assembled with the enclosure and being moveable toward a distal end of the enclosure for dispensing the flowable material from the enclosure.

In one embodiment, one or more apertures may be formed in at least one of the walls of the enclosure. In one embodiment, a gas permeable barrier covers at least one of the apertures formed in the at least one of the walls of the enclosure for enabling sterilization gases to pass through the at least one of the apertures covered by the gas permeable barrier while preventing the flowable material from passing through the at least one of the apertures covered by the gas permeable barrier.

In one embodiment, the gas permeable barrier that covers the at least one of the apertures formed in the at least one of the walls of the enclosure is permeable to the sterilization gases and impermeable to the flowable material disposed within the fluid chamber of the enclosure.

In one embodiment, the gas permeable barrier has a porosity that is high enough to enable ingress and egress of the sterilization gases during a sterilization procedure but is sufficiently low enough to prevent the flowable material from passing through the gas permeable barrier.

In one embodiment, the one or more apertures formed in the at least one of the walls of the enclosure comprise a plurality of apertures formed in the at least one of the walls of the enclosure.

In one embodiment, the gas permeable barrier comprises a plurality of gas permeable bodies filling each of the respective plurality of apertures formed in the at least one of the walls of the enclosure.

In one embodiment, the plurality of gas permeable bodies filling each of the respective plurality of apertures are bonded to the at least one of the walls of the enclosure.

In one embodiment, the gas permeable barrier may be made of silicone, room temperature vulcanized silicone (RTV), liquid silicone rubber (LSR), high consistency rubber (HCR), and synthetic flashspun high-density polyethylene fibers (e.g., a TYVEK^{®} layer).

In one embodiment, the sterilization gases may include ethylene oxide, and the flowable material may include liquids, topical skin adhesives and/or silicone-based topical skin adhesives.

In one embodiment, the enclosure may include a syringe barrel that surrounds the fluid chamber, a dispensing opening located at a distal end of the syringe barrel, and a plunger moveable toward the distal end of the syringe barrel for dispensing the flowable material via the dispensing opening located at the distal end of the syringe barrel.

In one embodiment, the gas permeable barrier covers the dispensing opening for enabling the sterilization gases to pass through the dispensing opening while preventing the flowable material from passing through the dispensing opening.

In one embodiment, the gas sterilizable syringe may include an end cap secured to the distal end of the syringe barrel and covering the dispensing opening. In one embodiment, one or more end cap openings may be formed in the end cap for enabling the sterilization gases to pass through the end cap.

In one embodiment, the gas permeable barrier is disposed inside the end cap and is located between the one or more end cap openings and the dispensing opening at the distal end of the syringe barrel.

In one embodiment, the end cap has a central hub that engages a distal end wall of the syringe barrel for forming an airtight seal between the central hub of the end cap and the distal end wall of the syringe barrel.

In one embodiment, a gas sterilizable syringe desirably includes a dual barrel syringe including a first syringe barrel having a first fluid chamber and a first fluid dispensing opening and a second syringe barrel having a second fluid chamber and a second fluid dispensing opening.

In one embodiment, the gas sterilizable syringe may include a dual barrel plunger including a first plunger disposed within the first fluid chamber of the first syringe barrel and a second plunger disposed within the second fluid chamber of the second syringe barrel.

In one embodiment, an end cap is releasably secured to a distal end of the dual barrel syringe for covering the first and second fluid dispensing openings.

In one embodiment, the end cap has at least one end cap opening formed therein.

In one embodiment, a gas permeable barrier is disposed within the end cap and is located between the at least one end cap opening and the first and second fluid dispensing openings of the respective first and second syringe barrels.

In one embodiment, a flowable material includes a first part and a second part that are configured for being mixed together. In one embodiment, the first part of the flowable material is disposed within the first fluid chamber of the first syringe barrel, and the second part of the flowable material is disposed within the second fluid chamber of the second syringe barrel.

In one embodiment, the gas permeable barrier desirably covers the first and second fluid dispensing openings of the first and second syringe barrels. The gas permeable barrier enables sterilization gases to pass through the first and second fluid dispensing openings while preventing the first and second parts of the flowable material from passing through the first and second fluid dispensing openings.

In one embodiment, the end cap includes a hub that engages a distal end wall of the dual barrel syringe for forming an airtight seal between the end cap hub and the distal end wall of the dual barrel syringe.

In one embodiment, the end cap hub has a proximal end that is open for receiving the first and second fluid dispensing openings and a distal end that is closed by a distal end wall that includes the at least one end cap opening.

In one embodiment, the end cap preferably includes at least one radially extending projection that extends outwardly from an outer surface of the end cap hub.

In one embodiment, the dual barrel syringe desirably includes at least one securing flange projecting from the distal end wall of the dual barrel syringe. The at least one securing flange is configured to engage the at least one radially extending projection of the end cap hub for releasably securing the end cap to the distal end wall of the dual barrel syringe.

In one embodiment, the gas sterilizable syringe desirably includes an applicator tip configured for expressing the flowable material from the distal end of the dual barrel syringe. The applicator tip desirably includes a dispensing tube having a proximal end and a distal end, an applicator tip connector secured to the proximal end of the dispensing tube, and a static mixer disposed within the dispensing tube.

In one embodiment, the applicator tip connector preferably has at least one radially extending projection that is configured to engage the at least one securing flange projecting from the distal end wall of the dual barrel syringe for releasable securing the applicator tip to the distal end wall of the dual barrel syringe.

In one embodiment, the gas permeable barrier is permeable to the sterilization gases and impermeable to the first and second parts of the flowable material. Thus, the sterilization gases can flow through the gas permeable barrier, however, the first and second parts of the flowable material cannot flow through the gas permeable barrier.

In one embodiment, the gas permeable barrier has a porosity that is high enough to enable ingress and egress of the sterilization gases during a sterilization procedure but sufficiently low enough to block passage of the first and second parts of the flowable material through the gas permeable barrier.

In one embodiment, a gas sterilizable syringe desirably includes a dual barrel syringe including a first syringe barrel and a second syringe barrel. The first syringe barrel preferably includes a first fluid chamber and a first fluid dispensing opening located at a distal end of the first syringe barrel, and the second syringe barrel preferably includes a second fluid chamber and a second fluid dispensing opening located at a distal end of the second syringe barrel.

In one embodiment, a first part of a flowable material is disposed within the first fluid chamber of the first syringe barrel and a second part of the flowable material is disposed within the second fluid chamber of the second syringe barrel.

In one embodiment, a dual barrel plunger is assembled with the dual barrel syringe. The dual barrel plunger preferably includes a first plunger disposed within the first fluid chamber of the first syringe barrel and a second plunger disposed within the second fluid chamber of the second syringe barrel.

In one embodiment, an end cap may be releasably secured to a distal end of the dual barrel syringe for covering the first and second fluid dispensing openings located at the distal ends of the respective first and second syringe barrels. In one embodiment, the end cap has at least one end cap opening formed therein.

In one embodiment, a gas permeable barrier is disposed within the end cap and is located between the at least one end cap opening and the first and second fluid dispensing openings located at the distal ends of the respective first and second syringe barrels. The gas permeably barrier is permeable to sterilization gases and impermeable to the flowable material for enabling the sterilization gases to pass through the first and second fluid dispensing openings while preventing the first and second parts of the flowable material from passing through the first and second fluid dispensing openings and/or the gas permeable barrier.

In one embodiment, the end cap desirably includes a central hub that engages a distal end wall of the dual barrel syringe for forming an airtight seal between the end cap hub and the distal end wall of the dual barrel syringe.

In one embodiment, the central hub of the end cap has a proximal end that is open for receiving the first and second fluid dispensing openings and a distal end that is closed by a distal end wall that includes the at least one end cap opening.

In one embodiment, the end cap includes at least one radially extending projection that extends outwardly from an outer surface of the central hub of the end cap.

In one embodiment, the dual barrel syringe desirably includes at least one securing flange projecting from the distal end wall of the dual barrel syringe that is configured to engage the at least one radially extending projection of the end cap for releasably securing the end cap to the distal end wall of the dual barrel syringe.

In one embodiment, the first syringe barrel preferably has a cylindrical-shaped outer wall that surrounds the first fluid chamber and that extends to the distal end of the first syringe barrel.

In one embodiment, at least one first air vent opening is formed in the cylindrical-shaped outer wall of the first syringe barrel.

A first one-way plunger stop may be formed in an inner surface of the cylindrical-shaped outer wall of the first syringe barrel. The first one-way plunger stop may be located between the at least one first air vent and the distal end of the first syringe barrel.

In one embodiment, at least one second air vent opening is formed in the cylindrical-shaped outer wall of the second syringe barrel.

A second one-way plunger stop may be formed in an inner surface of the cylindrical-shaped outer wall of the second syringe barrel. The second one-way plunger stop may be located between the at least one second air vent of the second syringe barrel and the distal end of the second syringe barrel.

In one embodiment, the first plunger preferably includes a first plunger head located at a distal end of the first plunger. In one embodiment, the first plunger head is in contact with the first one-way plunger stop and is located between the at least one first air vent and the distal end of the first syringe barrel.

In one embodiment, the second plunger preferably includes a second plunger head located at a distal end of the second plunger. In one embodiment, the second plunger head is in contact with the second one-way plunger stop and is located between the at least one second air vent and the distal end of the second syringe barrel.

In one embodiment, an enclosure (e.g., a syringe, a vial) that is configured to be filled with a flowable material (e.g., a liquid, a silicone-based topical skin adhesive) is gas permeable to allow sterilization gases (e.g., ethylene oxide) to pass into the enclosure while maintaining a liquid sealing capability to prevent leakage of the flowable material contained within the enclosure. The enclosure may be made of polymeric materials and/or glass.

In one embodiment, the enclosure has one or more walls and one or more apertures formed in at least one of the walls. In one embodiment, at least one of the apertures is filled with a gas permeable barrier (e.g., a gas permeable plug; a gas permeable mass; a gas permeable seal) that allows the sterilization gas to pass through the gas permeable barrier for sterilizing the content (e.g., the flowable material) of the enclosure while preventing the content of the enclosure from leaking out of the enclosure.

In one embodiment, an enclosure for a flowable material has a plurality of apertures with each aperture being filled with a gas permeable mass.

In one embodiment, the gas permeable masses are permeable to sterilization gases and impermeable to the flowable material (e.g., liquids, a flowable topical skin adhesive).

In one embodiment, an enclosure for a liquid composition is configured to allow gas sterilization. The enclosure has one or more apertures that may be formed anywhere on the enclosure. The one or more apertures are preferably filled with a porous, curable, composition that is permeable to sterilization gases. The gas permeable composition has a porosity that is high enough to allow ingress and egress of sterilization gases during sterilization, but sufficiently low enough to prevent leakage of the liquids stored within the enclosure.

In one embodiment, each of the apertures formed in the enclosure has a diameter of about 1-5mm. The gas permeable barriers preferably seal the apertures having the 1-5mm diameter. In one embodiment, the gas permeable barriers preferably bond to the enclosure within the respective apertures.

In one embodiment, the apertures may be filled by a curable composition having a liquid, uncured, or partially cured state. After being disposed within the apertures, the curable composition may be cured to form porous, gas permeable barriers, plugs, bodies and/or seals that enable sterilizing gases (e.g., ethylene oxide) to pass therethrough for sterilizing the inside of the enclosure while preventing a flowable material disposed within the enclosure from leaking out of the enclosure.

In one embodiment, the enclosure may include a syringe barrel, a syringe barrel distal end wall, a plunger, a dispensing tip, and an end cap that closing the dispensing opening of the dispensing tip. One or more of the above-described apertures may be formed in any one of the syringe barrel, syringe barrel distal end wall, plunger, dispensing tip, and end cap, and each aperture may be filled with one of the gas permeable barriers, plugs, bodies and/or seals disclosed herein.

In one embodiment, the formulation used to form the gas permeable barriers is preferably porous, curable, and capable of reliably plugging and/or sealing the holes while not affecting the stability of the flowable material (e.g., a silicone-based a topical skin adhesive) stored within the enclosure.

In one embodiment, one or more of the apertures may have an asymmetric shape and/or cross-section, and the formulation used to form the gas permeable barriers (e.g., seals; plugs) may be introduced into the apertures in an uncured state so that the formulation may conform to the shape of the apertures prior to being cured. After being fully cured, the gas permeable barriers preferably conform to the unique shapes of the apertures.

In one embodiment, a system for making a gas permeable enclosure may include a sacrificial sleeve/cylinder that is insertable into an enclosure (e.g., a syringe barrel) while introducing the formulation that is used to form the gas permeable seals into the one or more apertures formed in the enclosure. In one embodiment, the sacrificial sleeve/cylinder remains positioned inside the enclosure while curing the formulation used to form the gas permeable seals. After the formulation used to form the gas permeable seals has been cured, the sacrificial sleeve/cylinder may be removed from the enclosure. The sacrificial sleeve/cylinder may function as a frame that shapes the gas permeable seals to yield a smooth surface between the inner surface of the enclosure that includes the one or more apertures and the surfaces of the gas permeable seals that fill the one or more apertures.

Cyanoacrylate based skin closure systems are often used as topical skin adhesives for closing wounds, however, cyanoacrylate based adhesives are relatively inflexible and have a degree of elasticity that is <1%. When a wound that has been closed with a cyanoacrylate based adhesive is placed on a moving body part (e.g., a knee, a wrist, an elbow) and is stretched, the resulting distribution of stress effects can adversely impact the performance and durability of the wound closure.

Silicone-based topical skin adhesives have proven to be much more effective for sealing wounds on moving body parts. The holding forces between a silicone-based adhesive and skin is equal to or better than the results that can be attained when using cyanoacrylate based skin closure products. A silicone-based topical skin adhesive can be stretched up to 160% of its original length and fully recover to its original dimension. When a silicone-based topical skin adhesive is combined with an anisotropic mesh, the combination can be used to optimize the stress distribution and improve the performance on human skin compared to the relatively inelastic cyanoacrylate based systems.

In one embodiment, the flowable material that is dispensed from a gas sterilizable syringe preferably includes a silicone-based topical skin adhesive that may be used for closing and/or sealing wounds, including wounds located on moveable body parts.

The heat sterilization methods that are used for sterilizing cyanoacrylate based skin closure products are unsuitable for silicone-based topical skin adhesives. Thus, there is a need for improved systems, devices and methods for sterilizing devices (e.g., syringes) that are used for dispensing silicone-based topical skin adhesives.

In one embodiment, the present patent application discloses systems, devices and methods for enabling sterilization gases (e.g., ethylene oxide) to sterilize a flowable material (e.g., a silicone-based topical skin adhesive) while the flowable material is housed within a gas sterilizable syringe.

In one embodiment, the gas sterilizable syringe preferably includes an enclosure (e.g., a syringe barrel) having walls that define a fluid chamber that is configured to receive a flowable material (e.g., a silicone-based topical skin adhesive; a component of a topical skin adhesive). In one embodiment, the enclosure desirably includes a plunger that is moveable for dispensing the flowable material.

In one embodiment, a distal end of the enclosure includes a dispensing opening for expressing the flowable material from the distal end of the gas sterilizable syringe. In one embodiment, an end cap may be secured to the distal end of the enclosure for covering the dispensing opening. The end cap desirably has one or more end cap openings. A gas permeable barrier (e.g., plug; puck; body) is preferably disposed between the end cap and the dispensing opening of the enclosure. The gas permeable barrier is permeable to gases (e.g., sterilization gases) and impermeable to liquids (e.g., a silicone-based topical skin adhesive).

In one embodiment, during a sterilization procedure, the sterilization gases (e.g., ethylene oxide) pass through the end cap openings, the gas permeable barrier, and the dispensing opening for sterilizing the syringe, the enclosure, the fluid chamber, and the flowable material disposed within the fluid chamber of the enclosure.

In certain embodiments, the gas permeable barrier may be made of from one or more of the following materials: silicone materials including room temperature vulcanized silicone (RTV), liquid silicone rubber (LSR), and high consistency rubber (HCR).

In one embodiment, the gas permeable barrier comprises a closed cell structure that has voids and/or porosity (i.e., closed cell porosity). In one embodiment, the gas permeable barrier (e.g., a gas permeable plug) may be molded or die cut.

In one embodiment, the gas sterilizable syringe may include a double barrel syringe and the plunger may include a double barrel plunger.

In one embodiment, the gas sterilizable syringe may include a double barrel syringe that contains a two-part polymer material, with a first part disposed within a first syringe barrel and a second part disposed within a second syringe barrel. The gas sterilizable syringe allows for sterilization of the two parts of the polymer material prior to the two parts being extruded from the syringe.

In one embodiment, the gas sterilizable syringe may include a mixer (e.g., a static mixer, a mixing tip) that is configured for mixing together the two parts of the flowable polymer composition and applying the mixed polymer material to various locations on a human body.

In one embodiment, a gas sterilizable syringe may include a plunger that is vented for enabling sterilization gases to flow around the plunger and into the fluid chamber of the syringe barrel. In one embodiment, the plunger may have a plunger head including a gas permeable plunger seal that enables sterilization gases to flow through the gas permeable plunger seal and into the fluid chamber of the syringe barrel. The gas permeable plunger seal has a porosity that is high enough to enabling sterilization gases (e.g., ethylene oxide) to flow therethrough, but having a porosity that is sufficiently low to prevent the flowable material (e.g., silicone-based topical skin adhesive) from passing therethrough.

In one embodiment, a gas sterilizable syringe having a double barrel plunger may include a first gas permeable plunger seal located at a distal end of the first plunger and a second gas permeable plunger seal located at a distal end of the second plunger.

In one embodiment, the gas permeable barrier (e.g., a gas permeable plug; a gas permeable puck) disposed within the end cap and/or the gas permeable plunger seals securable to the distal ends of the respective plungers have a primarily closed cell porosity.

In one embodiment, the gas permeable barriers and gas permeable plunger seals disclosed herein may also be referred to as gas permeable plugs, gas permeable pucks, gas permeable bodies, gas permeable masses, gas permeable membranes, gas permeable layers, and/or gas permeable septums.

In one embodiment, the gas permeable barrier disposed within the end cap and/or the gas permeable plunger seals located at the distal ends of the plungers may be made from a variety of silicone rubber materials having a porosity range of between 1% and 90% of the total volume, and more preferably between 5% and 80% of the total volume, with mainly closed cell porosity.

In one embodiment, the gas permeable barrier disposed within the end cap and/or the gas permeable plunger seals located at the distal ends of the plungers are made of materials that do not chemically react with any component of the flowable material (e.g., silicone-based topical skin adhesive) contained within the gas sterilizable syringe while sealing the flowable material within the syringe during storage.

In one embodiment, the gas permeable barriers and/or the gas permeable plunger seals may be made of porous silicone materials having a Shore A hardness scale rating of 10-50.

In one embodiment, the porosity of the silicone-based polymer used to form the gas permeable seals may be generated during curing using an in-situ gas generation method.

In one embodiment, the gas permeable seals may be made using commercially available silicone RTV foams, whereby the formulation may be modified to adjust porosity levels to optimize the results when used with a gas sterilizable syringe.

In one embodiment the gas permeable barriers and/or gas permeable plunger seals may be molded or die cut. In one embodiment, the gas permeable barriers disposed within end caps may be die cut from a larger porous silicone rubber sheet. In one embodiment, the gas permeable plunger seals may be made using a molding process.

In other embodiments, the gas permeable barriers may be made using other gas permeable materials, such a rubbers and/or polymers materials.

In one embodiment, the gas permeable seals are made of materials that are compatible with the content of the syringe. Examples of these materials include but are not limited to silicone-polyethylene, silicone-acrylic or silicone-polyurethane copolymers, among others, which may be formed into the shape of the gas permeable sealing components disclosed herein.

In one embodiment, a gas sterilizable syringe may be filled at the end cap side of the enclosure. Filling via the end cap side of the enclosure may also be referred to as cap side filling. In one embodiment, a flowable material (e.g., a silicone-based topical skin adhesive) is loaded into the syringe via one or more dispensing openings located at the distal end of an enclosure (e.g., the distal end of a double barrel syringe).

In one embodiment of a filling method, a non-vented double barrel plunger may be fully inserted into a double barrel syringe so that the plunger heads are located at the closed distal ends of the syringe barrels. In one embodiment, flowable material is directed into the dispensing openings located at the distal ends of the syringe barrels. As the flowable material fills the fluid chambers of the syringe barrels, the fluid pressure created by the flowable material forces the non-vented plungers to move (i.e., retract) toward the proximal end of the syringe. The final resting position of the non-vented double barrel plunger is dependent upon the volume of the flowable material that is directed into the fluid chambers of the syringe barrels. The above-described cap side filling method results in a minimal volume of ambient air being entrapped within the syringe barrels. Once the syringe has been filled to the pre-selected fill volume, the end cap having one or more end cap openings and the gas permeable barrier disposed within the end cap are secured to the distal ends of the syringe barrels.

During a sterilization procedure, sterilization gases preferably pass through the end cap openings in the end cap, through the gas permeable barrier, through the dispensing openings and into the fluid chambers of the respective syringe barrels for sterilizing the inside of the syringe barrels, the fluid chambers, and the flowable material disposed within the syringe barrels.

In one embodiment of a filling method, cap side syringe filling may be achieved by first placing pistons at the closed distal ends of syringe barrels of a double barrel syringe. Vented plungers are preferably advanced into the open proximal ends of the syringe barrels and moved toward the closed distal ends of the syringe barrels until the distal ends of the plungers engage the pistons. The pistons create the appropriate seal with the inner surfaces of the syringe barrels and the vented plungers are free to be inserted behind the pistons. As the flowable material fills the fluid chambers of the syringe barrels, the fluid pressure created when the formulation enters the syringe from the cap side will force the pistons and the vented plungers to move proximally (i.e., retract) to a final resting position that is dependent upon the pre-selected volume of the flow material that is introduced into the syringes. The greater the volume of the flowable material loaded into the respective syringe barrels, the greater the proximal movement of the plungers relative to the distal ends of the syringe barrels. Once the pre-selected volume for the flowable material is achieved (i.e., post filling), the end cap having one or more end cap openings and the gas permeable barrier are secured to the distal end of the syringe barrels.

During a sterilization procedure, sterilization gases preferably pass through the end cap openings in the end cap, through the gas permeable barrier, through the dispensing openings and into the fluid chambers of the syringe barrels for sterilizing the inside of the syringe barrels and the content of the syringe barrels.

For gas sterilizable syringes having vented plungers, the sterilizing gases will preferably flow through the vented plungers and into the fluid chambers of the enclosure (e.g., double barrel syringe) for sterilizing the fluid chambers and the flowable material disposed within the fluid chambers.

In one embodiment, a gas sterilizable syringe may be filled via the proximal, open ends of the syringe barrels. This methodology may be referred to as plunger-side filling. In one embodiment, during a plunger side filling method, prior to filling the syringe barrels with a flowable material, the end cap having one or more end cap openings and the gas permeable barrier are secured to the end cap side of the syringe barrels. In one embodiment, the flowable material is directed into the open ends of the syringe barrels for filling the syringe barrels via the open plunger side. Next, plungers are inserted into the open ends of the syringe barrels to create appropriate seals for the flowable material that fills the syringe barrels. The compressible plungers are utilized for exhausting any ambient air that remains entrapped within the filled syringe barrels.

In one embodiment, plunger-side filling methods may be used to improve efficiency and minimize costs. In one embodiment, plunger side filling enables high throughput, automated filling equipment to be utilized for filling syringes with the flowable material. Plunger side filling methods also allow for the use of syringes having the same external dimensional footprint, whereby the system may be controlled to provide for multiple, different, fill volumes. Utilizing syringes having the same external footprint will also enable operators to use a single, shared blister package for syringes having different fill volumes.

In one embodiment, to achieve the above-noted efficiencies, a plunger-side syringe filling method uses a non-vented plunger that is inserted into a vented syringe barrel having a one-way plunger stop element and precisely placed syringe barrel air vent holes. With a known fill volume that does not exceed the available fill capacity of the syringe, the one-way plunger stop element and the syringe barrel air vent holes are positioned to allow ambient air to be effectively exhausted from the filled syringe barrels.

In one embodiment, after plunger side filling of the syringe barrels, the plungers are advanced distally toward the distal ends of the syringe barrels to evacuate air from the syringe barrels via the syringe barrel air vent holes. After the plunger heads have effectively evacuated air from the syringe barrels, and dependent upon the pre-selected volume of the flowable material that is used to fill the syringe barrels, the syringe barrel air vent holes are preferably positioned along the lengths of the respective syringe barrels at locations that are proximal to the leading edges of the plunger heads.

In one embodiment, the syringe barrel air vent holes also preferably serve a secondary function of aiding in the sterilization process by creating additional pathways for sterilization gas ingress and egress to and from the syringe barrels to sterilize the flowable material contained within the syringe barrels.

In one embodiment, the one-way plunger stop element is preferably formed into the inner diameter of the syringe barrels in such a way that upon plunger insertion into a filled syringe, the plunger may easily ride over the one-way plunger stop element when moving distally, but once the plunger head has moved distally beyond the one-way plunger stop element, the plunger has a structure (e.g., radially extending projections) that contacts the one-way plunger stop element to prevent the plunger from being easily retracted toward the proximal end of the syringe. In one embodiment, an excessive force is required to retract the plunger once the plunger head has moved beyond the one way plunger stop element.

Placing the syringe barrel vent holes at a precise location relative to the location of the one-way plunger stop element and dependent upon the volume of the flowable material used to fill the syringe barrels, ensures that the plunger head isolates the syringe barrel air vent holes from the flowable material disposed within the syringe barrels.

The precise locations of the syringe barrel air vent holes and the one-way stop may be modified within a standard set of syringe barrels to accommodate a multitude of unique fill volumes. Each unique fill volume preferably generates its own unique syringe design, however, all designs preferably fit within one universal set of syringe barrels having a constant outer dimension.

### BRIEF DESCRIPTION OF THE DRAWINGS

FIG. 1 is a schematic view of a gas sterilizable syringe including a syringe barrel having a distal end wall, a dispensing tip, and end cap, and a syringe plunger, the gas sterilizable syringe having a plurality of micro-apertures formed therein for enabling gas sterilization of the syringe, in accordance with one embodiment of the present patent application.
FIG. 2 shows a method of making a gas sterilizable syringe having a plurality of micro-apertures for enabling gas sterilization of the gas sterilizable syringe, in accordance with one embodiment of the present patent application.
FIG. 3A is a schematic view of a gas sterilizable syringe including a syringe barrel and a syringe plunger, the syringe barrel having a plurality of micro-apertures formed therein for enabling gas sterilization of the gas sterilizable syringe, in accordance with one embodiment of the present patent application.
FIG. 3B shows the gas sterilizable syringe of FIG. 3A with an end cap removed, and the syringe plunger moved to a location that is distal to the location of the syringe plunger shown in FIG. 3A.
FIG. 4 is a cross-sectional view of a syringe barrel of a gas sterilizable syringe, the syringe barrel including an outer wall having a plurality of blind micro-apertures formed therein for enabling gas sterilization of the gas sterilizable syringe while preventing a viscous, flowable material from leaking through the blind micro-apertures, in accordance with one embodiment of the present patent application.
FIG. 5A is a schematic view of a gas sterilizable syringe including a syringe barrel having a plurality of micro-apertures formed therein for enabling gas sterilization of the gas sterilizable syringe, the gas sterilizable syringe including a protective sleeve that is slidable over an outer surface of the syringe barrel for selectively covering the plurality of micro-apertures, in accordance with one embodiment of the present patent application.
FIG. 5B shows the gas sterilizable syringe of FIG. 5A with the protective sleeve in an extended position for covering the plurality of micro-apertures formed in the syringe barrel, in accordance with one embodiment of the present patent application.
FIG. 6 is a schematic view of a gas sterilizable syringe including a syringe barrel and a dispensing tip having a plurality of micro-apertures formed therein, the gas sterilizable syringe including a first microporous sleeve covering the micro-apertures formed in the syringe barrel and a second microporous sleeve covering the micro-apertures formed in the dispensing tip, in accordance with one embodiment of the present patent application.
FIG. 7 is a schematic view of a gas sterilizable syringe including a syringe barrel and a dispensing tip having a plurality of micro-apertures formed therein, and microporous sleeves covering inner surfaces of the syringe barrel and the dispensing tip, in accordance with one embodiment of the present patent application.
FIG. 8 is a schematic view of a gas sterilizable syringe including a syringe barrel, a distal end wall partially closing a distal end of the syringe barrel, a dispensing tip, an end cap covering a distal end of the dispensing tip, and a syringe plunger having a piston, the gas sterilizable syringe including a plurality of micro-apertures formed in the syringe barrel, the distal end wall, the end cap, and the piston that are covered by respective microporous sleeves, in accordance with one embodiment of the present patent application.
FIG. 9 is a schematic view of a gas sterilizable syringe system including a first syringe barrel having a plurality of micro-apertures formed therein and a second syringe barrel having a plurality of micro-apertures formed therein for enabling gas sterilization of the first and second syringe barrels, in accordance with one embodiment of the present patent application.
FIG. 10A is a schematic view of a subassembly of a gas sterilizable syringe including a syringe barrel having a distal end wall, a dispensing tip, and end cap, and a syringe plunger, the gas sterilizable syringe having a plurality of apertures formed therein, in accordance with one embodiment of the present patent application.
FIG. 10B is a schematic view of a gas sterilizable syringe including the subassembly of FIG. 10A, the gas sterilizable syringe having gas permeable barriers filling the apertures for enabling gas sterilization of the gas sterilizable syringe, in accordance with one embodiment of the present patent application.
FIG. 11A is a schematic view of a subassembly of a gas sterilizable syringe including a syringe barrel having a plurality of apertures formed therein, in accordance with one embodiment of the present patent application.
FIG. 11B shows the syringe barrel of FIG. 11A having gas permeable barriers filling the apertures for enabling gas sterilization of a gas sterilizable syringe, in accordance with one embodiment of the present patent application.
FIG. 12 illustrates a method of making a gas sterilizable syringe having apertures and gas permeable barriers that fill the apertures for enabling gas sterilization of the gas sterilizable syringe, in accordance with one embodiment of the present patent application.
FIG. 13 is an exploded view of a gas sterilizable syringe including a double barrel syringe, a double barrel plunger, an end cap having end cap openings, and a gas permeable barrier, in accordance with one embodiment of the present patent application.
FIG. 14 shows a top view of the gas permeable barrier shown in FIG. 13.
FIG. 15 shows a distal end view of a gas sterilizable syringe including a double barrel syringe, an end cap having end cap openings secured to a distal end of the double barrel syringe, and a gas permeable barrier disposed within the end cap, in accordance with one embodiment of the present patent application.
FIG. 16A is an exploded view of a gas sterilizable syringe including a double barrel syringe, a double barrel plunger having first and second plungers, plunger seals secured to the distal ends of the respective first and second plungers, and an end cap securable over the distal end of the double barrel syringe, in accordance with one embodiment of the present patent application.
FIG. 16B shows a side view of the gas sterilizable syringe shown in FIG. 16A after the double barrel syringe, the double barrel plunger having the plunger seals secured to the distal ends of the respective first and second plungers, and the end cap have been assembly together, and with the syringe barrels filled with a flowable material.
FIG. 17 is an exploded view of a gas sterilizable syringe including a double barrel syringe, an end cap having end cap openings, a gas permeable barrier located at a distal end of the double barrel syringe, a double barrel plunger having first and second plungers insertable into the double barrel syringe, first and second gas permeable plunger seals securable to the distal ends of the respective first and second plungers, and a mixing and dispensing tip, in accordance with one embodiment of the present patent application.
FIG. 18A is a perspective view of a gas sterilizable syringe including a double barrel syringe, a double barrel plunger, and an end cap having end cap openings, in accordance with one embodiment of the present patent application.
FIG. 18B is a front view of the gas sterilizable syringe shown in FIG. 18A.
FIG. 19 is an exploded view of the gas sterilizable syringe shown in FIGS. 18A and 18B including the double barrel syringe, the double barrel plunger, the end cap having end cap openings, and a gas permeable barrier configured for being disposed within the end cap, in accordance with one embodiment of the present patent application.
FIG. 20 is another exploded view of the gas sterilizable syringe shown in FIG. 19.
FIG. 21A is a perspective view of a distal end of the double barrel plunger shown in FIG. 19, the double barrel plunger including a first plunger and a second plunger.
FIG. 21B is a front view of the double barrel plunger shown in FIG. 21A.
FIG. 22A is a magnified view of the distal ends of the first and second plungers of the double barrel plunger shown in FIG. 21B.
FIG. 22B is a perspective view of the distal ends of the first and second plungers of the double barrel plunger shown in FIGS. 21A and 22A.
FIG. 23 is a cross-sectional view of the double barrel syringe shown in FIG. 19, the double barrel syringe including a first syringe barrel and a second syringe barrel.
FIG. 24 is a magnified view of a midsection of the first syringe barrel shown in FIG. 23.
FIG. 25 is a cross-sectional view of the double barrel syringe shown in FIG. 19.
FIG. 26 is a cross-sectional view of the double barrel plunger shown in FIG. 19.
FIG. 27 is a cross-sectional view of the gas sterilizable syringe shown in FIG. 18B.
FIG. 28A is a magnified view of a midsection of the gas sterilizable syringe shown in FIG. 18B.
FIG. 28B is a cross-sectional view of a midsection of a first syringe barrel of a gas sterilizable syringe, in accordance with one embodiment of the present patent application.
FIG. 29A is a perspective view of the end cap of the gas sterilizable syringe shown in FIGS. 18A and 18B.
FIG. 29B is another perspective view of the end cap shown in FIG. 29B.
FIG. 29C is a front view of the end cap shown in FIGS. 29A and 29B.
FIG. 29D is a top view of the end cap shown in FIGS. 29A-29C.
FIG. 30 is a cross-sectional view of the end cap and the gas permeable barrier shown in FIG. 19.
FIG. 31 shows the end cap of FIG. 30 with the gas permeable barrier disposed inside the end cap, in accordance with one embodiment of the present patent application.
FIG. 32 shows a cross-sectional view of a distal end of a gas sterilizable syringe including a double barrel syringe, a double barrel plunger, an end cap secured to the distal end of the double barrel syringe, and a gas permeable barrier, in accordance with one embodiment of the present patent application.
FIG. 33 shows a top view of a subassembly of a gas sterilizable syringe including a double barrel syringe and a double barrel plunger, in accordance with one embodiment of the present patent application.
FIG. 34A shows a perspective view of a multi-function connector that is secured over the distal end of the double barrel syringe shown in FIG. 33, in accordance with one embodiment of the present patent application.
FIG. 34B shows a distal end view of the multi-function connector shown in FIG. 34A.
FIG. 35 shows an exploded view of the multi-function connector of FIGS. 34A and 34B, first and second gas permeable barriers that are insertable into D-shaped openings of the multi-function connector, and an end cap securable to an externally threaded post of the multi-function connector, in accordance with one embodiment of the present patent application.
FIG. 36 shows the end cap of FIG. 35 secured over the externally threaded post of the multi-function connector of FIG. 35.
FIG. 37 shows a perspective view of a plurality of gas sterilizable syringes, each syringe having the multi-function connector and the end cap of FIG. 36 secured to a distal end thereof.
FIG. 38 shows a side view of a mixing and dispensing tip of a gas sterilizable syringe, in accordance with one embodiment of the present patent application.
FIG. 39 shows the mixing and dispensing tip of FIG. 38 secured to a distal end of a gas sterilizable syringe, in accordance with one embodiment of the present patent application.
FIG. 40 shows a distal end view of a multi-function connector for a gas permeable syringe, the multi-function connector having first and second D-shaped openings that are adapted to receive gas permeable barriers, in accordance with one embodiment of the present patent application.
FIG. 41 shows a distal end view of a prior art connector for a double barrel syringe.

### DETAILED DESCRIPTION OF PREFERRED EMBODIMENTS

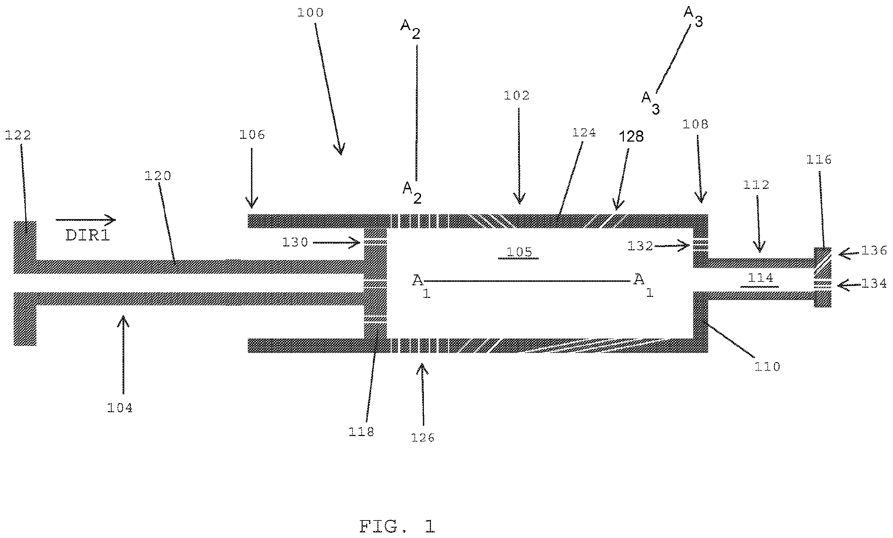

Referring to FIG. 1, in one embodiment, a gas sterilizable syringe 100 preferably includes a syringe barrel 102 and a syringe plunger 104 that is configured for being assembled with the syringe barrel. The syringe barrel 102 preferably has a fluid chamber 105 that is configured to receive a flowable material such as a precursor or component of a tissue adhesive. In one embodiment, the flowable material is a viscous, flowable material having a relatively high viscosity of about 1,000-100,000 centipoise, more preferably about 2,000-75,000 centipoise, and even more preferably about 30,000-60,000 centipoise. In one embodiment, the syringe plunger 104 may be depressed in the distal direction DIR1 for dispensing the viscous, flowable material. The gas sterilizable syringe 100 may be made of a variety of materials including polymeric materials, glass, and rubber, and/or a combination of the afore-mentioned materials.

In one embodiment, the viscous, flowable material may comprise or be a precursor of a two-part silicone adhesive formulation as disclosed in U.S. Patent Application Ser. No. 16/885,361, filed on May 28, 2020 (Attorney Docket No. ETH6070USNP1), U.S. Patent Application Ser. No. 17/327,940, filed on May 24, 2021 (Attorney Docket No. ETH6070USCIP1), U.S. Patent Application Ser. No. 16/885,366, filed on May 28, 2020 (Attorney Docket No. ETH6084USNP1), and U.S. Patent Application Ser. No. 17/327,952, filed on May 24, 2021 (Attorney Docket No. ETH6084USCIP1), the disclosures of which are hereby incorporated by reference herein.

In one embodiment, the syringe barrel 102 preferably has a proximal end 106 that is open and distal end 108 that is partially closed by a distal end wall 110. The gas sterilizable syringe 100 preferably includes a dispensing tip 112 that projects distally from the distal end wall 110. The dispensing tip 112 has a conduit 114 that provides a flow path for dispensing the flowable material that is disposed within the fluid chamber 105 of the syringe barrel 102. An opening at the distal end of the conduit 114 may be covered by an end cap 116. The end cap 116 may cover the distal end of the dispensing tip 112 during shipment and storage. The end cap 116 may be removed from the distal end of the dispensing tip 112 for dispensing the flowable material.

In one embodiment, the syringe plunger 104 preferably includes a piston 118 that is disposed inside the fluid chamber 105 of the syringe barrel 102. The piston 118 preferably has an outer perimeter that engages an inner surface of the syringe barrel 102 to form a seal between the piston and the inner surface of the syringe barrel. The piston 118 may be made of rubber or polymeric material. The plunger 104 preferably includes a plunger stem 120 having a distal end that is secured to a proximal side of the piston 118. In one embodiment, the plunger 104 preferably includes a thumb tab 122 that is desirably secured to a proximal end of the plunger stem 120. The thumb tab 122 may be depressed in the distal direction designated DIR1 for forcing the piston 118 to slide distally toward the distal end wall 110 of the syringe barrel 102. In one embodiment, with the end cap 116 removed, as the thumb tab 122 is depressed in the distal direction DIR1, a flowable material that has been pre-loaded into the fluid chamber 105 of the syringe barrel 102 may be forced to flow downstream into the conduit 114 of the dispensing tip 110 for being dispensed from an opening at the distal end of the dispensing tip 112.

In one embodiment, the syringe barrel 102 desirably has an outer wall 124 and a plurality of micro-apertures 126 may be formed (e.g., drilled, laser drilled, mechanically drilled) in the outer wall 124 of the syringe barrel 102. The micro-apertures 126 may be formed by using a laser device that laser drills the small openings. In one embodiment, the syringe barrel 102 has a length that extends along a longitudinal axis A₁ and the micro-apertures 126 may extend along an axis A₂ that is perpendicular to the longitudinal axis A₁ of the syringe barrel 102. In one embodiment, one or more micro-apertures 128 may extend through the outer wall 124 of the syringe barrel 102 along an axis A₃ that is diagonal to and/or not perpendicular with the longitudinal axis A₁.

In one embodiment, one or more micro-apertures 130 may extend through the piston 118 of the syringe plunger 104.

In one embodiment, one or more micro-apertures 132 may be formed in the distal end wall 110 of the syringe barrel 102. The micro-apertures 132 formed in the distal end wall 110 may extend along respective axes that are parallel with the longitudinal axis A₁ of the syringe barrel 102. In other embodiments, however, the micro-apertures 132 formed in the distal end wall 110 may extend along respective axes that define an angle relative to the longitudinal axis A₁ of the syringe barrel 102.

In one embodiment, one or more of the micro-apertures 130 extending through the piston 118 may be positioned within and/or aligned with the plunger stem 120 of the plunger 104 that extends between the thumb tab 122 and the piston 118.

In one embodiment, one or more micro-apertures 134 may be formed in the end cap 116 that is secured over the distal end of the dispensing tip 112. In one embodiment, the end cap 116 may have micro-apertures 134 that extend along respective axes that are parallel with the longitudinal axis A₁ of the syringe barrel 102. In one embodiment, the end cap 116 may have one or more micro-apertures 136 that extend along respective axes that are non-parallel (e.g., diagonal) with the longitudinal axis A₁ of the syringe barrel 102.

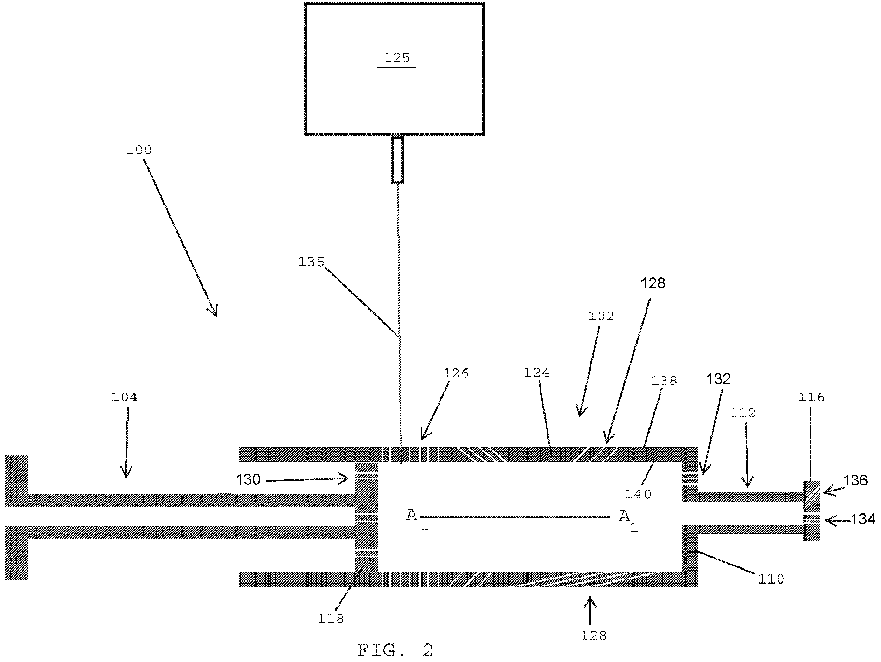

The micro-apertures disclosed herein may be formed using a variety of techniques and methodologies. Referring to FIG. 2, in one embodiment, a laser device 125 may be utilized for forming the micro-apertures 126 in the outer wall 124 of the syringe barrel 102. **In** one embodiment, the laser device 125 preferably generates laser light 135 that contacts the outer wall 124 of the syringe barrel 102 for laser drilling the micro-apertures 126, which extend completely through the outer wall 124 (i.e., from an outer surface 138 to an inner surface 140 of the outer wall 124). In one embodiment, the laser device 125 may be configured for forming one or more micro-apertures that are perpendicular to the longitudinal axis A₁ of the syringe barrel. The laser device 125 may also be programmed for forming one or more micro-apertures 128 that are parallel, diagonal and/or non-perpendicular to the longitudinal axis A₁ of the syringe barrel 102.

In one embodiment, the laser device 125 may be utilized for forming one or more micro-apertures 130 in the piston 118 of the plunger 104, one or more micro-apertures 132 in the distal end wall 110 of the syringe barrel 102, and/or one or more micro-apertures 134, 136, in the end cap 116 that is secured over the distal end of the dispensing tip 112. The laser device may also be used for laser drilling micro-apertures in the dispensing tip 112.

Other methods may be used for forming micro-apertures in one or more components of a gas sterilizable syringe including using mechanical drills, using heated probes, and using water jets.

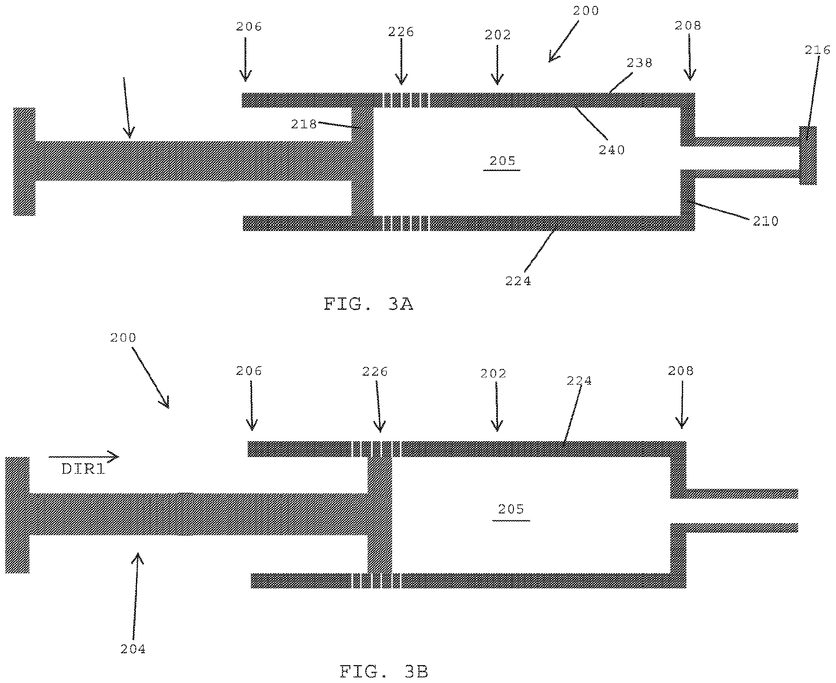

Referring to FIG. 3A, in one embodiment, a syringe 200 preferably includes a syringe barrel 202 having an outer wall 224 with an outer surface 238 and an inner surface 240. The syringe barrel 202 preferably has a proximal end 206 that is open and a distal end 208 that is at least partially closed by a distal end wall 210. The outer wall 224 of the syringe barrel 202 preferably defines and/or surrounds a fluid chamber 205 that is configured to receive a viscous, flowable material (not shown). The syringe 200 has a distal end wall 210 and a dispensing tip 212 that projects from the distal end wall 210. A distal end of the dispensing tip 212 is covered by an end cap 216 that may be removed for dispensing the viscous, flowable material.

In one embodiment, the syringe barrel 202 preferably has one or more micro-apertures 226 that are formed in the outer wall 224 of the syringe barrel 202 and that extend from the outer surface 238 to the inner surface 240 of the outer wall 224. In one embodiment, the one or more micro-apertures 226 are closer to the proximal end 206 of the syringe barrel 202 than the distal end 208 of the syringe barrel.

Referring to FIGS. 3A and 3B, in one embodiment, as a result of positioning the one or more micro-apertures 226 closer to the proximal end 206 than the distal end 208 of the outer wall 226 of the syringe barrel 202, the opportunity for the viscous, flowable material to leak through the one or more micro-apertures 226 as the plunger 204 is depressed in the distal direction DIR1 is greatly minimized. As shown in FIGS. 3A and 3B, at the start of a procedure for dispensing a flowable material (i.e., the position of the plunger 204 shown in FIG. 3A), after the end cap 216 (FIG. 3A) has been removed from the distal end of the dispensing tip 212, the piston 218 is located near the proximal end 206 of the outer wall 224 of the syringe barrel 202. As the syringe plunger 204 is depressed to start a dispensing procedure, the piston 218 moves distally (DIR1) beyond the one or more micro-apertures 226 formed in the syringe barrel. As a result, as a dispensing operation progresses, the piston 218 moves beyond the micro-apertures 226 and advances distally into a section of the outer wall 224 that has no micro-apertures, thereby minimizing the likelihood that the viscous, flowable material will be able to leak through the micro-apertures while providing for a continuous expression of the flowable material.

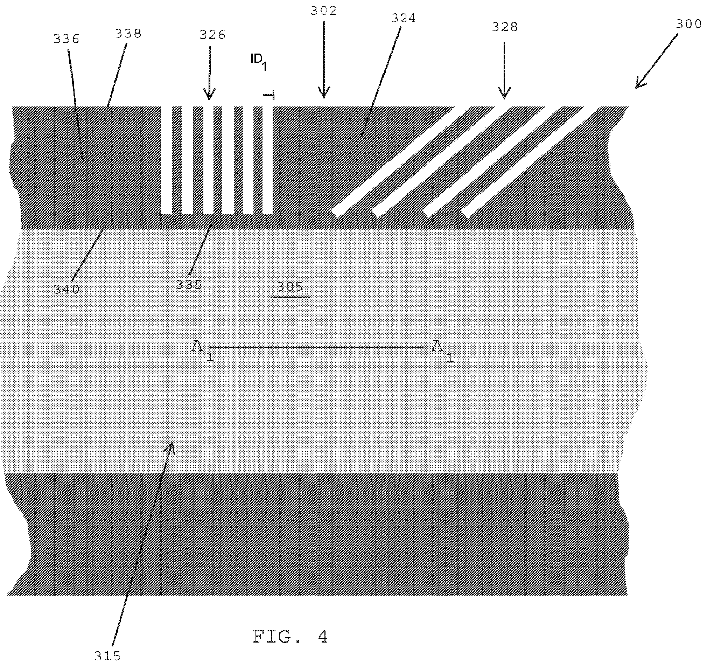

Referring to FIG. 4, in one embodiment, a gas sterilizable syringe 300 preferably includes a syringe barrel 302 having an outer wall 324 that extends along the longitudinal axis A₁ of the syringe barrel 302. The outer wall 324 of the syringe barrel 302 preferably includes an outer surface 338 and an inner surface 340 that defines a fluid chamber 305 that is adapted to contain a viscous, flowable material 315 that is dispensed during operation of the syringe 300. In one embodiment, the syringe 300 preferably includes one or more micro-apertures 326 having blind openings that are formed in the outer wall 324 of the syringe barrel 302. The blind openings of the micro-apertures 326 do not extend completely through the outer wall 324 and do not reach the inner surface 340 of the outer wall 324, thereby leaving a thin membrane 335 formed of the outer wall 324 that extends between the closed ends of the blind openings of the micro-apertures and the inner surface 340 of the outer wall 324 of the syringe barrel 302.

In one embodiment, the micro-apertures 326, 328 preferably have inner diameters ID₁ of about 0.1 microns to about 25 microns, and more preferably about one (1) micron. In one embodiment, the viscous, flowable material 315 disposed within the fluid chamber 305 preferably has a viscosity of about 1,000-100,000 centipoise, more preferably about 2,000-75,000 centipoise, and even more preferably about 30,000-60,000 centipoise. As a result, a sterilization gas (e.g., ethylene oxide) is able to pass through the micro-apertures 326, 328 and the membrane 335 for sterilizing inside the fluid chamber 305, however, during a dispensing operation, when the flowable material 315 is under positive pressure, the higher viscosity of the flowable material 315 prevents the flowable material 315 from leaking through the membrane 335 or out of the micro-apertures 326, 328.

In one embodiment, a first group of the micro-apertures 326 having blind openings may extend along respective axes that are perpendicular to the longitudinal axis A₁ of the syringe barrel 302.

In one embodiment, a second group of the micro-apertures 328 having blind openings may extend along respective axes that are diagonal and/or not perpendicular to the longitudinal axis A₁ of the syringe barrel 302.

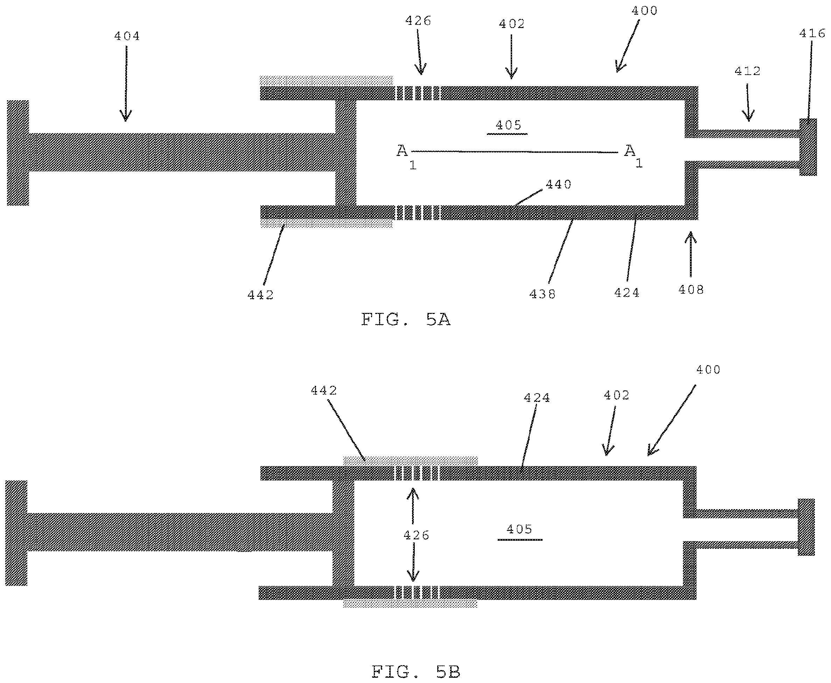

Referring to FIG. 5A, in one embodiment, a gas sterilizable syringe 400 preferably includes a protective sleeve 442 that may be moved over a syringe barrel between a retracted position (FIG. 5A) for sterilizing the syringe and a viscous, flowable material loaded therein, and an extended position (FIG. 5B) for dispensing the viscous, flowable material. In one embodiment, the gas sterilizable syringe 400 desirably includes a syringe barrel 402, a plunger 404, a dispensing tip 412 projecting from the distal end 408 of the syringe barrel 402, and an end cap 416 that covers an opening at the distal end of the dispensing tip 412. In one embodiment, the syringe 400 preferably includes one or more micro-apertures 426 that are formed in the outer wall 424 of the syringe barrel 402 and that extend from the outer surface 438 to the inner surface 440 of the syringe barrel 402. The one or more micro-apertures 426 may have axes that are perpendicular to and/or non-perpendicular (e.g., extend diagonally) relative to the longitudinal axis A₁ of the syringe barrel 402.

Referring to FIGS. 5A and 5B, in one embodiment, the protective sleeve 442, which may be made of a non-porous material, is adapted to slide over the outer surface 438 of the outer wall 424 of the syringe barrel 402, between the retracted position shown in FIG. 5A and the extended position shown in FIG. 5B. In the retracted position shown in FIG. 5A, the protective sleeve 442 does not cover the one or more micro-apertures 426 so that a sterilization gas (e.g., ethylene oxide) may be utilized for sterilizing the syringe 400 and a viscous, flowable material (not shown) disposed within the fluid chamber 405 of the syringe barrel 402. With the protective sleeve 442 retracted (FIG. 5A), the sterilization gases are able to pass through the micro-apertures 426 for sterilizing the syringe 400 including the viscous, flowable material within the fluid chamber 405.

In the position shown in FIG. 5B, the protective sleeve 442 has been moved into the extended position for covering the one or more micro-apertures 426 that are formed in the outer wall 424 of the syringe barrel 402. In one embodiment, the protective sleeve 442 is provided on the outer wall 424 of the syringe barrel 402 so that it can slide over the one or more micro-apertures 426 after sterilization and before dispensing the flowable material to seal the micro-apertures 426 to prevent the flowable material from leaking through the micro-apertures as it is being dispensed from the syringe 400. When the flowable material (not shown) is under positive pressure during a dispensing operation, the extended protective sleeve 442 (FIG. 5B) blocks the flowable material from leaking out of the micro-apertures 426.

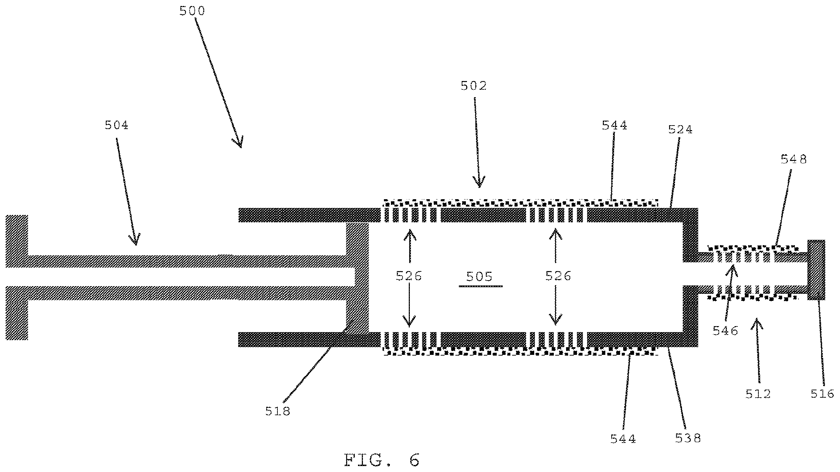

Referring to FIG. 6, in one embodiment, a gas sterilizable syringe 500 preferably includes a syringe barrel 502 that defines a fluid chamber 505 adapted to receive a flowable material (e.g., a precursor of a tissue adhesive), a plunger 504 having a piston 518, a dispensing tip 512, and an end cap 516 that covers a dispensing opening at the distal end of the dispensing tip 512. In one embodiment, a plurality of micro-apertures 526 are formed in the outer wall 524 of the syringe barrel 502. A gas permeable barrier, such as a microporous sleeve 544 (e.g., a TYVEK^{®} sleeve), which is not permeable to the flowable material stored within the fluid chamber 505 of the syringe barrel 502, but which is permeable to sterilization gases (e.g., ethylene oxide) may be provided over the outer surface 538 of the outer wall 524 of the syringe barrel 502 to cover the micro-apertures 526 formed in the outer wall 524 of the syringe barrel.

In one embodiment, the gas sterilizable syringe 500 may include additional micro-apertures formed in other locations that are covered by one or more microporous sleeves. In one embodiment, the dispensing tip 512 of the syringe 500 may have one or more micro-apertures 546 formed therein that are covered by a dispensing tip microporous sleeve 548, which is similar in construction to the microporous sleeve 544 that covers the micro-apertures 526 formed in the syringe barrel 502.

The dispensing tip microporous sleeve 548 (e.g., a TYVEK^{®} sleeve) is not permeable to the flowable material stored within the fluid chamber 505 of the syringe barrel 502 (to prevent leakage), but is permeable to sterilization gases (e.g., ethylene oxide). The dispensing tip microporous sleeve 548 may be wrapped around an outer surface of the dispensing tip 512 for covering the micro-apertures 526 formed in the outer wall of the dispensing tip. In one embodiment, a microporous material may line an inner surface of the dispensing tip.

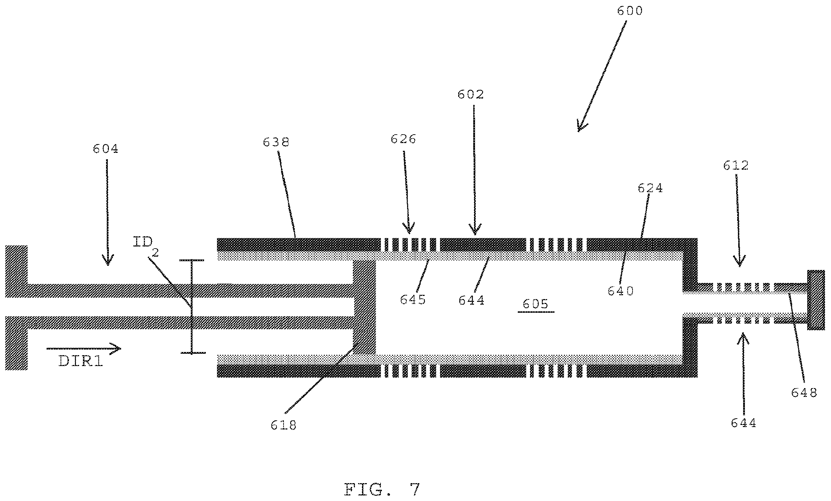

Referring to FIG. 7, in one embodiment, a gas sterilizable syringe 600 preferably includes a syringe barrel 602, a plunger 604, and a dispensing tip 612 that projects from a distal end of syringe barrel 602. In one embodiment, the syringe 600 preferably includes a plurality of micro-apertures 626 that are formed in the outer wall 624 of the syringe barrel 602 and that extend from the outer surface 638 to the inner surface 640 of the outer wall 624 of the syringe barrel 602. The micro-apertures 626 are preferably in fluid communication with the fluid chamber 605 of the syringe barrel 602. In one embodiment, a microporous sleeve 644 (e.g., a TYVEK^{®} sleeve) preferably lines the inner surface 640 of the outer wall 624 of the syringe barrel 602 to cover the micro-apertures 626 that extend through the outer wall 624 of the syringe barrel 602. The microporous sleeve 644 preferably defines an inner diameter ID₂ that enables the piston 618 of the plunger 604 to slide over an inner surface 645 of the microporous sleeve 644 while preferably maintaining a fluid-tight seal with the inner surface of the microporous sleeve 644. In one embodiment, when the syringe plunger 604 is depressed in the distal direction DIR1 for dispensing a viscous, flowable material via the dispensing tip 612, the piston 618 preferably slides over the inner surface 645 of the microporous sleeve 644.

In one embodiment, the dispensing tip 612 preferably includes a plurality of micro-apertures 644 formed in an outer wall of the dispensing tip 612, which are in fluid communication with the fluid chamber 605 of the syringe barrel 602. In one embodiment, a second microporous sleeve 648 preferably lines the inner surface of the dispensing tip 612 and covers the micro-apertures 644 formed in the dispensing tip 612. The second microporous sleeve 648 is not permeable to the flowable fluid stored within the fluid chamber 605 of the syringe barrel 602, however, the second microporous sleeve 644 is permeable to sterilizing gases (e.g., ethylene oxide) that may be utilized for sterilizing the syringe 600 and the flowable fluid disposed within the fluid chamber 605 of the syringe.

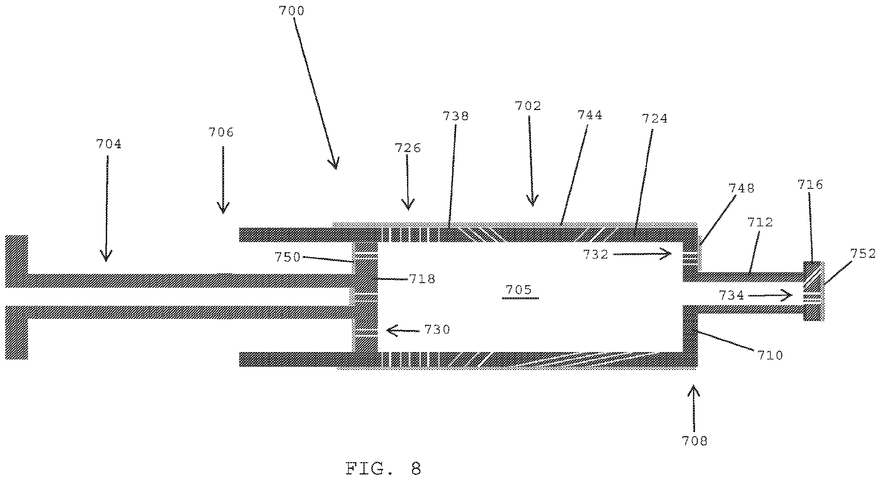

Referring to FIG. 8, in one embodiment, a gas sterilizable syringe 700 preferably includes a syringe barrel 702, a syringe plunger 704 including a piston 718, a dispensing tip 712 and an end cap 716 that selectively covers an opening at a distal end of dispensing tip 712. The syringe barrel 702 desirably has a proximal end 706 that is open for receiving the plunger 704 and a distal end 708 that is partially closed by a distal end wall 710.

In one embodiment, micro-apertures 726 are formed in the outer wall 724 of the syringe barrel 702. In one embodiment, a microporous sleeve 744 covers the outer surface 738 of the outer wall 724 of the syringe barrel 702 to cover the micro-apertures 726 formed in the outer wall 724 of the syringe barrel 702. The microporous sleeve 744 allows sterilizing gases to pass through the micro-apertures 726 for sterilizing the syringe 700 and a flowable material disposed therein while preventing the flowable material disposed within the fluid chamber 705 of the syringe barrel 702 from leaking through the micro-apertures 726.

In one embodiment, the distal end wall 710 preferably has one or more micro-apertures 732 formed therein. A microporous layer 748 preferably covers the micro-apertures 732 formed in the distal end wall 710. The microporous layer 748 allows sterilizing gases to pass through the micro-apertures 732 formed in the distal end wall 710 for sterilizing the syringe 700 and a flowable material disposed therein while preventing the flowable material disposed within the fluid chamber 705 of the syringe barrel 702 from leaking through the micro-apertures 732.

In one embodiment, the end cap 716 has one or more micro-apertures 734 formed therein and a microporous layer 752 covers the one or more micro-apertures 743 in the end cap 716. The microporous layer 752 allows sterilizing gases to pass through the micro-apertures 734 formed in the end cap 716 for sterilizing the syringe 700 and the flowable material while preventing flowable material disposed within the fluid chamber 705 of the syringe barrel 702 from leaking through the micro-apertures 734.

In one embodiment, the piston 718 of the syringe plunger 704 preferably has one or more micro-apertures 730 formed therein and a microporous layer 750 covers the one or more micro-apertures 730 formed in the piston 718. The microporous layer 750 allows sterilizing gases to pass through the micro-apertures 730 formed in the piston 718 for sterilizing the syringe 700 and the flowable material while preventing the flowable material disposed within the fluid chamber 705 of the syringe barrel 702 from leaking through the micro-apertures 730 of the piston 718.

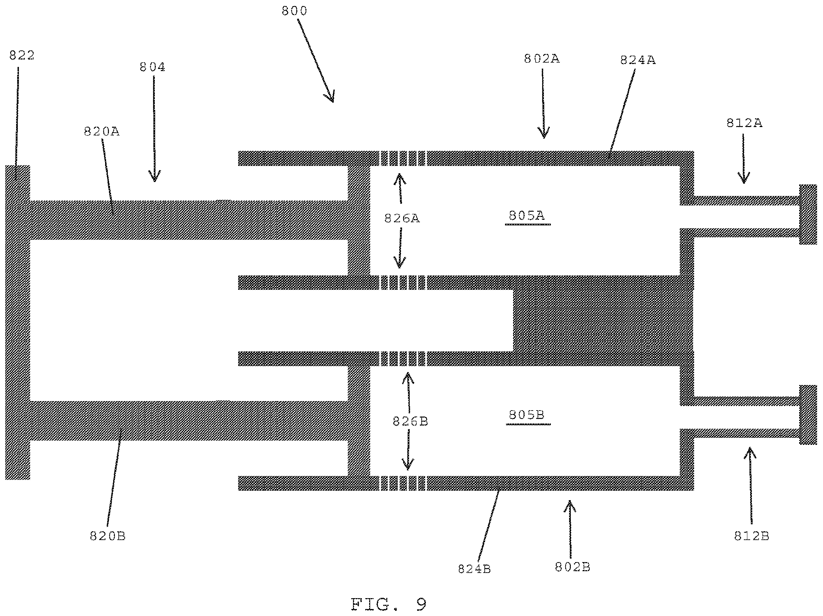

Referring to FIG. 9, in one embodiment, a syringe system 800 for dispensing precursors of a tissue adhesive may include a first syringe barrel 802A containing a first precursor of a tissue adhesive having a plurality of micro-apertures 826A formed therein that extend from an outer surface to an inner surface of an outer wall 824A of the first syringe barrel 802A.

In one embodiment, the system 800 preferably includes a second syringe barrel 802B containing a second precursor of a tissue adhesive having a plurality of micro-apertures 826B formed therein that extend from an outer surface to an inner surface of an outer wall 824B of the second syringe barrel 802B.

In one embodiment, the syringe system 800 preferably includes a plunger 804 that enables the two flowable materials disposed in the respective first and second syringe barrel 802A, 802B to be simultaneously and/or near simultaneously dispensed from the dispensing tips 812A, 812B of the respective first and second syringe barrels 802A, 802B. Upon being dispensed, the first and second precursors are preferably mixed and/or joined together to form a tissue adhesive.

In one embodiment, one or more of the gas sterilizable syringes disclosed herein may be sterilized by placing a syringe inside a pouch that is adapted to be used during a gas sterilization procedure. The pouch may have one or more non-porous panels (e.g., made of foil; made of a flexible polymer), and one or more porous panels (e.g., made of TYVEK^{®} material) that allow sterilization gases to pass therethrough. The pouches may be sealed for sealing the syringes inside the pouches. After sealing the pouches, sterilization gases may pass through the one or more porous panels of the sealed pouches for sterilizing the syringe(s) disposed inside the sealed pouch. After sterilization, the one or more porous panels prevent bacteria and contaminants from passing therethrough to maintain the sterile state of the syringe(s).

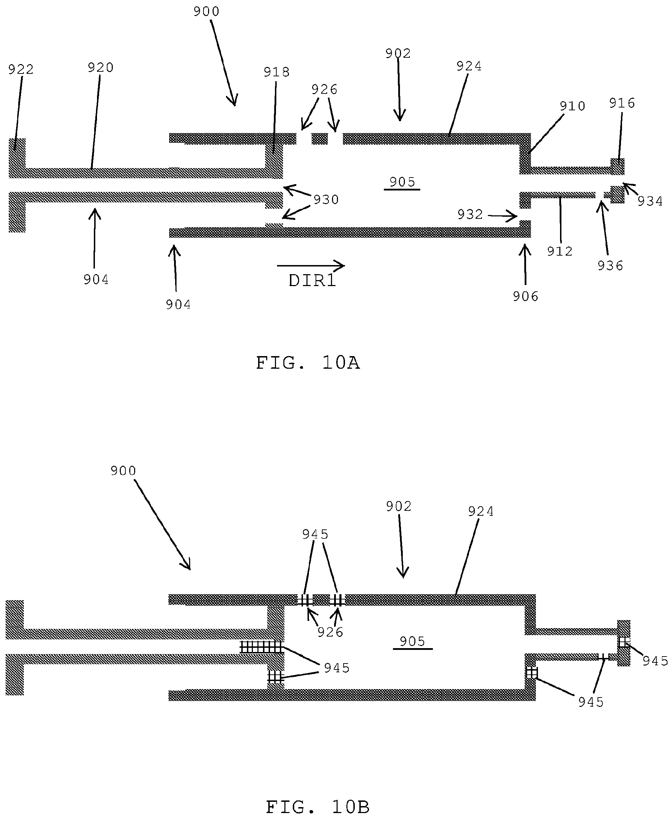

Referring to FIG. 10A, in one embodiment, a gas sterilizable enclosure 900 (e.g., a gas sterilizable syringe) preferably includes a syringe barrel 902 and a syringe plunger 904 that is configured for being assembled with the syringe barrel. The syringe barrel 902 preferably defines a fluid chamber 905 that is configured to receive a flowable material such as a precursor or a component of a tissue adhesive (e.g., a silicone-based topical skin adhesive).

In one embodiment, the syringe barrel 902 preferably has a proximal end 906 that is open and a distal end 908 that is at least partially closed by a distal end wall 910. The gas sterilizable syringe 900 preferably includes a dispensing tip 912 that projects distally from the distal end wall 910. The dispensing tip 912 has a conduit 914 that provides a flow path for dispensing a flowable material (not shown). The flowable material may be disposed within the fluid chamber 905 of the syringe barrel 902. A dispensing opening at the distal end of the conduit 914 of the dispensing tip 912 is preferably covered by an end cap 916. The end cap 916 may cover the distal end of the dispensing tip 912 during shipment and storage of the gas sterilizable syringe 900. In one embodiment, the end cap 916 may be removed from the distal end of the dispensing tip 912 for dispensing and/or expressing the flowable material from the distal end of the syringe barrel 902.

In one embodiment, the syringe plunger 904 preferably includes a piston 918 (e.g., a plunger head) that may be disposed inside the fluid chamber 905 of the syringe barrel 902. The piston 918 preferably has an outer perimeter that engages an inner surface of the syringe barrel 902 to form a fluid-tight seal between the piston and the inner surface of the syringe barrel. In one embodiment, the piston 918 may be made of a variety of conventional materials including rubber and/or a polymer. The plunger 904 preferably includes a plunger stem 920 having a distal end that is secured to a proximal side of the piston 918. In one embodiment, the plunger 904 preferably includes a thumb tab 922 that is desirably secured to a proximal end of the plunger stem 920. The thumb tab 922 may be depressed in the distal direction designated DIR1 for forcing the piston 918 to slide distally within the fluid chamber 905 and toward the distal end wall 910 of the syringe barrel 902. In one embodiment, with the end cap 916 removed, as the thumb tab 922 is depressed in the distal direction DIR1, a flowable material that has been pre-loaded into the fluid chamber 905 of the syringe barrel 902 may be forced to flow downstream into the conduit 914 of the dispensing tip 912 for being expressed from the dispensing opening at the distal end of the dispensing tip 912.

In one embodiment, the syringe barrel 902 has an outer wall 924, and the gas sterilizable syringe 900 may include one or more apertures 926 formed (e.g., drilled, lasered drilled, mechanically drilled) in the outer wall 924 of the syringe barrel 902.

In one embodiment, the gas sterilizable syringe 900 may include one or more apertures 930 that extend through the piston 918 of the syringe plunger 904. In one embodiment, the one or more of the apertures 930 extending through the piston 918 may be positioned within and/or aligned with the plunger stem 920 of the plunger 904 that extends between the thumb tab 922 and the piston 918. The plunger 904 may be a vented plunger.

In one embodiment, the gas sterilizable syringe 900 may include one or more apertures 932 formed in the distal end wall 910 of the syringe barrel 902.

In one embodiment, the gas sterilizable syringe 900 may include one or more apertures 934 formed in a wall of the end cap 916, which is securable over the dispensing opening located at the distal end of the dispensing tip 912.

In one embodiment, the gas sterilizable syringe 900 may include one or more apertures 936 extending through an outer wall of the dispensing tip 912.

In one embodiment, each of the apertures 926, 930, 932, 934, and 936 formed in the gas sterilizable syringe 900 preferably has a diameter of about 1-5mm.

In one embodiment, the apertures 926, 930, 932, 934, and 936 of the gas sterilizable syringe 900 are preferably filled with gas permeable barriers 945 (i.e., gas permeable plugs; gas permeable seals) that enable sterilization gases to pass through the apertures for sterilizing the gas sterilizable syringe 900 while preventing the viscous, flowable material disposed within the fluid chamber 905 of the syringe barrel 902 from passing (e.g., leaking) through the apertures 926, 930, 932, 934, and 936 that have been filled with the gas permeable barriers 945.

In one embodiment, the formulation used to form the gas permeable barriers is preferably porous, curable, and capable of reliably plugging and/or sealing the apertures while not affecting the stability of the flowable material (e.g., a silicone-based topical skin adhesive) stored within the enclosure (e.g., syringe).

In one embodiment, one or more of the apertures may have an asymmetric shape and/or cross-section, and the formulation used to form the gas permeable barriers is introduced into the apertures in an uncured state so that it may conform to the shape of the apertures prior to being cured. After being fully cured, the gas permeable barriers preferably conform to the shapes of the apertures to seal the apertures for enabling sterilization gases to pass therethrough while preventing the flowable material from passing through the gas permeable barriers. In one embodiment, the material (e.g., silicone rubber) used to form the gas permeable barriers preferably bonds to the material used to form the syringe

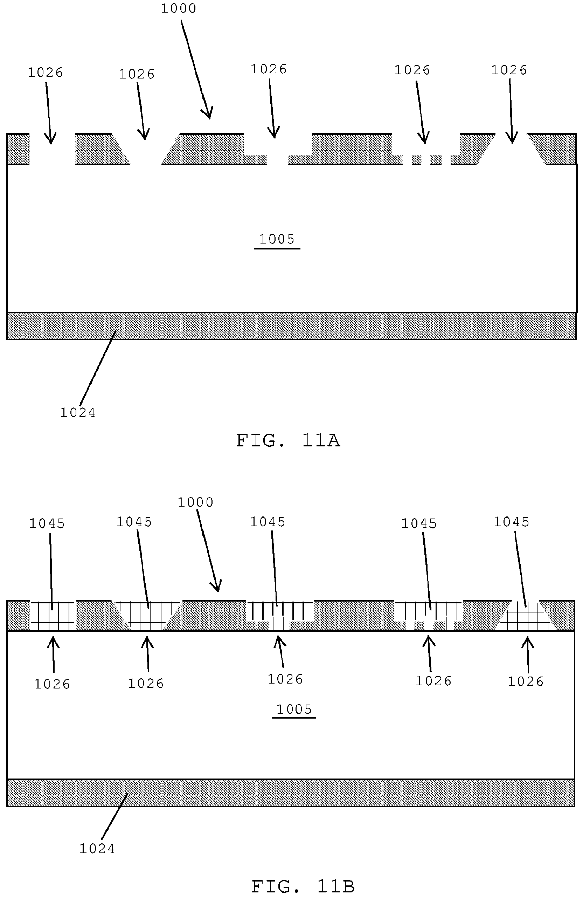

Referring to FIG. 11A, in one embodiment, a gas sterilizable syringe 1000 preferably includes a syringe barrel 1002 having an outer wall 1024 that surrounds a fluid chamber 1005. In one embodiment, a plurality of apertures 1026 may be formed in the outer wall 1024 of the syringe barrel 1002. The apertures 1026 may have different shapes, configurations and/or diameters. The apertures may have asymmetric and/or non-uniform shapes. The apertures 1026 may have a diameter of about 1-5mm.

Referring to FIG. 11B, in one embodiment, the respective apertures 1026 formed in the outer wall 1024 of the syringe barrel 1002 may be filled with gas permeable barriers 1045 (e.g., plugs) that enable sterilization gases to pass through the apertures 1026 for reaching the viscous, flowable material (not shown) disposed within the fluid chamber 1005, while preventing the viscous, flowable material from leaking and/or escaping through the plugged apertures 1026. In one embodiment, the porous material that fills the apertures 1026 to form the gas permeable barriers 1045 may initially fill the apertures when in an uncured, flowable state, and then be cured to form the gas permeable barriers. In the initial uncured state, the material used to form the gas permeable barriers 1045 preferably conforms to the particular shape of the aperture 1026 associated therewith. After being disposed within the respective apertures 1026 formed in the outer wall 1024 of the syringe barrel 1002, the gas permeable barrier material may be cured for forming structurally stable gas permeable barriers 1045 (e.g., plugs) that fill each of the respective apertures 1026 formed in the outer wall 1024 of the syringe barrel 1002 of the sterilizable syringe 1000. In one embodiment, the gas permeable barriers 1045 may be made of a porous silicone rubber that bonds with the one or more walls of the syringe and/or the syringe barrel 1002.

In one embodiment, the holes 926, 930, 932, 934 and 936 of the sterilizable syringe 900 shown and described above in FIGS. 10A and 10B may have shapes, configurations and/or diameters that are similar to the shapes, configurations, and/or diameters of the holes 1026 shown in FIGS. 11A and 11B.

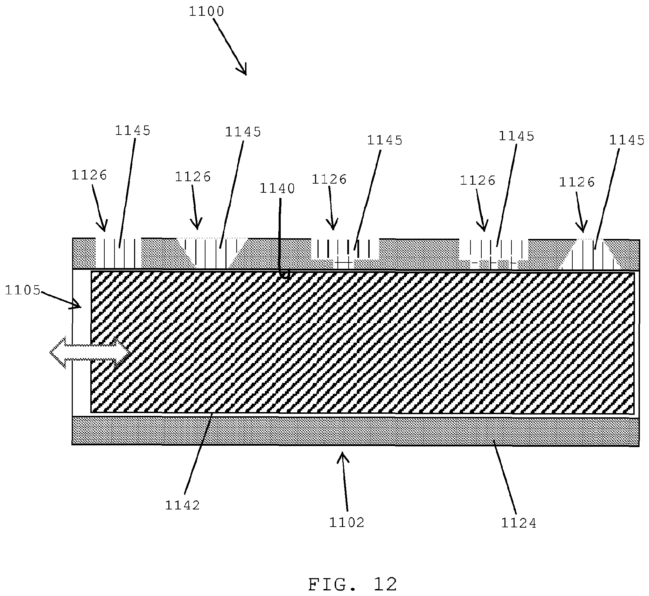

In one embodiment, a sacrificial sleeve may be used for forming and/or shaping the gas permeable barriers that fill the holes formed in a sterilizable syringe. Referring to FIG. 12, in one embodiment, a gas sterilizable syringe 1100 desirably includes a syringe barrel 1102 having an outer wall 1124 that surrounds a fluid chamber 1105, which is configured to receive a flowable material (e.g., a tissue adhesive). In one embodiment, one or more apertures 1126 are formed in the outer wall 1124 of the syringe barrel 1102 to provide communication with the fluid chamber 1105 of the syringe barrel 1102. In one embodiment, prior to filling the apertures 1126 with a material that may be cured to form gas permeable barriers 1145, a sacrificial sleeve 1142 (e.g., a tube-shaped structure) may be inserted into the syringe barrel 1102. An outer surface of the sacrificial sleeve 1142 may engage or be located closely opposite an inner surface 1140 of the outer wall 1124 of the syringe barrel 1102. With the sacrificial sleeve 1142 disposed inside the syringe barrel 1102 for covering the apertures 1126, a flowable, curable, porous material (e.g., silicone) may be disposed within the apertures 1126 to form the gas permeable barriers 1145 that fill the apertures 1126. The uncured material that fills the holes 1126 preferably conforms to the shapes of the holes and the outer surface of the sacrificial sleeve 1142 that contacts the uncured material. After the material disposed within the apertures 1126 has been cured, the sacrificial sleeve 1142 may be removed from the inside of the outer wall 1124 of the syringe barrel 1102 to provide a smooth interface between the gas permeable barriers 1145 and the inner surface 1140 of the outer wall 1124 of the syringe barrel 1102. In one embodiment, the sacrificial sleeve 1142 may be made of silicone rubber.

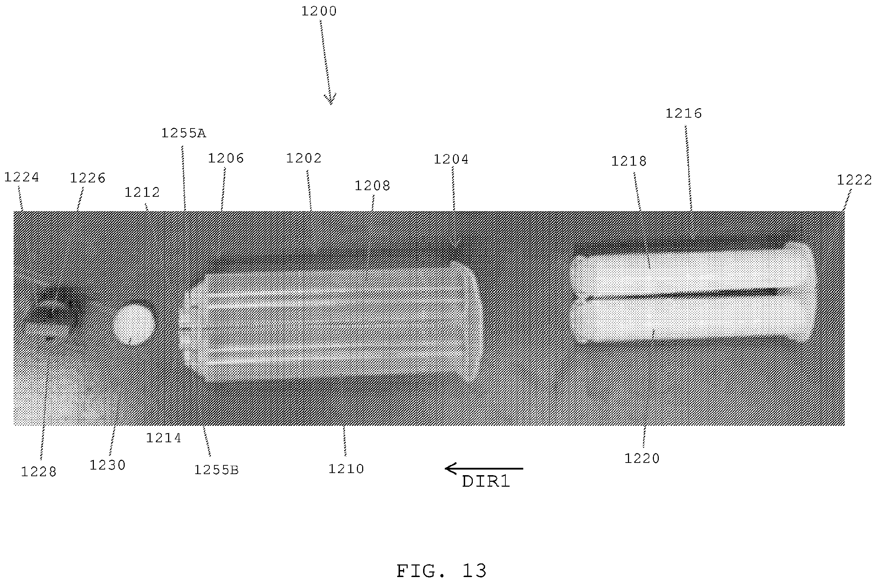

Referring to FIG. 13, in one embodiment, a gas sterilizable syringe 1200 preferably includes an enclosure 1202 (e.g., a double barrel syringe) having a proximal end 1204 and a distal end 1206. The enclosure 1202 preferably includes a first syringe barrel 1208 that is configured to receive a first part of a flowable material, and a second syringe barrel 1210 that is configured to receive a second part of the flowable material.

In one embodiment, the enclosure 1202 preferably includes a first dispensing opening 1212 that is in fluid communication with the distal end of the first syringe barrel 1208, and a second dispensing opening 1214 that is in fluid communication with a distal end of the second syringe barrel 1210. In one embodiment, after the first and second parts of the flowable material have been expressed via the respective first and second dispensing openings 1212, 1214, the first and second parts of the flowable material may be mixed together (e.g., with a static mixer) for forming a biocompatible material that is used during a surgical procedure, such as a silicone-based topical skin adhesive.

In one embodiment, the gas sterilizable syringe 1200 preferably includes a double barrel plunger 1216 including a first plunger 1218 that is configured for being inserted into the open end of the first syringe barrel 1208, and a second plunger 1220 that is configured for being inserted into the open end of the second syringe barrel 1210. In one embodiment, the gas sterilizable syringe 1200 preferably includes a thumb tab 1222 that interconnects proximal ends of the first and second plungers 1218, 1220. The thumb tab 1222 may be engaged for simultaneously depressing the first and second plungers 1218, 1220 in the distal direction DIR1 toward the distal end 1206 of the enclosure 1202, which, in turn, expresses the first and second parts of the flowable material from the respective dispensing openings 1212, 1214.

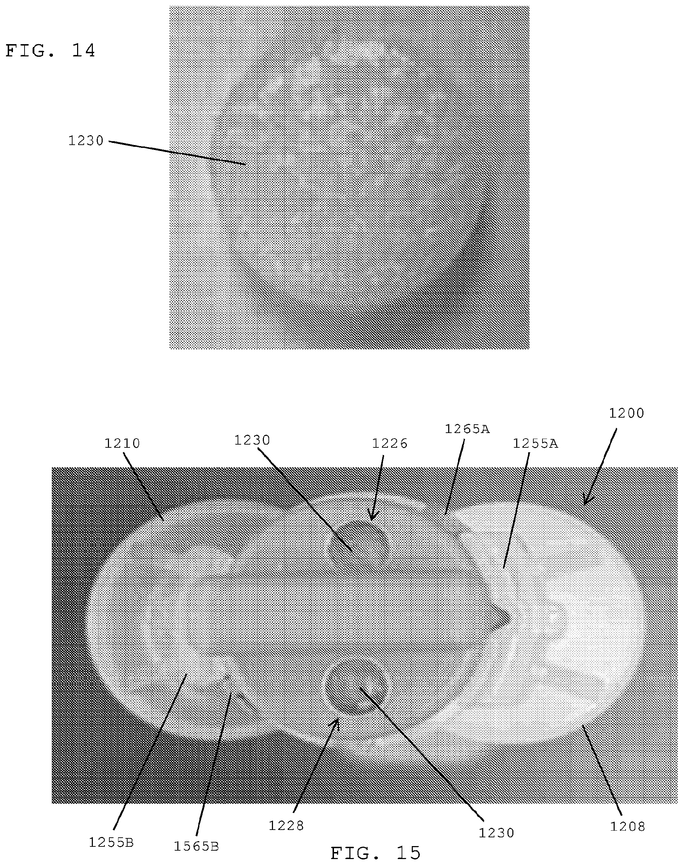

In one embodiment, the gas sterilizable syringe 1200 preferably includes an end cap 1224 that is adapted for being secured over the dispensing openings 1212, 1214 located at the distal end 1206 of the enclosure 1202. In one embodiment, the distal end 1206 of the enclosure desirably includes end cap connecting flanges 1255A, 1255B that are configured to engage radially extending projections 1265A 1265B (FIG. 15) provided on an outer surface of a hub of the end cap 1224 for securing the end cap 1224 to the distal end 1206 of the enclosure 1202 (e.g., a double barrel syringe). The end cap 1224 preferably includes a first end cap opening 1226 and a second end cap opening 1228 formed therein. With the end cap 1224 secured to the distal end 1206 of the enclosure 1202, the end cap openings 1226, 1228 are preferably adapted to enable sterilization gases to pass through the end cap 1224 and the first and second dispensing openings 1212, 1214 for sterilizing the first and second parts of the flowable material that are disposed within the fluid chambers of the respective first and second syringe barrels 1208, 1210.

In one embodiment, the gas sterilizable syringe 1200 preferably includes a gas permeable barrier 1230 (e.g., a cured silicone plug) that is configured for being disposed within the end cap 1224 and that is positioned between the distal ends of the first and second dispensing openings 1212, 1214 and an inner surface of the end cap 1224. In one embodiment, the gas permeable barrier may be made of silicone rubber having closed cell porosity.

In certain embodiments, the gas permeable barrier 1230 may be made of one or more of the following materials: silicone materials including room temperature vulcanized silicone (RTV), liquid silicone rubber (LSR), and high consistency rubber (HCR).

In one embodiment, the gas permeable barrier 1230 comprises a closed cell structure that has voids and/or porosity. In one embodiment, the gas permeable barrier 1230 may be molded or die cut. In one embodiment, no through holes (i.e., the absence of open cell pores) are present in the gas permeable barrier to seal off the content (e.g., flowable material) of the syringe barrels during repeat vacuum cycles of a gas sterilization process (e.g., an ethylene oxide gas sterilization process).

In one embodiment, the gas permeable barrier 1230 disposed within the end cap 1224 may be made from a variety of silicone rubber materials having a porosity range of between 1% and 90% of the total volume, and more preferably between 5% and 80% of the total volume, with mainly closed cell porosity.

In one embodiment, the gas permeable barrier 1230 disposed within the end cap 1224 is made of materials that are compatible with and do not chemically react with the content of the gas sterilizable syringe. Examples of preferred materials for the gas permeable barrier include but are not limited to silicone-polyethylene, silicone-acrylic or silicone-polyurethane copolymers, among others, which may be formed into the shape of the gas permeable barriers disclosed herein

In one embodiment, the gas permeable barrier 1230 may be made of porous silicone materials having a Shore A hardness scale rating of 5-70.

In one embodiment, the porosity of a silicone-based polymer used to form the gas permeable barrier 1230 may be generated during curing using an in-situ gas generation method.

In one embodiment, the gas permeable barrier 1230 may be made using commercially available silicone RTV foams, whereby the formulation may be modified to adjust porosity levels to optimize the results when used with a gas sterilizable syringe.

In one embodiment the gas permeable barrier 1230 may be molded or die cut. In one embodiment, the gas permeable barrier may be die cut from a larger porous silicone rubber sheet.

In one embodiment, the gas permeable barrier may be made of other highly gas permeable materials, such a rubbers and/or polymers materials.

In one embodiment, after the gas permeable barrier 1230 has been assembled with the end cap 1224, the end cap is preferably secured to the distal end of the enclosure 1202. The hub of the end cap preferably forms an air-tight seal with the distal end of the enclosure so that the sterilization gases cannot pass between the lower end of the hub of the end cap and the distal end wall of the enclosure.

With the end cap 1224 secured to the distal end 1206 of the enclosure 1202, the end cap openings 1226 and 1228 are preferably adapted to enable sterilization gases to pass through the end cap 1224, the gas permeable barrier 1230, and the first and second dispensing openings 1212, 1214 for sterilizing the first and second parts of the flowable material that are disposed within the fluid chambers of the respective first and second syringe barrels 1208, 1210.

Referring to FIG. 14, in one embodiment, the gas permeable barrier 1230 is preferably porous for enabling sterilization gases to pass through the gas permeable barrier for sterilizing the first and second parts of the flowable material disposed within the enclosure 1202 (FIG. 13) of the gas sterilizable syringe 1200. In one embodiment, the gas permeable barrier 1230 is made of silicone rubber, which contains closed cell pores. In one embodiment, there are no open pores (e.g., micro apertures) present in the gas permeable barrier 1230 in order to prevent leakage of the flowable material disposed within the syringe during the sterilization process. The pores of the gas permeable barrier 1230 are not interconnected to each other to enable the sterilization gases to pass through the gas permeable barrier while blocking the parts of the flowable material from passing therethrough. Thus, the gas permeable barrier 1230 is designed for allowing sterilization gases to flow therethrough while preventing a more viscous liquid from passing and/or flowing therethrough.

Referring to FIG. 15, in one embodiment, the end cap 1224 may be secured over the dispensing openings 1212, 1214 (FIG. 13) located at the distal ends of the respective first and second syringe barrels 1208, 1210. In one embodiment, the distal end 1206 of the enclosure 1202 (FIG. 13) desirably includes the end cap connecting flanges 1255A, 1255B that are configured to engage the radially extending projections 12656A 1265B (FIG. 15) provided on an outer surface of the hub of the end cap 1224 for securing the end cap 1224 to the distal end 1206 of the enclosure 1202 (e.g., a double barrel syringe). The lower end of the hub of the end cap 1224 preferably forms an air-tight seal with the distal end of the enclosure 1202.

In one embodiment, the gas permeable barrier 1230 (FIG. 14) is disposed within the end cap 1224 and is in alignment with the first and second end cap openings 1226, 1228 formed in the end cap 1224. The end cap 1224 preferably includes the first end cap opening 1226 and the second end cap opening 1228 that enable sterilization gases to pass through the end cap 1224 and the gas permeable barrier 1230 for sterilizing the parts of the flowable material disposed within the fluid chambers of the respective syringe barrels 1208, 1210.

In one embodiment, sterilization gases preferably pass through the first and second end cap openings 1226, 1228 and the gas permeable barrier 1230 for entering into the respective fluid chambers of the first and second syringe barrels 1208, 1210.

In one embodiment, the only entry pathway for the sterilization gases into the fluid chambers of the enclosure is through the first and second end cap openings 1228 formed in the end cap 1224.

In one embodiment, an end cap for a gas sterilizable syringe may have a single end cap opening.

In one embodiment, the end cap 1224 for a gas sterilizable syringe may have three or more end cap openings.

In one embodiment, after the gas sterilizable syringe 1200 has been sterilized, the end cap 1224 preferably remains in place (i.e., secured to the distal end of the enclosure 1202) during shipment and storage of the syringe. During a surgical procedure, the end cap 1224 may be disconnected from the distal end 1206 of the enclosure and replaced by a mixing and dispensing tip or a dispensing tip (not shown) that is securable to the distal end of the enclosure for mixing and/or expressing the flowable material disposed within the first and second syringe barrels 1208, 1210.

Experiment 1. Gas sterilizable syringes having a structure similar to that shown in FIGS. 13-15, each containing a two-part silicone adhesive formulation, were sterilized using ethylene oxide (EO) gas. Biologic indicators (BI) were placed inside the fluid chambers of the respective syringe barrels of the gas sterilizable syringes to test the functionality of the gas permeable barriers (e.g., gas permeable plugs) and/or the gas permeable plunger seals. The biologic indicators in all of tested devices showed negative results, which indicates that ethylene oxide sterilization gases were able to pass into the syringes and effectively sterilize each of the two-parts of the silicone adhesive formulations disposed within the syringes. The EO exposure time was 785 cycles (about 5 hours) followed by application of a vacuum pressure less than 50 mmHg for removal of residual EO gas in the sterilization chamber. The BI indicated that all the bacteria had been neutralized by the EO cycle, which proved that the syringe is EO permeable. In addition, the residual EO content inside of the sterilized syringes were tested. The result of the testing indicated a residual EO content of 3.59 µg /cm², which is well below the required limit of 10 µg /cm².

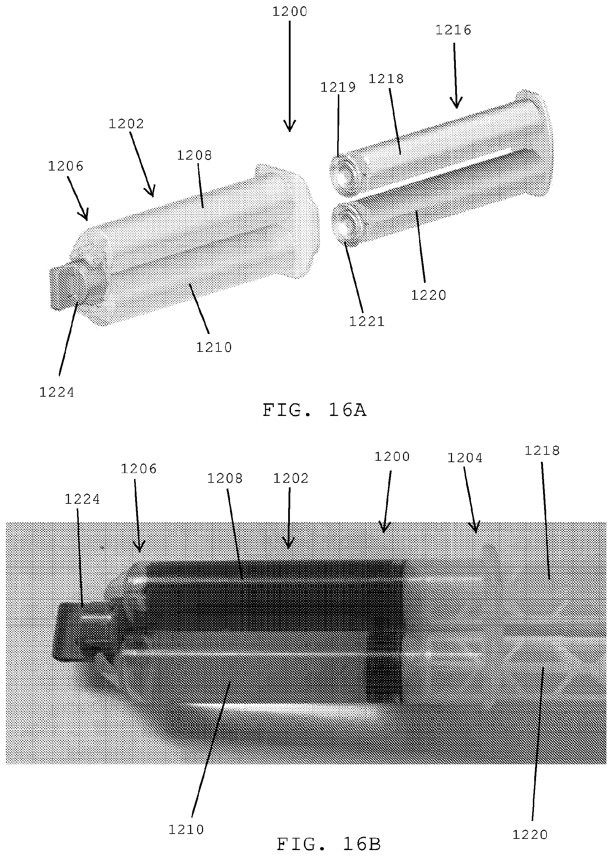

Referring to FIG. 16A, in one embodiment, the gas sterilizable syringe 1200 preferably includes the enclosure 1202 (e.g., a double barrel syringe) having the first syringe barrel 1208 and the second syringe barrel 1210. The end cap 1224 is preferably secured to the distal end 1206 of the enclosure 1202 for covering the first and second dispensing openings 1212, 1214 (FIG. 13) located at the distal ends of the respective first and second syringe barrels 1208, 1210.

In one embodiment, the distal ends of the respective first and second plungers 1218, 1220 of the double barrel plunger 1216 are inserted into openings located at the proximal ends of the respective first and second syringe barrels 1208, 1210. The distal end of the first plunger 1218 may include a first plunger seal 1219 that is configured to form a liquid-tight seal with an inner surface of the first syringe barrel 1208 and the distal end of the second plunger 1220 may include a second plunger seal 1221 that is configured to form a liquid-tight seal with an inner surface of the second syringe barrel 1210. In one embodiment, when the double barrel plunger 1216 is assembled with the double barrel syringe 1202, sterilization gases may pass between the outer perimeter of the first plunger seal 1219 and the inner surface of the outer wall of the first syringe barrel 1208, and pass into the first fluid chamber of the first syringe barrel for sterilizing a first part of a flowable composition disposed within the first fluid chamber of the first syringe barrel. In one embodiment, a similar structure is provided for sterilizing the second part of the flowable composition disposed within the second fluid chamber of the second syringe barrel 1210. In one embodiment, sterilization gases may pass between the outer perimeter of the second plunger seal 1221 and the inner surface of the outer wall of the second syringe barrel 1210, and pass into the second fluid chamber of the second syringe barrel for sterilizing a second part of the flowable composition disposed within the second fluid chamber of the second syringe barrel 1210.

Referring to FIG. 16B, in one embodiment, the first syringe barrel 1208 is preferably filled with a first part of a flowable material and the second syringe barrel 1210 is preferably filled with a second part of a flowable material. The distal ends of the first and second plungers 1218, 1220 are preferably inserted into the openings at the proximal end 1204 of the enclosure 1202. The end cap 1224 is preferably secured to the distal end 1206 of the enclosure 1202 for covering the dispensing openings 1212, 1214 (FIG. 13) located at the distal ends of the respective first and second syringe barrels 1208, 1210.

The syringe and the content of the syringe is preferably sterilized by passing sterilization gases through the end cap openings of the end cap 1224. After the syringe 1200 has been sterilized, the end cap 1224 preferably remains in place during shipment and storage of the device. During a surgical procedure, with the syringe located within a sterile environment, the end cap 1224 may be removed and replaced by a mixing and dispensing tip or a dispensing tip that is secured to the distal end 1206 of the enclosure for mixing and/or dispensing the first and second parts of a flowable material that are contained within the respective first and second syringe barrels 1208, 1210.

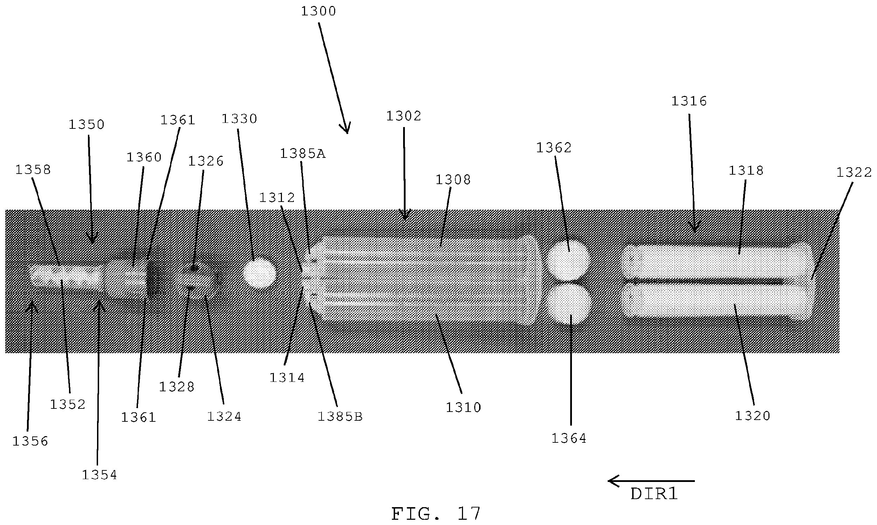

Referring to FIG. 17, in one embodiment, a gas sterilizable syringe 1300 preferably includes an enclosure 1302 having a proximal end 1304 and a distal end 1306. The enclosure 1302 preferably includes a first syringe barrel 1308 having a first fluid compartment configured to receive a first part of a flowable material and a second syringe barrel 1310 having a second fluid compartment configured to receive a second part of a flowable material.

In one embodiment, the enclosure 1302 preferably includes a first dispensing opening 1312 in fluid communication with the first fluid compartment of the first syringe barrel 1308 and a second dispensing opening 1314 in fluid communication with the second fluid compartment of the second syringe barrel 1310.

In one embodiment, the gas sterilizable syringe 1300 preferably includes an end cap 1324 including a first end cap opening 1326 and second end cap opening 1328. In one embodiment, the first end cap 1326 is adapted to receive a gas permeable barrier 1330 (e.g., gas permeable plug) that is preferably disposed within the end cap 1324 and that is in alignment with the first and second end cap openings 1326, 1328. In one embodiment, the end cap 1326 including the gas permeable barrier 1330 is adapted to be secured to the distal end 1306 of the enclosure 1302 for covering the dispensing openings 1312, 1314 at the distal ends of the respective first and second syringe barrels 1308, 1310. In one embodiment, the combination of the end cap 1324 and the gas permeable barrier 1330 enables sterilization gases to pass therethrough for sterilizing the first and second parts of the flowable material that are disposed within the respective first and second syringe barrels 1308, 1310.

In one embodiment, the gas sterilizable syringe 1300 preferably includes a double barrel plunger 1316 including a first plunger 1318 adapted to be disposed within the first syringe barrel 1308 and second plunger 1320 adapted to be disposed within the second syringe barrel 1310. In one embodiment, the double barrel plunger 1316 preferably includes a thumb tab 1322 secured to proximal ends of the respective first and second plungers 1318, 1320 so that the double barrel plunger 1316 may be simultaneously depressed toward the distal end 1306 of the enclosure 1302.

In one embodiment, a first gas permeable plunger seal 1362 is preferably securable to a distal end of the first plunger 1318 and a second gas permeable plunger seal 1364 is preferably securable to a distal end of the second plunger 1320. The first and second gas permeable plunger seals 1362, 1364 are preferably configured for enabling sterilization gases to pass therethrough for sterilizing the parts of the flowable material disposed within the respective first and second syringe barrels 1308, 1310. In one embodiment, the first and second gas permeable plunger seals may be made of silicone rubber, which contains closed cell pores. In one embodiment, there are no open pores (e.g., micro apertures) present in the gas permeable plunger seals in order to prevent leakage of the content (e.g., flowable material) disposed within the first and second syringe barrels during a sterilization process.

In one embodiment, the double barrel plunger 1316 may include vents and/or conduits that enable sterilization gases to pass therethrough for sterilizing first and second fluid compartments of the first and second syringe barrels 1308, 1310. The vents and/or conduits may be in fluid communication with the first and second gas permeable plunger seals 1362, 1364.

In one embodiment, the end cap 1324 and the gas permeable barrier 1330 are secured over the distal end 1306 of the enclosure 1302 for covering the openings of the first and second dispensing openings 1312, 1314. With the end cap 1324 and the gas permeable barrier 1330 secured to the distal end of the enclosure 1302, sterilization gases may pass through the first and second end cap openings 1326, 1328, the gas permeable barrier 1330, and the dispensing openings 1312, 1314 for sterilizing the first and second parts of the flowable material disposed within the fluid chambers of the respective first and second syringe barrels 1308, 1310.

With the first and second plungers 1318, 1320 disposed within the respective first and second syringe barrels 1308, 1310, the sterilization gases may pass through the plungers and/or the first and second gas permeable plunger seals 1362, 1364 for sterilizing the parts of the flowable material disposed within the respective first and second syringe barrels 1308, 1310.

In one embodiment, the gas sterilizable syringe preferably includes a mixing and dispensing tip 1350 that may be secured to the distal end 1306 of the enclosure 1302. In one embodiment, the mixing and dispensing tip 1350 is preferably configured for mixing together the first and second parts of the flowable composition, and expressing the flowable composition from the distal end of the gas sterilizable syringe 1300.

In one embodiment, the mixing and dispensing tip 1350 preferably includes a mixing and dispensing tube 1352 having a proximal end 1354 and a distal end 1356. A static mixer 1358 is preferably disposed within the mixing and dispensing tube 1352 for mixing together the first and second parts of the flowable material that is disposed within the respective first and second syringe barrels 1308, 1310.

In one embodiment, the mixing and dispensing tip 1350 preferably includes a connector 1360 that is adapted for connecting the mixing and dispensing tip 1350 to the distal end 1306 of the enclosure 1302 of the gas sterilizable syringe 1300. In one embodiment, the mixing and dispensing tip 1350 preferably includes one or more radially extending projections 1361 that extend from the outer surface of the connector 1360. In one embodiment, the one or more radially extending projections 1361 are preferably configured to engage securing flanges 1385A, 1385B located at the distal end of the enclosure 1302 for securing the mixing and dispensing tip 1350 to the distal end of the enclosure 1302.

In one embodiment, during a surgical procedure, the end cap 1324 including the gas permeable barrier 1330 may be disconnected from the distal end 1306 of the enclosure 1302, and replaced by the mixing and dispensing tip 1350. The connector 1360 located at the proximal end of the mixing and dispensing tip 1350 may be secured over the exposed first and second dispensing openings 1312, 1314 located at the distal end 1306 of the enclosure 1302. The one or more radially extending projections 1361 at the proximal end of the connector 1360 preferably engage the securing flanges 1385 at the distal end of the enclosure 1302 for securing the mixing and dispensing tip to the distal end of the enclosure. The thumb tab 1322 secured to the proximal ends of the first and second plungers 1318, 1320 may be depressed in the distal direction DIR1 towards the distal end 1306 of the enclosure 1302 to force the first and second parts of the flowable material to flow out of the first and second dispensing openings 1312, 1314 and into the mixing and dispensing tube 1352 of the mixing and dispensing tip 1350. As the first and second parts of the flowable material flow in a distal direction DIR1 through the mixing and dispensing tube 1352, the two parts of the flowable material are mixed together via the static mixer 1358 that is disposed within the mixing and dispensing tube 1352.

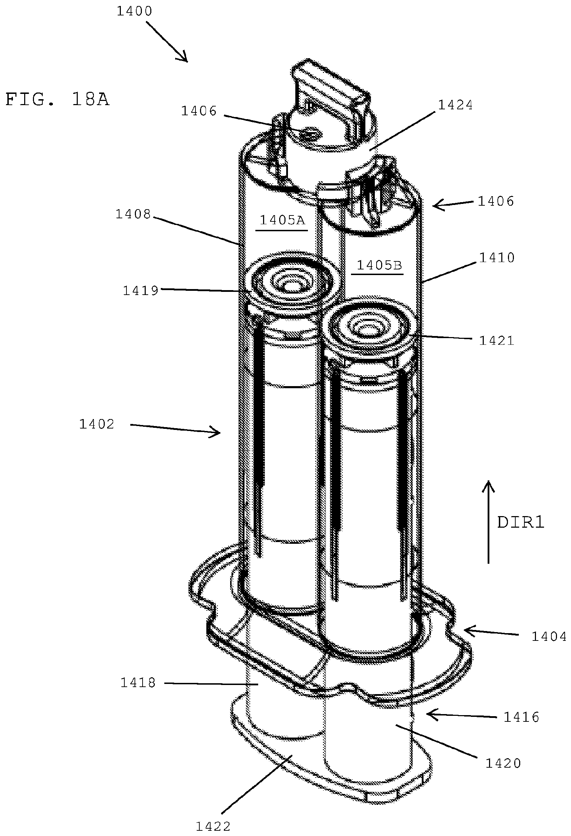

Referring to FIGS. 18A-18B, 19 and 20, in one embodiment, a gas sterilizable syringe 1400 preferably includes an enclosure 1402 having a proximal end 1404 and a distal end 1406. In one embodiment, the enclosure 1402 is preferably a dual barrel syringe including a first syringe barrel 1408 having a first fluid compartment 1405A and second syringe barrel 1410 having a second fluid compartment 1405B.

In one embodiment, the gas sterilizable syringe 1400 preferably includes a dual barrel plunger 1416 including a first plunger 1418 that is adapted to be inserted into a proximal end of the first syringe barrel 1408 and a second plunger 1420 that is adapted to be inserted into a proximal end of the second syringe barrel 1410.

In one embodiment, the first plunger 1418 preferably includes a first plunger head 1419 located at a distal end thereof and the second plunger 1420 preferably includes a second plunger head 1421 located at a distal end thereof. In one embodiment, the outer perimeters of the first and second plunger heads 1419, 1421 preferably form a liquid-tight seal with inner surfaces of the respective first and second syringe barrels 1408, 1410.

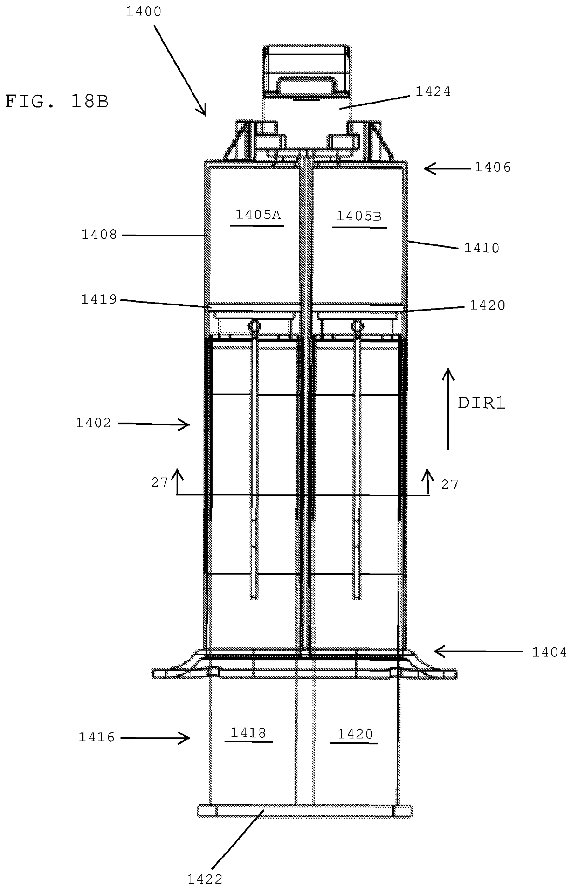

In one embodiment, the dual barrel plunger 1416 preferably includes a thumb tab 1422 that interconnects proximal ends of the first and second plungers 1418, 1420. In one embodiment, the thumb tab 1422 may be used for depressing the dual barrel plunger 1416 in the distal direction DIR1 (FIGS. 18A and 18B) for expressing first and second parts of a flowable material from the distal ends of the respective first and second syringe barrels 1408, 1410.

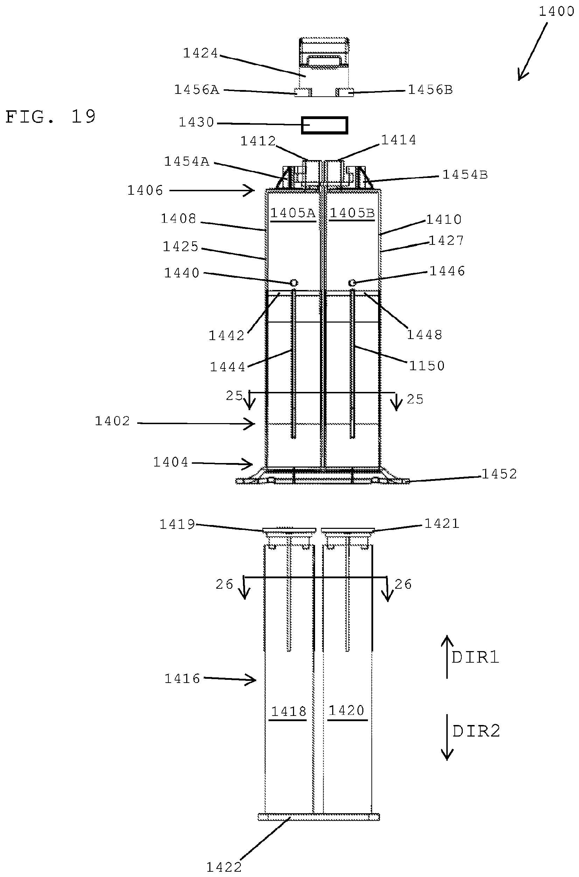

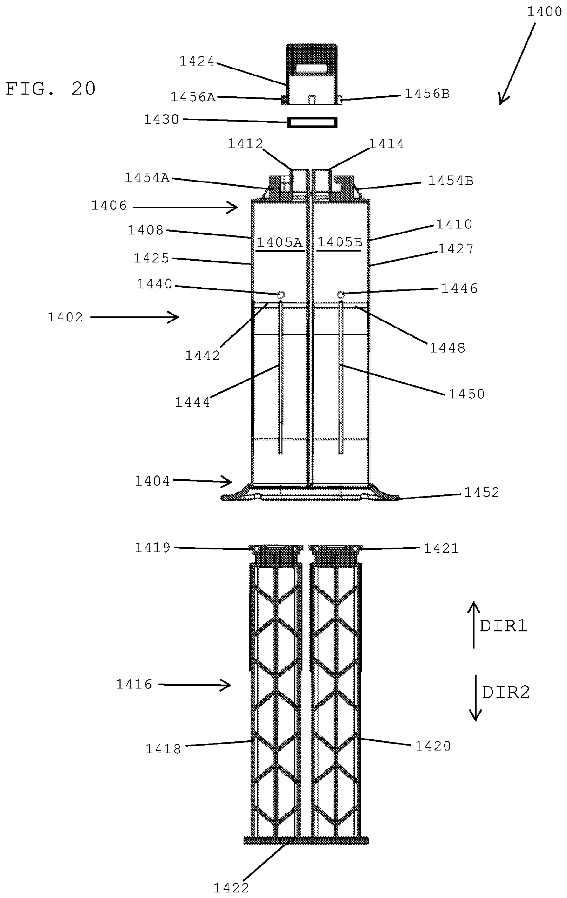

In one embodiment, the gas sterilizable syringe 1400 preferably includes an end cap 1424 secured to the distal end 1406 of the enclosure 1402. A gas permeable barrier 1430 (FIGS. 19 and 20) may be disposed within the end cap 1424 for enabling sterilization gases to pass through the end cap and into the first and second fluid compartments 1405A, 1405B of the respective first and second syringe barrels 1408, 1410 for sterilizing the first and second parts of a flowable material disposed within the enclosure 1402, while preventing the flowable material from passing through the gas permeable barrier 1430. In one embodiment, the end cap 1424 preferably includes one or more end cap openings 1426 (FIG. 18A) that are configured for enabling the sterilization gases to pass through the end cap 1424 and the gas permeable barrier 1430 (FIGS. 19 and 20) for sterilizing the first and second parts of the flowable material disposed within the first and second fluid chambers 1405A, 1405B of the respective first and second syringe barrels 1408, 1410.

Referring to FIGS. 19 and 20, in one embodiment, the enclosure 1402 preferably includes a first dispensing opening 1412 in fluid communication with the first fluid compartment 1405A of the first syringe barrel 1408, and a second dispensing opening 1414 in fluid communication with the second fluid compartment 1405B of the second syringe barrel 1410.

In one embodiment, the first syringe barrel 1408 preferably has an outer wall 1425 having one or more air vent openings 1440 formed therein. The first syringe barrel 1408 preferably includes a first one-way plunger stop 1442 formed on an inner surface of the outer wall 1425 that is located between the one or more air vent openings 1440 and the proximal end 1404 of the enclosure 1402. In one embodiment, after the first plunger head 1419 has moved distally beyond the first one-way plunger stop 1442, the first one-way plunger stop 1442 engages the first plunger to make it easier to move the first plunger 1418 in the distal direction DIR1, and more difficult to retract the first plunger 1418 in the proximal direction DIR2.

In one embodiment, the first one-way plunger stop 1442 is designed into the inner diameter of the first syringe barrel 1408 such that upon plunger insertion into a filled syringe, the plunger could easily ride over a tapered introduction to the first one-way plunger stop 1442, but once past the first one-way plunger stop 1442, the plunger would butt up against the first one-way plunger stop 1442 and not be able to be retracted without excessive force from this rest position. In one embodiment, the one-way plunger stop 1442 is designed to prevent migration of the first plunger during the pressure changes and vacuum cycles that occur during sterilization.

In one embodiment, the first syringe barrel 1424 desirably includes elongated rifling 1444 formed in the inner surface of the outer wall 1424 that extends along the length of the first syringe barrel 1408. The elongated rifling 1444 preferably terminates adjacent the one-way plunger stop 1442 of the first syringe barrel 1408. The elongated rifling 1444 preferably guides the sliding movement of the first plunger 1418 relative to the first syringe barrel 1408.

In one embodiment, the second syringe barrel 1410 preferably includes an outer wall 1427 having one or more air vent holes 1446 formed therein. The second syringe barrel 1410 preferably includes a second one-way plunger stop 1448 that is located between the one or more air vent holes 1446 of the second syringe barrel and the proximal end 1404 of the enclosure 1402. In one embodiment, after the second plunger head 1420 has moved distally beyond the second one-way plunger stop 1448, the second one-way plunger stop 1448 engages the second plunger 1420 for making it easier to move the second plunger 1420 in the distal direction DIR1, while making it more difficult to retract the second plunger 1420 in the proximal direction DIR2. The second syringe barrel 1410 preferably includes elongated rifling 1450 that extends along the length of the second syringe barrel 1410 and that it terminates adjacent the second one-way plunger stop 1448. The elongated rifling 1450 preferably guides the sliding movement of the second plunger 1420 relative to the second syringe barrel 1410.

In one embodiment, the second one-way plunger stop 1448 is designed into the inner diameter of the second syringe barrel 1410 such that upon plunger insertion into a filled syringe, the plunger could easily ride over a tapered introduction to the second one-way plunger stop 1448, but once past the second one-way plunger stop 1448, the plunger would butt up against the second one-way plunger stop 1448 and not be able to be retracted without excessive force from this rest position. In one embodiment, the second one-way plunger stop 1448 is designed to prevent migration of the second plunger during the pressure changes and vacuum cycles that occur during sterilization.

In certain embodiments, it may be advantageous to use syringes having a single external dimensional footprint for a product line that provides multiple, different, fill volumes. The shared footprint may be desired to simplify to a single, shared rigid blister packaging for syringes having different fill volumes. The exact location of the one-way plunger stops 1442, 1448 within the syringe barrels may be modified, adjusted, and/or customized so that the one-way plunger stops can be placed inside the syringe barrels at a variety of desired fill volume levels to allow for a single syringe footprint having different fill volumes. For a syringe designed to have a greater fill volume, the one-way plunger stops will be positioned further away from the distal ends of the syringe barrels, and for a syringe designed to have a smaller fill volume, the one-way plunger stops will be positioned closer to the distal ends of the syringe barrel.

In one embodiment, the gas sterilizable syringe 1400 preferably includes a circumferential flange 1452 located at the proximal end 1404 of the enclosure 1402 and extending around the proximal ends of the respective first and second syringe barrels 1408, 1410. In one embodiment, the circumferential flange 1452 preferably interconnects the proximal ends of the first and second syringe barrels 1408, 1410. A user may engage the circumferential flange with fingers for expressing a flowable material from the distal end of the gas sterilizable syringe.

In one embodiment, the gas sterilizable syringe 1400 preferably includes end cap connecting flanges 1454A, 1454B located at the distal end 1406 of the enclosure 1402, which are configured to engage radially extending projections 1456A, 1456B provided on an outer surface of the end cap 1424 for releasably securing the end cap 1424 to the distal end 1406 of the enclosure 1402. When secured to the distal end of the enclosure, the end cap preferably forms an air-tight seal with the distal end wall of the enclosure.

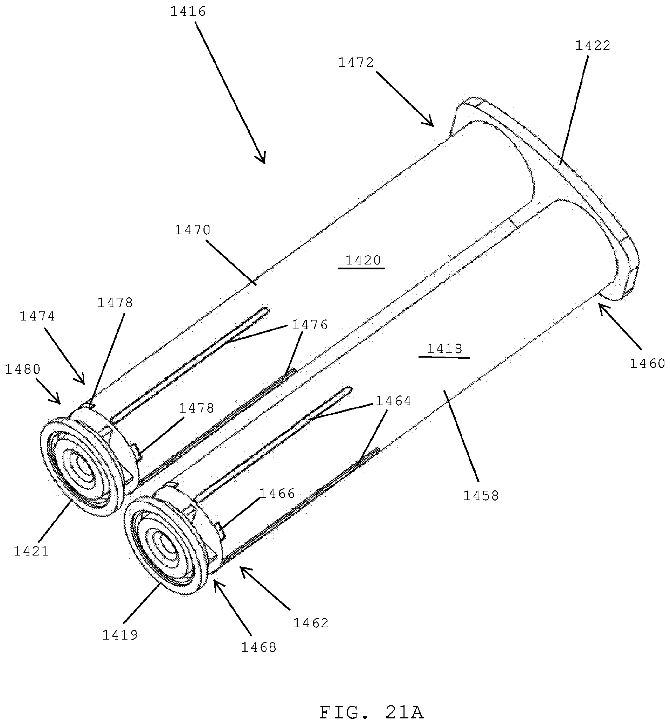

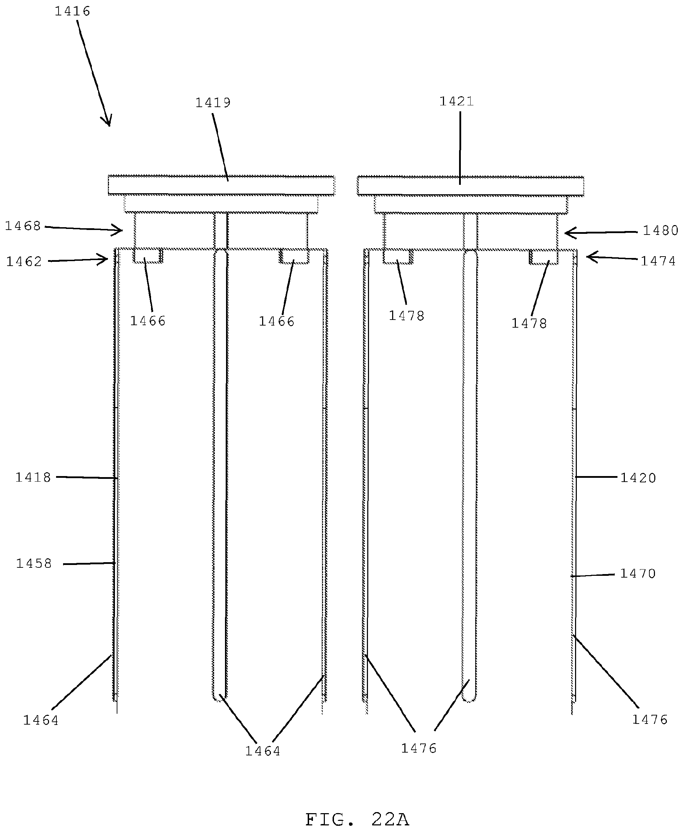

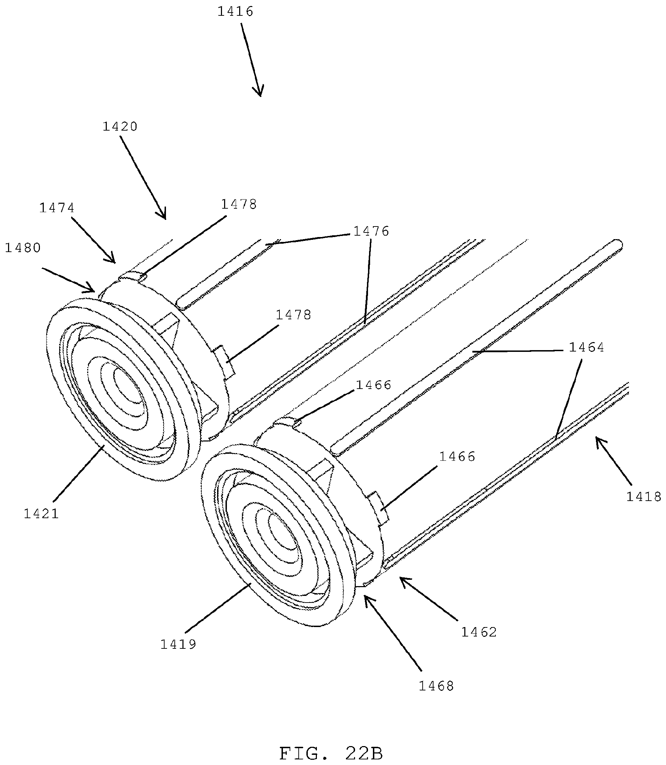

Referring to FIGS. 21A-21B and 22A-22B, in one embodiment, the dual barrel plunger 1416 preferably includes the first plunger 1418 and the second plunger 1420. The thumb tab 1422 interconnects proximal ends of the respective first and second plungers 1418, 1420.

In one embodiment, the first plunger 1418 preferably includes a first plunger shaft 1458 having a proximal end 1460 and a distal end 1462. The first plunger 1418 preferably includes elongated guide rails 1464 that extend over an outer surface of the first plunger shaft 1458 and along the length of the first plunger shaft. In one embodiment, the elongated guide rails 1464 desirably terminate at the distal end 1462 of the first plunger shaft 1458. In one embodiment, the distal end 1462 of the first plunger shaft 1458 preferably includes one or more radially extending projections 1466 that are adapted to engage the first one-way plunger stop 1442 of the first syringe barrel 1408 (FIG. 19), for preventing retraction of the first plunger after the one or more radially extending projections 1466 having moved distally beyond the first one-way plunger stop 1442.

In one embodiment, the first plunger head 1419 is spaced away from the distal end 1462 of the first plunger shaft 1458 to define a first plunger cavity 1468 that is located between the distal end of the first plunger shaft and the first plunger head 1419.

In one embodiment, the second plunger 1420 preferably includes a second plunger shaft 1470 having a proximal end 1472 and a distal end 1474. The second plunger 1420 preferably includes elongated guide rails 1476 that extend over an outer surface of the second plunger shaft 1470 and along the length of the second plunger shaft. The elongated guide rails 1476 desirably terminate at the distal end 1474 of the second plunger shaft 1470. In one embodiment, the distal end 1474 of the second plunger shaft 1470 preferably includes one or more radially extending projections 1478 that are adapted to engage the second one-way plunger stop 1448 of the second syringe barrel 1410 (FIG. 19), for preventing retraction of the second plunger after the one or more radially extending projections 1478 have moved distally beyond the second one-way plunger stop 1448.

In one embodiment, the second plunger head 1421 is spaced away from the distal end 1474 of the second plunger shaft 1470 to define a second plunger cavity 1480 that is located between the distal end of the second plunger shaft 1470 and the second plunger head 1421.

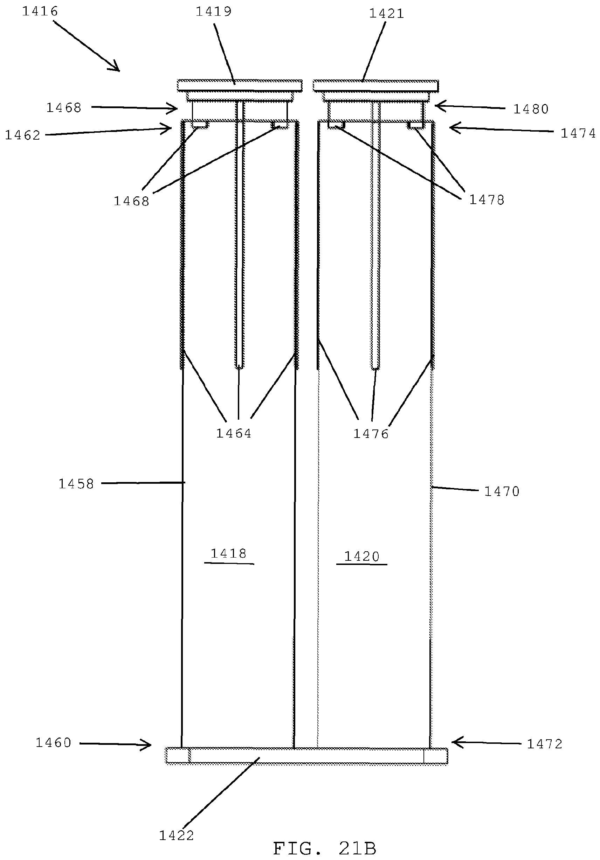

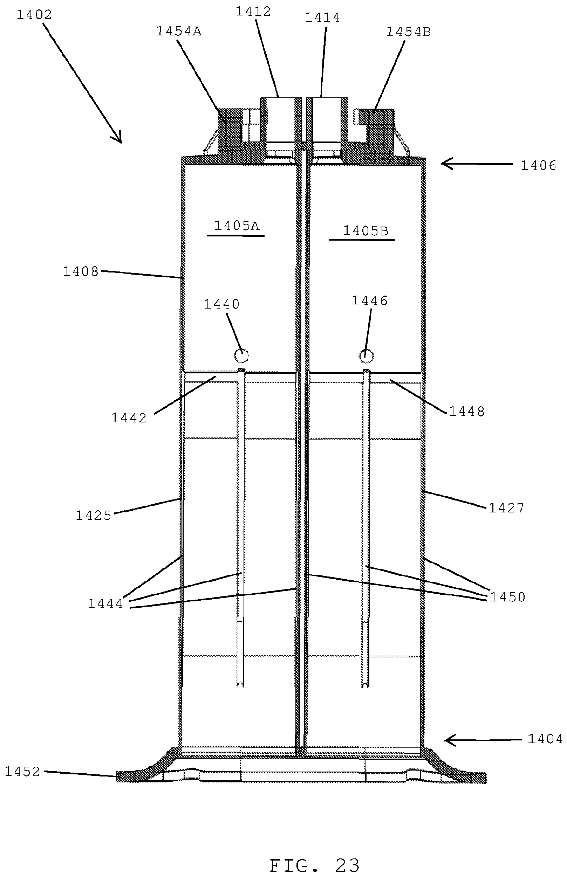

Referring to FIG. 23, in one embodiment, the dual barrel syringe 1402 is configured to receive the dual barrel plunger 1416 shown and described above in FIGS. 21A-21B and 22A-22B. In one embodiment, the first syringe barrel 1408 is configured to receive the first plunger 1418 (FIGS. 21A and 21B) and the second syringe barrel 1410 is configured to receive the second plunger 1420 (FIGS. 21A and 21B).

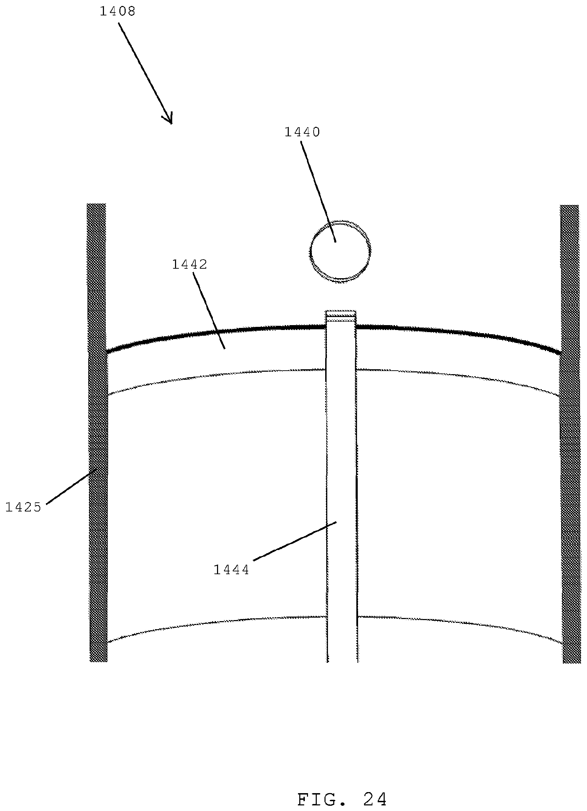

Referring to FIGS. 23 and 24, in one embodiment, the first syringe barrel 1408 preferably includes an outer wall 1425 that extends along the length of the first syringe barrel. The first syringe barrel 1408 preferably includes one or more air vent holes 1440 that pass through the outer wall 1425. The air vent holes 1440 allow ambient air present in the first syringe barrel 1408 to be expressed from the first syringe barrel. The air vent holes 1440 may also provide a supplemental pathway for sterilization gas into the fluid chamber 1405A of the first syringe barrel. The first one-way plunger stop 1442 is preferably formed over the inner surface of the outer wall 1425 of the first syringe barrel 1408. The first one-way plunger stop 142 may be a depression or a projection. The one or more elongated guide slots 1444 (e.g., rifling) are formed in the inner surface of the outer wall 1425 and extend along the length of the first syringe barrel 1408. In one embodiment, the one or more elongated guide slots 1444 are adapted to receive the one or more guide rails 1464 that extend along the length of the first plunger 1418 (FIGS. 21A and 21B).

Referring to FIG. 23, in one embodiment, the second syringe barrel 1410 preferably includes an outer wall 1427 that extends along the length of the second syringe barrel. The second syringe barrel 1410 preferably includes one or more air vent holes 1446 that pass through the outer wall 1427. The air vent holes 1446 allow ambient air present in the second syringe barrel 1410 to be expressed from the second syringe barrel. The air vent holes 1446 may also provide a supplemental pathway for sterilization gas into the fluid chamber 1405B of the second syringe barrel 1410. The second one-way plunger stop 1448 is preferably formed over the inner surface of the outer wall 1427 of the first syringe barrel 1410. The one or more elongated guide slots 1450 (e.g., rifling) are formed in the inner surface of the outer wall and extend along the length of the second syringe barrel 1410. In one embodiment, the one or more elongated guide slots 1450 are adapted to receive the one or more guide rails 1476 that extend along the length of the second plunger 1420 (FIGS. 21A and 21B) of the dual barrel plunger.

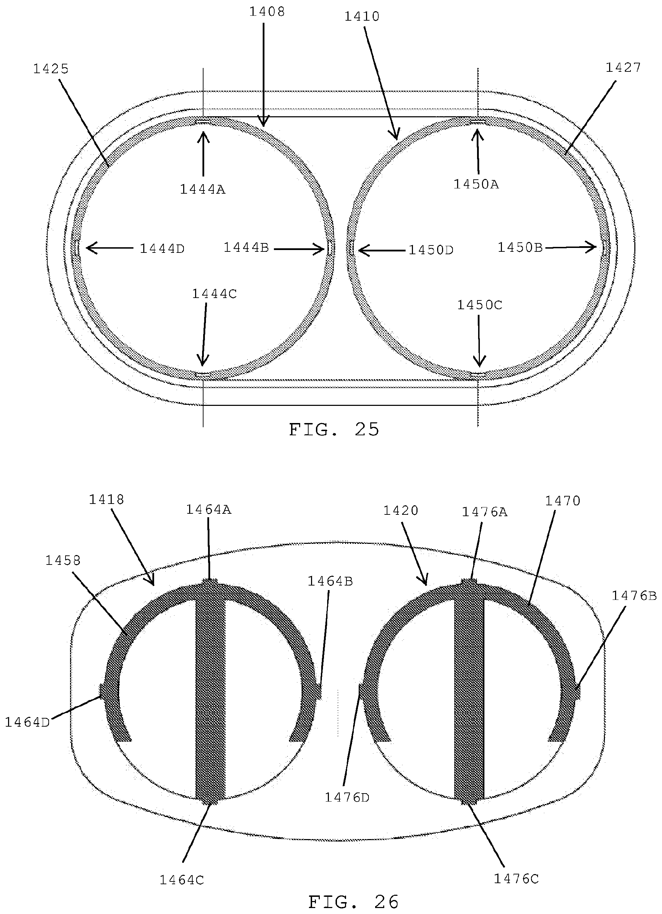

Referring to FIG. 25, in one embodiment, the first syringe barrel 1408 includes the outer wall 1425 that extends along the length of the first syringe barrel. The one or more elongated guide slots of the first syringe barrel may be evenly spaced from one another over the inner surface of the first syringe barrel 1408. In one embodiment, the one or more elongated guide slots preferably include four elongated guide slots 1444A-1444D that are formed over the inner surface of the outer wall 1425 of the first syringe barrel 1408. In one embodiment, the four elongated guide slots 1444A-1444D extend along respective axes that are parallel to one another.

In one embodiment, the second syringe barrel 1410 includes the outer wall 1427 that extends along the length of the first syringe barrel. The one or more elongated guide slots of the second syringe barrel may be evenly spaced from one another over the inner surface of the second syringe barrel 1410. In one embodiment, the one or more elongated guide slots preferably include four elongated guide slots 1450A-1450D that are formed over the inner surface of the outer wall 1427 of the second syringe barrel 1410. In one embodiment, the four elongated guide slots 1450A-1450D extend along respective axes that are parallel to one another.

Referring to FIG. 26, in one embodiment, the first plunger 1418 includes the first plunger shaft 1458 that extends along the length of the first plunger. In one embodiment, the one or more elongated guide rails preferably include four elongated guide rails 1464A-1464D that are formed over the outer surface of the first plunger shaft 1458 of the first plunger 1418. In one embodiment, the four elongated guide rails 1464A-1464D are evenly spaced from one another over the outer surface of the first plunger shaft 1458 of the first plunger 1418. In one embodiment, the four elongated guide rails 1464A-1464D extend along respective axes that are parallel to one another.

In one embodiment, the second plunger 1420 includes the second plunger shaft 1470 that extends along the length of the second plunger. In one embodiment, the one or more elongated guide rails preferably include four elongated guide rails 1476A-1476D that are formed over the outer surface of the second plunger shaft 1470 of the second plunger 1420. In one embodiment, the four elongated guide rails 1476A-1476D are evenly spaced from one another over the outer surface of the second plunger shaft 1470 of the second plunger 1420. In one embodiment, the four elongated guide rails 1476A-1476D extend along respective axes that are parallel to one another.

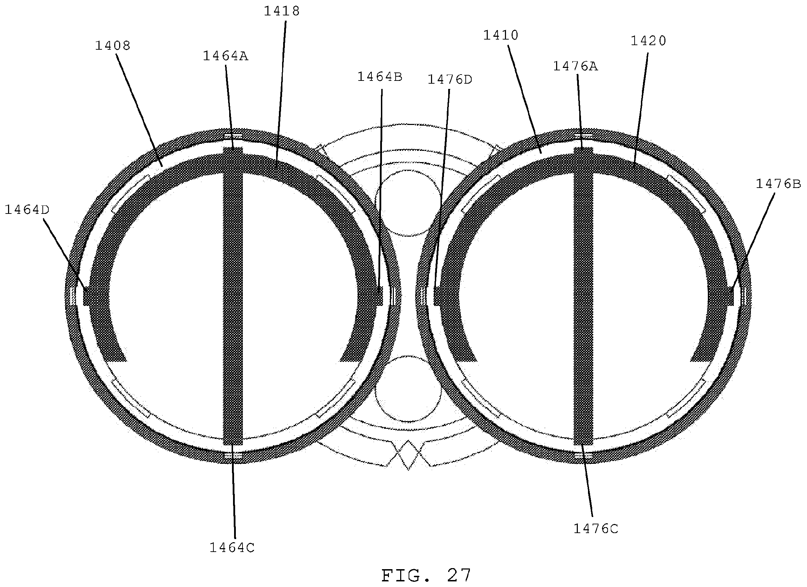

Referring to FIG. 27, in one embodiment, when the dual barrel syringe and the dual barrel plunger are assembled together, the first plunger 1418 is inserted into the first syringe barrel 1408 and the second plunger 1420 is inserted into the second syringe barrel 1410.

In one embodiment, the four elongated guide rails 1464A-1464D (FIG. 26) of the first plunger 1418 are seated within the respective four elongated guide slots 1444A-1444D (FIG. 25) of the first syringe barrel 1408 for guiding the sliding movement of the first plunger toward the distal end 1406 of the enclosure 1402.

In one embodiment, the four elongated guide rails 1476A-1476D (FIG. 26) of the second plunger 1420 are seated within the respective four elongated guide slots 1450A-1450D (FIG. 25) of the second syringe barrel 1410 for guiding the sliding movement of the second plunger toward the distal end 1406 of the enclosure 1402.

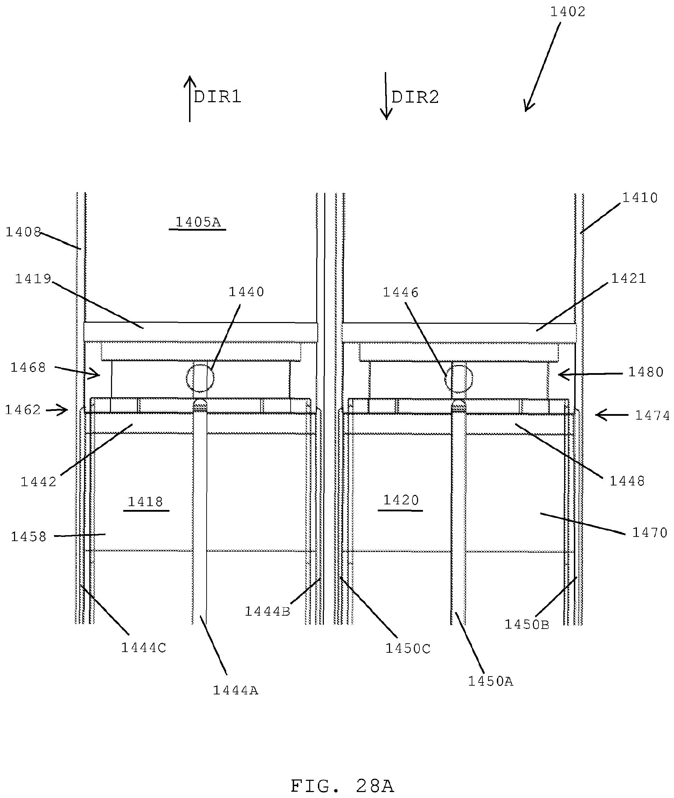

Referring to FIG. 28A, in one embodiment, the dual barrel plunger 1416 (FIG. 21A) is inserted into the dual barrel syringe 1402 so that the first plunger 1418 is disposed within the first syringe barrel 1408 and the second plunger 1420 is disposed within the second syringe barrel 1410. In one embodiment, the first and second plungers 1418, 1420 may be advanced in the distal direction DIR1 to the position shown in FIG. 28A so that the first plunger head 1419 is distal to the one or more vent openings 1440 of the first syringe barrel 1408, the distal end 1462 of the first plunger shaft 1458 is proximal to the one or more air vent openings 1440 of the first syringe barrel 1408, and the first plunger cavity 1468 is aligned with the one or more air vent openings 1440 of the first syringe barrel 1408. The radial projections 1466 located at the distal end 1462 of the first plunger barrel shaft 1458 engage the first one-way plunger stop 1442 of the first syringe barrel for preventing retraction of the first plunger 1418 in the proximal direction DIR2.

Simultaneously, the second plunger head 1421 is distal to the one or more air vent openings 1446 of the second syringe barrel 1410, the distal end 1474 of the second plunger shaft 1470 is proximal to the one or more air vent openings 1446 of the second syringe barrel 1410, and the second plunger cavity 1480 is aligned with the one or more air vent openings 1446 of the second syringe barrel 1410. The radial projections 1478 located at the distal end 1474 of the second plunger shaft 1470 engage the second one-way plunger stop 1448 of the second syringe barrel 1410 for preventing retraction of the second plunger 1420 in the proximal direction DIR2.

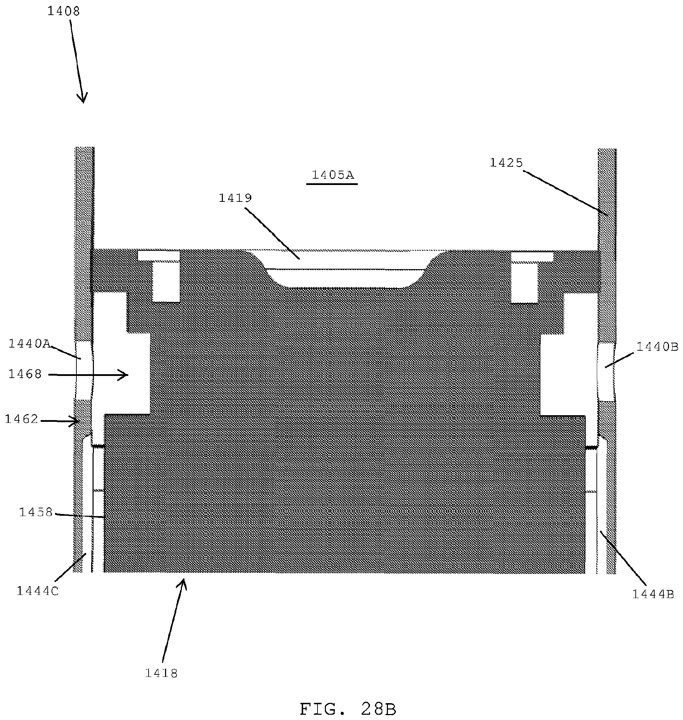

Referring to FIG. 28B, with the first plunger head 1419 being distal to the one or more air vent openings 1440A, 1440B of the first syringe barrel 1408, the distal end 1462 of the first plunger shaft 1458 is proximal to the air vent openings 1440A, 1440B that extend through the outer wall 1425 of the first syringe barrel 1408. The first plunger cavity 1468, located between the first plunger head 1419 and the distal end 1462 of the first plunger shaft 1458, is aligned with the air vent openings 1440A, 1440B that extend through the outer wall 1425 of the first syringe barrel 1408.

In one embodiment, a first part of a flowable composition is disposed within the first fluid compartment 1405A of the first syringe barrel 1408. The first plunger head 1419 has an outer perimeter that forms a liquid-tight and/or fluid-tight seal with an inner surface of the outer wall 1425 of the first syringe barrel 1408. Sterilization gases may pass through the air vent openings 1440A, 1440B, flow between the outer perimeter of the first plunger head 1419 and the inner surface of the outer wall 1425 of the first syringe barrel 1408, and pass into the first fluid chamber 1405A for sterilizing the first part of the flowable composition disposed within the first fluid chamber 1405 of the first syringe barrel. A similar structure is provided for sterilizing the flowable composition disposed within the second fluid chamber of the second syringe barrel.

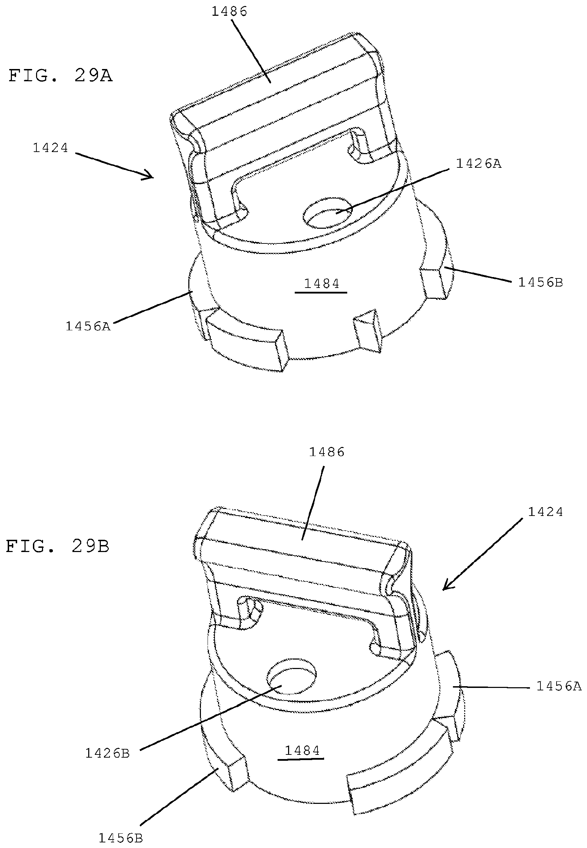

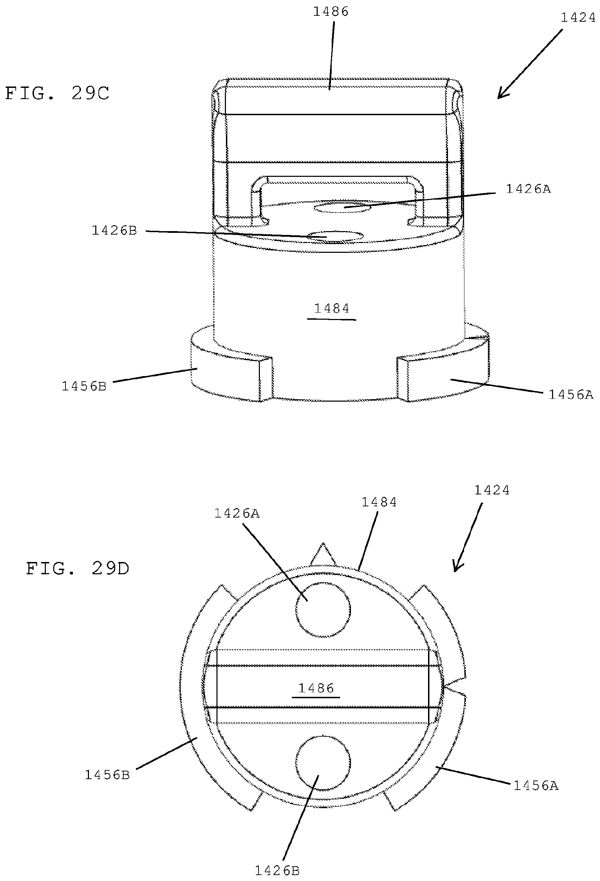

Referring to FIGS. 29A-29D, in one embodiment, the end cap 1424 is configured for being secured to the distal end of the enclosure 1402 (FIGS. 18A and 18B). The end cap preferably includes first and second end cap openings 1426A, 1426B that enable sterilization gases to pass through the end cap for sterilizing the gas sterilizable syringe 1400 and the first and second parts of the flowable composition disposed within the first and second syringe barrels 1408, 1410 (FIGS 18A and 18B).

In one embodiment, the end cap 1424 includes a central hub 1484 with radially extending projections 1456A, 1456B that extend outwardly from the central hub. The end cap 1424 includes an end cap grip 1486 that projects from a distal end of the end cap that may be used for securing the end cap to the distal end of the enclosure 1402 of the gas sterilizable syringe (FIGS. 18a and 18B).

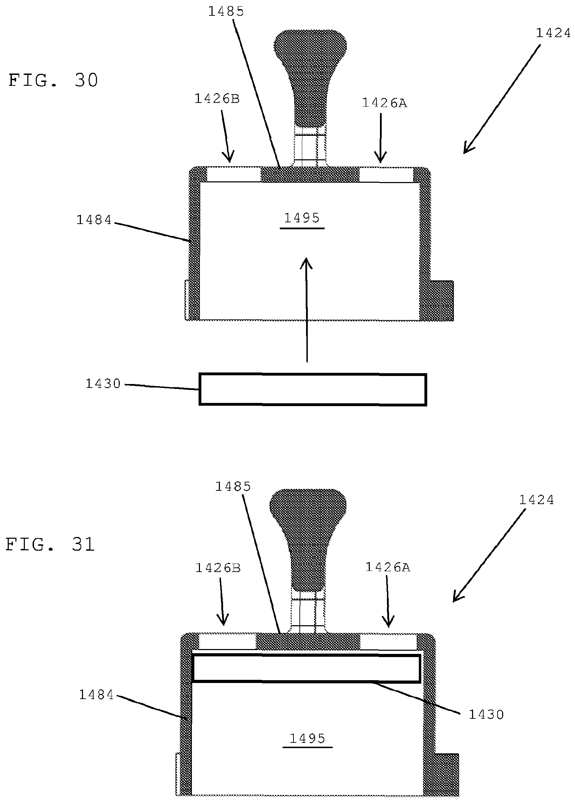

Referring to FIG. 30, in one embodiment, the end cap 1424 includes the central hub 1484 having a lower end that is open and an upper end that is closed by an end wall 1485. The end cap openings 1426A, 1426B pass through the end wall 1485 of the end cap. The hub 1484 and the end wall 1485 of the end cap define an end cap chamber 1495 that is adapted to receive the gas permeable barrier 1430.

Referring to FIG. 31, in one embodiment, the gas permeable barrier 1430 may be inserted into the end cap chamber 1495, whereupon the end cap openings 1426A, 1426B are preferably in alignment with the gas permeable barrier 1430. In one embodiment, the gas permeable barrier 1430 is preferably positioned adjacent the upper end of the central hub 1484 for being located near a bottom surface of the end cap end wall 1495. The lower end of the central hub 1484 preferably forms an air-tight seal with the distal end wall of the enclosure or syringe so that the sterilization gases may only flow into the gas sterilizable syringe via the first and second end cap openings 1426A, 1426B.

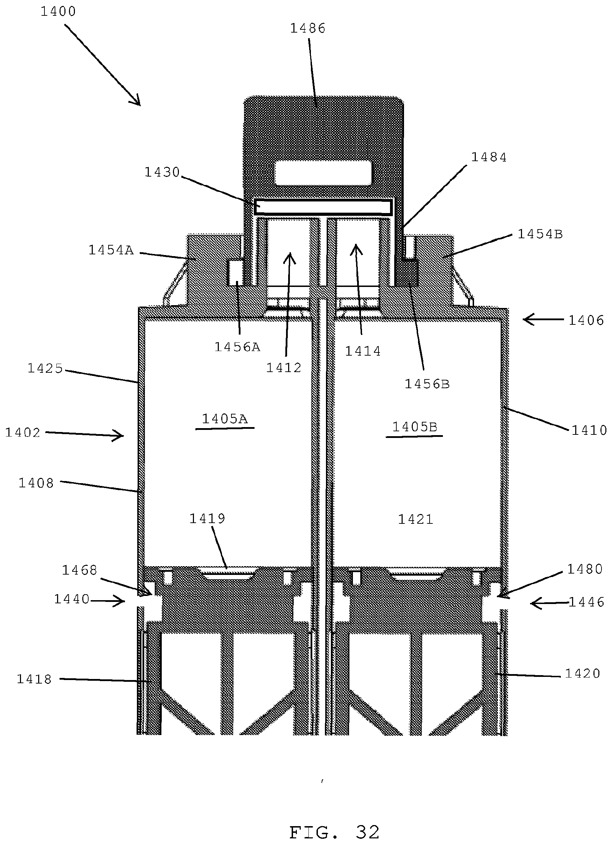

Referring to FIG. 32, in one embodiment, the end cap 1424 with the gas permeable barrier 1430 disposed therein may be secured to the distal end 1406 of the enclosure 1402 of the gas sterilizable syringe 1400. In one embodiment, the open lower end of the hub 1484 of the end cap 1424 is positioned over the free ends of the first and second dispensing openings 1412, 1414 of the enclosure 1402. The end cap grip 1486 may be used for rotating the end cap 1424 relative to the distal end 1406 of the enclosure 1402 so that the first and second radially extending projections 1566A, 1456B of the end cap engage the end cap securing flanges 1454A, 1454B of the enclosure for securing the end cap 1424 over the first and second dispensing openings 1412, 1414. When secured to the enclosure, the lower end of the hub of the end cap preferably forms an air-tight seal with the distal end of the enclosure.

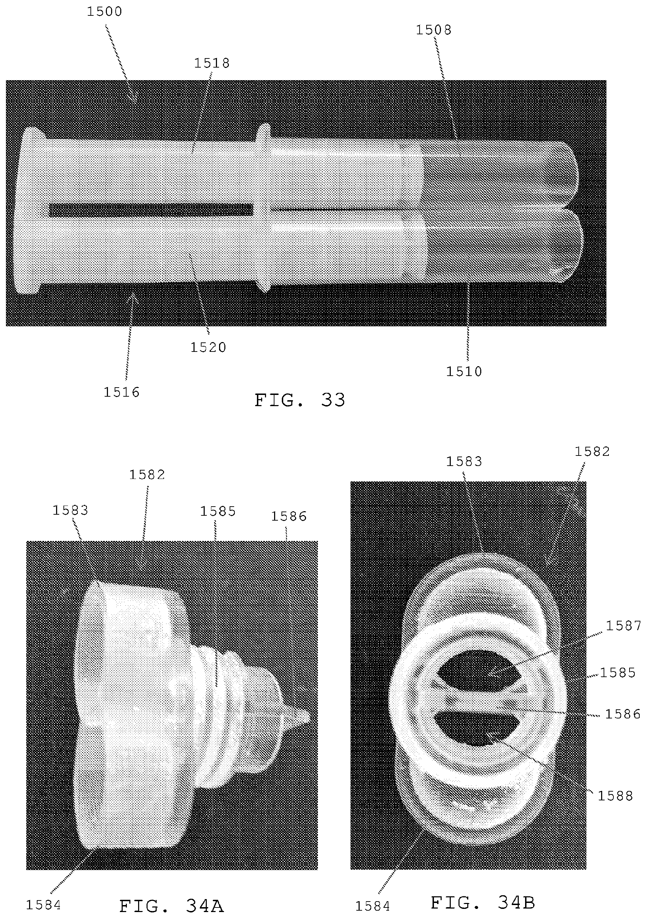

In one embodiment, with the end cap 1424 secured to the distal end 1406 of the enclosure 1402, the gas permeable barrier 1430 is disposed between the end cap openings 1426A, 1426B (FIG. 33) and the first and second dispensing openings 1412, 1414 that are in fluid communication with the respective first and second fluid chambers 1405A, 1405B. In one embodiment, the gas sterilizable syringe 1400 may be exposed to sterilization gases, whereupon the sterilization gases pass through the end cap openings 1426A, 1425B, the gas permeable barrier 1430, the first and second dispensing tips 1412, 1414, and into the first and second fluid chambers 1405A, 1405B for sterilizing the interior of the gas sterilizable syringe 1400 and the parts of the flowable material disposed within the first and second fluid chambers.

In one embodiment, the sterilization gases may also pass through the air vent openings 1440, 1446 of the first and second syringe barrels 1408, 1410 to provide supplemental pathways for sterilizing gases to enter into the first and second fluid chambers 1405A, 1405B of the syringe barrels 1408, 1410 of the gas sterilizable syringe 1400.

In one embodiment, the gas sterilizable syringe 1400 may be filled with the first and second parts of a flowable material (e.g., a silicon-based topical skin adhesive). In one embodiment, with the end cap 1424 and the gas permeable barrier 1430 secured over the distal end 1406 of the enclosure 1402, the open, proximal ends of the first and second syringe barrels 1408, 1410 are filled with the flowable material. In one embodiment, a first part of the flowable material is disposed within the first fluid chamber 1405A of the first syringe barrel 1408 and a second part of the flowable material is disposed within the second fluid chamber 1405B of the second syringe barrel 1410.

In one embodiment, the dual barrel plunger 1416 is assembled with the double barrel syringe 1402 so that the first plunger 1418 is disposed within the first fluid chamber 1405A of the first syringe barrel 1408 and the second plunger 1420 is disposed within the second fluid chamber 1405B of the second syringe barrel 1410. The dual barrel plunger 1416 is then advanced in the distal direction DIR1 to exhaust any air that is present within the first and second syringe barrels 1408, 1410 via the air vents openings 1440, 1446 that are formed in the first and second syringe barrels.

In one embodiment, in the rest position shown in FIG. 32, the radial projections at the distal ends of the plunger shafts preferably engage the one-way plunger stops 1442, 1448 (FIG. 28A) to prevent retraction of the first and second plungers. The plunger cavities 1468, 1480 are desirably in alignment with the respective air vent openings 1440, 1446, whereupon the air vent openings preferably provide a supplemental pathway for sterilization gases to enter into the first and second fluid compartments 1405A, 1405B of the respective first and second syringe barrels 1408, 1410.

In one embodiment, the sterilization gases preferably pass through the one or more air vent openings 1440 of the first syringe barrel 1408, into the first plunger cavity 1468, and between the outer perimeter of the first plunger head 1419 and the inner surface of the outer wall 1425 of the first syringe barrel for entering into the first fluid chamber 1405A of the first syringe barrel, thereby providing a supplemental path for sterilization gases to flow into the first fluid chamber 1405A.

In one embodiment, the sterilization gases preferably pass through the one or more air vent openings 1446 of the second syringe barrel 1410, into the second plunger cavity 1480, and between the outer perimeter of the second plunger head 1421 and the inner surface of the outer wall 1427 of the second syringe barrel 1410 for entering into the second fluid chamber 1405B of the second syringe barrel, thereby providing a supplemental path for sterilization gases to flow into the second fluid chamber 1405B.

Referring to FIG. 33, in one embodiment, a gas sterilizable syringe 1500 preferably includes an enclosure 1502 having a first syringe barrel 1508 and a second syringe barrel 1510. The gas sterilizable syringe 1500 desirably includes a dual barrel plunger 1516 including a first plunger 1518 disposed within the first syringe barrel 1508 and a second plunger 1520 disposed within the second syringe barrel 1510.

Referring to FIGS. 34A and 34B, in one embodiment, the gas sterilizable syringe 1500 (FIG. 33) desirably includes a multi-function connector 1582 that enables sterilizing, mixing, and dispensing operations to be performed. In one embodiment, the multi-function connector preferably includes a first hub 1583 adapted to be coupled with a distal end of the first syringe barrel 1508 (FIG. 33) and a second hub 1584 adapted to be secured to a distal end of the second syringe barrel 1510 (FIG. 33). The multi-function connector 1582 preferably includes an externally threaded post 1585 that extends distally from the first and second hub 1583, 1584. In one embodiment, a distally extending flange 1586 bisects the externally threaded post 1585 for dividing the externally threaded post 1584 into a first D-shaped opening 1587 and a second D-shaped opening 1588.

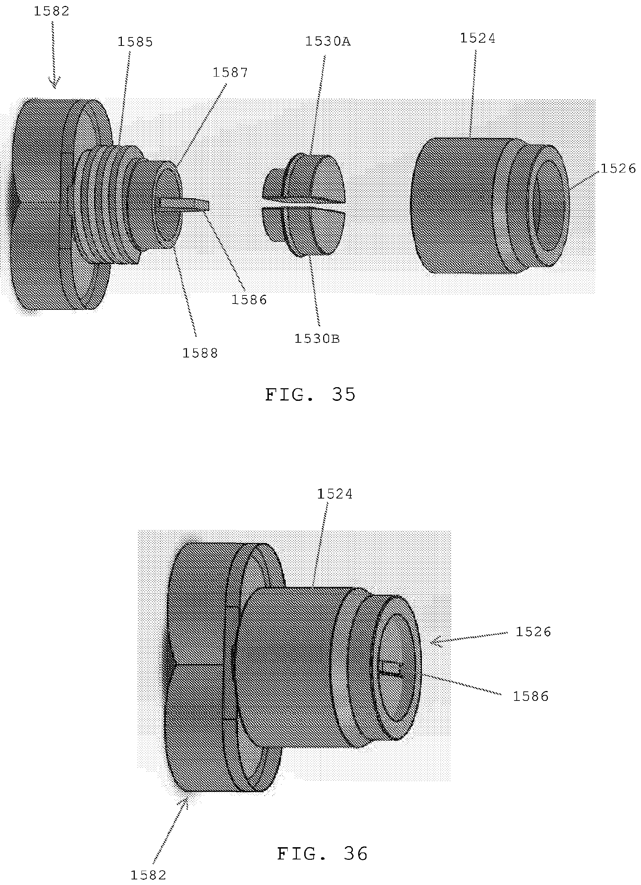

Referring to FIG. 35, in one embodiment, the first D-shaped opening 1587 of the multi-function connector 1582 is adapted to receive a first gas permeable barrier 1530A (e.g., a first gas permeable plug) and the second D-shaped opening 1588 of the multi-function connector 1582 is adapted to receive a second gas permeable barrier 1530B (e.g., a second gas permeable plug). In one embodiment, the first gas permeable barrier 1530A has a D-shape that matches the shaped of the first D-shaped opening 1587 of the connector 1582, and the second gas permeable barrier 1530B has a D-shape that matches the shape of the second D-shaped opening 1588 of the connector 1582.

Referring to FIGS. 35 and 36, in one embodiment, after the first and second gas permeable barriers 1530A, 1530B have been inserted into the respective D-shaped openings 1587, 1588 of the multi-function connector 1582, an end cap 1524 having a central opening 1526 is positioned over the gas permeable barriers and the distal end of the externally threaded post 1585. The distally extending flange 1586 of the multi-function connector 1582 bisects the central opening 1526 of the end cap 1524. In one embodiment, the end cap 1524 has internal threads (not shown) that are adapted to mesh with the external threads of the externally threaded post 1585 for securing the end cap 1524 to the externally threaded post 1585.

Referring to FIGS. 33, 36, and 37, in one embodiment, the multi-function connector 1582 may be assembled with the distal ends of the first and second syringe barrels 1508, 1510 of the gas sterilizable syringe 1500. In one embodiment, the distal end of the first syringe barrel 1508 is preferably insertable into the first hub 1583 of the multi-function connector 1582, and the distal end of the second syringe barrel 1510 is preferably insertable into the second hub 1584 of the multi-function connector 1582.

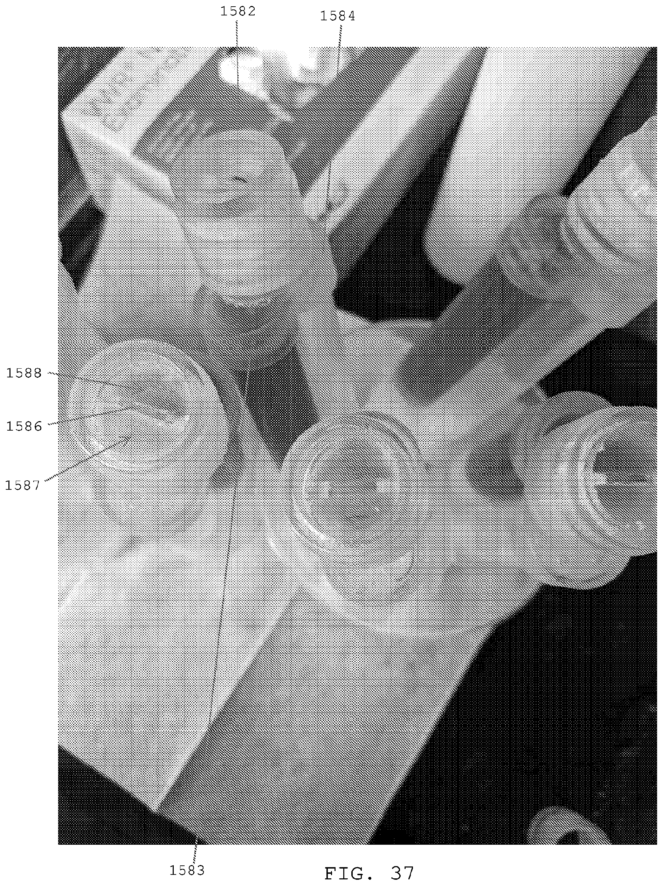

Referring to FIG. 37, in one embodiment, the multi-function connector 1582 preferably includes the first hub 1583 that is configured for being secured to the distal end of the first syringe barrel 1508 (FIG. 33) and the second hub 1584 that is configured for being secured to the distal end of the second syringe barrel 1510 (FIG. 33). The distally extending flange 1586 of the connector 1582 bisects the externally threaded post 1585 for dividing the externally threaded post into a first D-shaped opening 1587 located on one side of the distally extending flange 1586 and the second D-shaped opening 1588 located on the opposite side of the distally extending flange 1586. The gas permeable barriers 1530A, 1530B (FIG. 35) are inserted into the respective D-shaped openings 1587, 1588.

Referring to FIGS. 35, 36, 38 and 39, in one embodiment, the end cap 1524 may be unthreaded from its connection with the externally threaded post 1585 of the multi-function connector 1582. The first and second gas permeable barriers 1530A, 1530B may be removed from the respective D-shaped openings 1587, 1588 of the externally threaded post 1585. After removing the end cap 1534 and the gas permeable barriers 1530A, 1530B, a mixing and dispensing tip 1550 may be threaded onto the externally threaded post 1585 of the multi-function connector 1582 for expressing the flowable material from the syringe 1500.

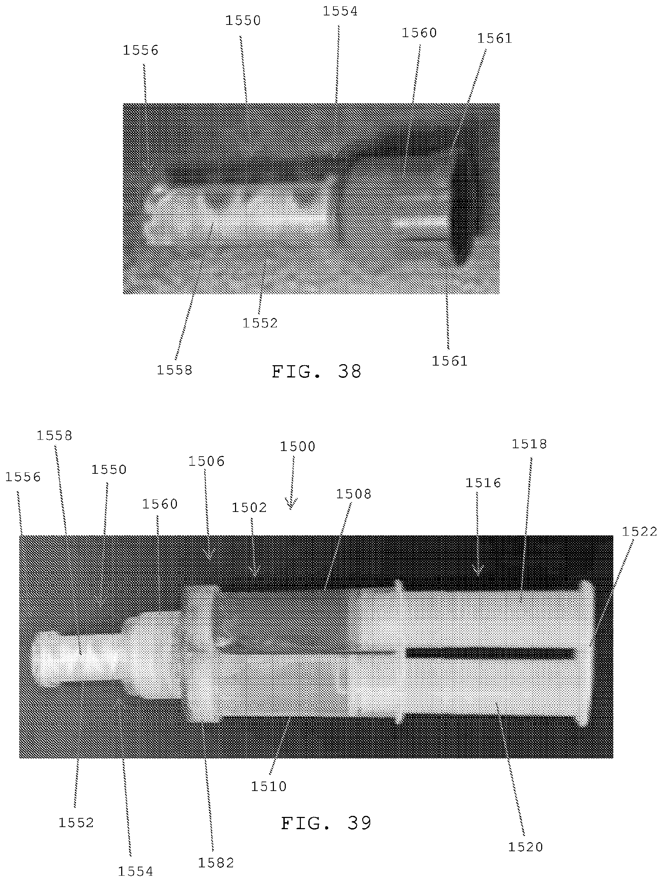

Referring to FIG. 38, in one embodiment, the mixing and dispensing tip 1550 preferably includes one or more radially extending projections 1561 that extend from the outer surface of the connector 1560. The one or more radially extending projections 1561 are preferably adapted to engage securing flanges located at the distal end of an enclosure (e.g., a syringe; a dual barrel syringe) for securing the mixing and dispensing tip 1550 to the distal end of the enclosure.

Referring to FIGS. 38 and 39, in one embodiment, the mixing and dispensing tip 1550 is preferably configured for mixing together the first and second parts of the flowable composition, and expressing the flowable composition from the distal end of the gas sterilizable syringe 1500.

In one embodiment, the mixing and dispensing tip 1550 preferably includes a mixing and dispensing tube 1552 having a proximal end 1554 and a distal end 1556. A static mixer 1558 is preferably disposed within the mixing and dispensing tube 1552 for mixing together the first and second parts of the flowable material that is disposed within the respective first and second syringe barrels 1508, 1510. The static mixer 1558 is configured for mixing together the first and second parts of a flowable material as the first and second parts flow along the length of the mixing and dispensing tube 1552.

In one embodiment, the mixing and dispensing tip 1550 preferably includes a connector 1560 having internal threads (not shown) that are configured to mesh with the external threads of the externally threaded post 1585 (FIG. 35). In one embodiment, the gas sterilizable syringe 1500 preferably includes a dual barrel plunger 1516 having a first plunger 1518 and a second plunger 1520. The gas sterilizable syringe 1500 preferably includes a thumb tab 1522 that may be depressed in the distal direction DIR1 toward the distal end 1506 of the dual barrel syringe 1502 for forcing the content within the first and second syringe barrels 1508, 1510 into the mixing and dispensing tip 1550.

Referring to FIG. 39, in one embodiment, a flowable material may be dispensed and/or expressed from the mixing and dispensing tip 1550 by depressing the thumb tab 1522 of the dual barrel plunger 1516 in the distal direction designated DIR1 for forcing the two parts of the flowable material to be expressed into the proximal end 1554 of the mixing and dispensing tube 1552. The static mixer 1558 is desirably configured for mixing together the first and second parts of the flowable material as the first and second parts flow along the length of the static mixer 1558 and the mixing and dispensing tube 1552. In one embodiment, during a surgical procedure, the flowable material is preferably expressed from the distal end 1556 of the mixing and dispensing tube 1552.

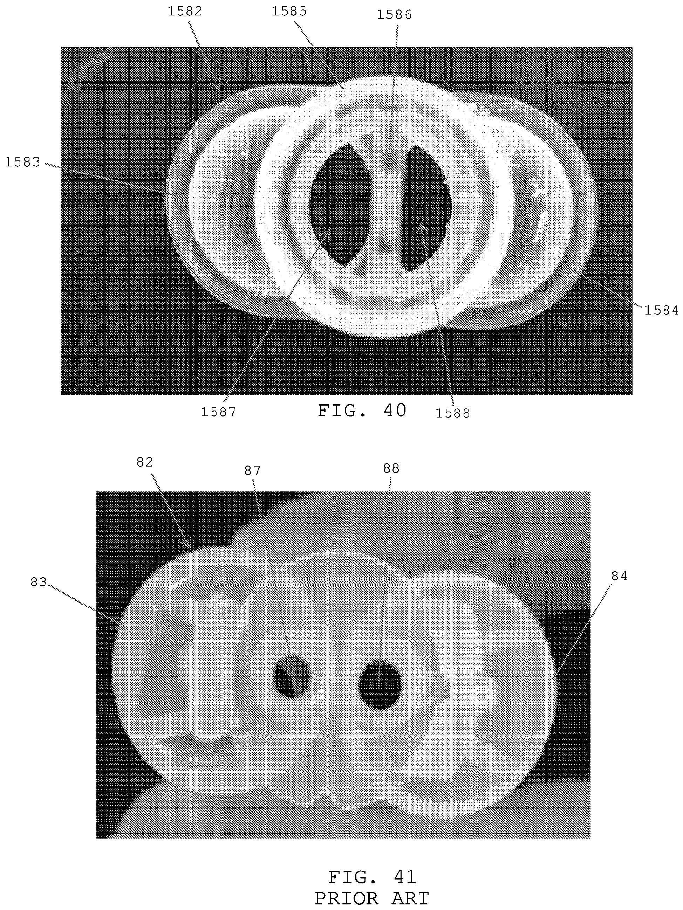

Referring to FIG. 40, in one embodiment, the multi-function connector 1582 preferably includes the externally threaded post 1585 (FIG. 35) and the dividing wall 1586 that bisects the externally threaded post 1585 into the first D-shaped opening 1587 and the second D-shaped opening 1588. The first and second hubs 1583, 1584 of the multi-function connector 1582 define a cross-sectional area which is substantially similar to the cross-sectional area of the first and second syringe barrels 1508, 1510 (FIG. 33). The D-shaped openings 1587, 1588 preferably define a cross-sectional area that is 20% of the cross-sectional area defined by the first hub 1583 and the second hub 1584 of the multi-function connector 1582.

FIG. 41 shows a prior art connector 82 having dispensing openings 87, 88 that comprise only 5% of the cross-sectional area defined by the first hub 83 and the second hub 84 of the prior art connector 82.

The multi-function connector 1582 shown in FIG. 40 provides benefits over the prior art connector 82 shown in FIG. 41. First, the multi-function connector has larger outlets (i.e., D-shaped openings 1587 and 1588) than the outlets (i.e., dispensing openings 87 and 88). The novel syringe design with the D-shaped openings 1587 and 1588 and the gas permeable stoppers inserted into the D-shaped openings improve sterilization of gas permeable syringes because the syringe's larger outlets allow more sterilizing gas inside the syringe. In addition, the larger size of the D-shaped openings 1587 and 1588 reduces the expression force required to express the flowable composition.

While the foregoing is directed to embodiments of the present invention, other and further embodiments of the invention may be devised without departing from the basic scope thereof, which is only limited by the scope of the claims that follow. For example, the present invention contemplates that any of the features shown in any of the embodiments described herein, or incorporated by reference herein, may be incorporated with any of the features shown in any of the other embodiments described herein, or incorporated by reference herein, and still fall within the scope of the present invention.

### Examples

The following are numbered examples of the disclosure that may or may not be claimed.
1. A gas sterilizable syringe comprising:
   an enclosure having walls that define a fluid chamber;
   a flowable material disposed within said fluid chamber of said enclosure;
   a plunger assembled with said enclosure and being moveable toward a distal end of said enclosure for dispensing said flowable material from said enclosure;
   one or more apertures formed in at least one of said walls of said enclosure;
   a gas permeable barrier covering at least one of said apertures formed in the at least one of said walls of said enclosure for enabling sterilization gases to pass through the at least one of said apertures covered by said gas permeable barrier while preventing said flowable material from passing through the at least one of said apertures covered by said gas permeable barrier.
2. The gas sterilizable syringe of example 1, wherein said gas permeable barrier that covers the at least one of said apertures formed in the at least one of said walls of said enclosure is permeable to said sterilization gases and impermeable to said flowable material disposed within said fluid chamber of said enclosure.
3. The gas sterilizable syringe of example 2, wherein said gas permeable barrier has a closed porosity configured to enable ingress and egress of said sterilization gases during a sterilization procedure and to prevent said flowable material from passing through said gas permeable barrier.
4. The gas sterilizable syringe of example 1, wherein the one or more apertures formed in the at least one of said walls of said enclosure comprise a plurality of apertures formed in the at least one of said walls of said enclosure, and wherein said gas permeable barrier comprises a plurality of gas permeable bodies filling each of the respective plurality of apertures formed in the at least one of said walls of said enclosure.
5. The gas sterilizable syringe of example 4, wherein said plurality of gas permeable bodies filling each of the respective plurality of apertures are bonded to the at least one of said walls of said enclosure.
6. The gas sterilizable syringe of example 1, wherein said gas permeable barrier is made of a material selected from the group consisting of silicone, room temperature vulcanized silicone (RTV), liquid silicone rubber (LSR), high consistency rubber (HCR), and synthetic flashspun high-density polyethylene fibers.
7. The gas sterilizable syringe of example 1, wherein said sterilization gases includes ethylene oxide, and said flowable material is selected from the group consisting of liquids and topical skin adhesives including silicone-based topical skin adhesives.
8. The gas sterilizable syringe of example 1, wherein said enclosure comprises:
   a syringe barrel that surrounds said fluid chamber;
   a dispensing opening located at a distal end of said syringe barrel;
   a plunger moveable toward the distal end of said syringe barrel for dispensing said flowable material via said dispensing opening located at the distal end of said syringe barrel;
   said gas permeable barrier covering said dispensing opening for enabling said sterilization gases to pass through said dispensing opening while preventing said flowable material from passing through said dispensing opening.
9. The gas sterilizable syringe of example 8, further comprising:
   an end cap secured to the distal end of said syringe barrel and covering said dispensing opening;
   one or more end cap openings formed in said end cap for enabling said sterilization gases to pass through said end cap;
   said gas permeable barrier being disposed inside said end cap and being located between said one or more end cap openings and said dispensing opening at the distal end of said syringe barrel;
   said end cap having a central hub that engages a distal end wall of said syringe barrel for forming an airtight seal between said central hub of said end cap and the distal end wall of said syringe barrel.
10. A gas sterilizable syringe comprising:
   a dual barrel syringe including a first syringe barrel having a first fluid chamber and a first fluid dispensing opening and a second syringe barrel having a second fluid chamber and a second fluid dispensing opening;
   a dual barrel plunger including a first plunger disposed within said first fluid chamber of said first syringe barrel and a second plunger disposed within said second fluid chamber of said second syringe barrel;
   an end cap releasably secured to a distal end of said dual barrel syringe for covering said first and second fluid dispensing openings, said end cap having at least one end cap opening formed therein;
   a gas permeable barrier disposed within said end cap and being located between said at least one end cap opening and said first and second fluid dispensing openings of said respective first and second syringe barrels.
11. The gas sterilizable syringe of example 10, further comprising:
   a flowable material including a first part and a second part that are configured for being mixed together;
   said first part of said flowable material being disposed within said first fluid chamber of said first syringe barrel;
   said second part of said flowable material being disposed within said second fluid chamber of said second syringe barrel;
   said gas permeable barrier covering said first and second fluid dispensing openings of
   said first and second syringe barrels, wherein said gas permeable barrier enables sterilization gases to pass through said first and second fluid dispensing openings while preventing said first and second parts of said flowable material from passing through said first and second fluid dispensing openings.
12. The gas sterilizable syringe of example 11, wherein said end cap comprises a hub that engages a distal end wall of said dual barrel syringe for forming an airtight seal between said end cap hub and the distal end wall of said dual barrel syringe.
13. The gas sterilizable syringe of example 12, further comprising:
   said end cap hub having a proximal end that is open for receiving said first and second fluid dispensing openings and a distal end that is closed by a distal end wall that includes said at least one end cap opening;
   said end cap including at least one radially extending projection that extends outwardly from an outer surface of said end cap hub;
   said dual barrel syringe including at least one securing flange projecting from the distal end wall of said dual barrel syringe, wherein said at least one securing flange is configured to engage said at least one radially extending projection of said end cap hub for releasably securing said end cap to the distal end wall of said dual barrel syringe.
14. The gas sterilizable syringe of example 13, further comprising:
   an applicator tip configured for expressing said flowable material from the distal end of said dual barrel syringe, said applicator tip including a dispensing tube having a proximal end and a distal end, an applicator tip connector secured to the proximal end of said dispensing tube, and a static mixer disposed within said dispensing tube;
   said applicator tip connector having at least one radially extending projection that is configured to engage said at least one securing flange projecting from the distal end wall of said dual barrel syringe for releasable securing said applicator tip to the distal end wall of said dual barrel syringe.
15. The gas sterilizable syringe of example 11, wherein said gas permeable barrier is permeable to said sterilization gases and impermeable to said first and second parts of said flowable material.
16. The gas sterilizable syringe of example 15, wherein said gas permeable barrier has a closed cell porosity configured to enable ingress and egress of said sterilization gases during a sterilization procedure and to block passage of said first and second parts of said flowable material through said gas permeable barrier.
17. A gas sterilizable syringe comprising:
   a dual barrel syringe including a first syringe barrel and a second syringe barrel;
   said first syringe barrel including a first fluid chamber and a first fluid dispensing opening located at a distal end of said first syringe barrel;
   said second syringe barrel including a second fluid chamber and a second fluid dispensing opening located at a distal end of said second syringe barrel;
   a first part of a flowable material disposed within said first fluid chamber of said first syringe barrel;
   a second part of said flowable material disposed within said second fluid chamber of said second syringe barrel;
   a dual barrel plunger assembled with said dual barrel syringe, said dual barrel plunger including a first plunger disposed within said first fluid chamber of said first syringe barrel and a second plunger disposed within said second fluid chamber of said second syringe barrel;
   an end cap releasably secured to a distal end of said dual barrel syringe for covering said first and second fluid dispensing openings located at the distal ends of said respective first and second syringe barrels;
   said end cap having at least one end cap opening formed therein;
   a gas permeable barrier disposed within said end cap and being located between said at least one end cap opening and said first and second fluid dispensing openings located at the distal ends of said respective first and second syringe barrels, wherein said gas permeably barrier is permeable to sterilization gases and impermeable to said flowable material for enabling said sterilization gases to pass through said first and second fluid dispensing openings while preventing said first and second parts of said flowable material from passing through said gas permeable barrier.
18. The gas sterilizable syringe of example 17, further comprising:
   said end cap comprising a central hub that engages a distal end wall of said dual barrel syringe for forming an airtight seal between said end cap hub and the distal end wall of said dual barrel syringe.
   said central hub of said end cap having a proximal end that is open for receiving said first and second fluid dispensing openings and a distal end that is closed by a distal end wall that includes said at least one end cap opening;
   said end cap including at least one radially extending projection that extends outwardly from an outer surface of said central hub of said end cap;
   said dual barrel syringe including at least one securing flange projecting from the distal end wall of said dual barrel syringe that is configured to engage said at least one radially extending projection of said end cap for releasably securing said end cap to the distal end wall of said dual barrel syringe.
19. The gas sterilizable syringe of example 17, further comprising:
   said first syringe barrel having a cylindrical-shaped outer wall that surrounds said first fluid chamber and that extends to the distal end of said first syringe barrel;
   at least one first air vent opening formed in said cylindrical-shaped outer wall of said first syringe barrel;
   a first one-way plunger stop formed in an inner surface of said cylindrical-shaped outer wall of said first syringe barrel and being located between said at least one first air vent and the distal end of said first syringe barrel;
   at least one second air vent opening formed in said cylindrical-shaped outer wall of said second syringe barrel;
   a second one-way plunger stop formed in an inner surface of said cylindrical-shaped outer wall of said second syringe barrel and being located between said at least one second air vent of said second syringe barrel and the distal end of said second syringe barrel.
20. The gas sterilizable syringe of example 19, further comprising:
   said first plunger having a first plunger head located at a distal end of said first plunger, said first plunger head being in contact with said first one-way plunger stop and being located between said at least one first air vent and the distal end of said first syringe barrel;
   said second plunger having a second plunger head located at a distal end of said second plunger, said second plunger head being in contact with said second one-way plunger stop and being located between said at least one second air vent and the distal end of said second syringe barrel.

## Claims

1. A gas sterilizable syringe (100, 200, 300, 400, 500, 600, 700, 900, 1000, 1100) comprising:
an enclosure having walls that define a fluid chamber, wherein the fluid chamber is configured to receive a flowable material;
a plunger assembled with said enclosure and being moveable toward a distal end of said enclosure for dispensing said flowable material from said enclosure; wherein said enclosure comprises:
a syringe barrel (102, 202, 302, 402, 502, 602, 702, 902, 1002, 1102) that surrounds said fluid chamber;
a dispensing opening located at a distal end of said syringe barrel;
the gas sterilizable syringe further comprising:
an end cap (116, 216, 416, 516, 716, 916) secured to the distal end of said syringe barrel and covering said dispensing opening;
one or more end cap openings (134, 136, 945, 934) formed in said end cap for enabling said sterilization gases to pass through said end cap;
a gas permeable barrier being disposed inside said end cap (116, 216, 416, 516, 716, 916) and being located between said one or more end cap openings and said dispensing opening at the distal end of said syringe barrel (102, 202, 302, 402, 502, 602, 702, 902, 1002, 1102).

2. The gas sterilizable syringe (100, 200, 300, 400, 500, 600, 700, 900, 1000, 1100) as claimed in claim 1 comprising:
one or more apertures (126, 130, 226, 326, 426, 526, 626, 726, 1026, 1126) formed in at least one of said walls of said enclosure;
said gas permeable barrier (442, 335, 544, 644, 744, 1045, 1145) covering at least one of said apertures formed in the at least one of said walls of said enclosure for enabling sterilization gases to pass through the at least one of said apertures covered by said gas permeable barrier.

3. The gas sterilizable syringe (100, 200, 300, 400, 500, 600, 700, 900, 1000, 1100) as claimed in claim 2, wherein the one or more apertures (126, 130, 226, 326, 426, 526, 626, 726, 1026, 1126) formed in the at least one of said walls of said enclosure comprise a plurality of apertures formed in the at least one of said walls of said enclosure, and wherein said gas permeable barrier (442, 335, 544, 644, 744, 1045, 1145) comprises a plurality of gas permeable bodies filling each of the respective plurality of apertures formed in the at least one of said walls of said enclosure; optionally wherein said plurality of gas permeable bodies filling each of the respective plurality of apertures are bonded to the at least one of said walls of said enclosure.

4. The gas sterilizable syringe (100, 200, 300, 400, 500, 600, 700, 900, 1000, 1100) as claimed in any one of claims 1 to 3, comprising:
a flowable material disposed within said fluid chamber of said enclosure; said gas permeable barrier (442, 335, 544, 644, 744, 1045, 1145) covering said dispensing opening for enabling said sterilization gases to pass through said dispensing opening while preventing said flowable material from passing through said dispensing opening; optionally wherein said flowable material is selected from the group consisting of liquids and topical skin adhesives including silicone-based topical skin adhesives.

5. The gas sterilizable syringe (100, 200, 300, 400, 500, 600, 700, 900, 1000, 1100) as claimed in claim 4, wherein said gas permeable barrier is permeable to said sterilization gases and impermeable to said flowable material disposed within said fluid chamber of said enclosure; optionally wherein said gas permeable barrier has a closed porosity configured to enable ingress and egress of said sterilization gases during a sterilization procedure and to prevent said flowable material from passing through said gas permeable barrier.

6. The gas sterilizable syringe (100, 200, 300, 400, 500, 600, 700, 900, 1000, 1100) as claimed in any one of claims 1 to 5, said end cap (116, 216, 416, 516, 716, 916) having a central hub that engages a distal end wall of said syringe barrel for forming an airtight seal between said central hub of said end cap and the distal end wall of said syringe barrel.

7. The gas sterilizable syringe as claimed in any one of the preceding claims, wherein one or both of the following options is fulfilled
a) said gas permeable barrier (442, 335, 544, 644, 744, 1045, 1145) is made of a material selected from the group consisting of silicone, room temperature vulcanized silicone (RTV), liquid silicone rubber (LSR), high consistency rubber (HCR), and synthetic flashspun high-density polyethylene fibers; and
b) said sterilization gases includes ethylene oxide.
